(19) Europäisches Patentamt
European Patent Office
Office européen des brevets

(11) Numéro de publication: **0 053 538 B1**

# (12) FASCICULE DE BREVET EUROPEEN

(45) Date de publication du fascicule du brevet: **12.03.86**

(51) Int. Cl.⁴: **C 07 D 501/24**, C 07 D 501/26 // C07D253/06

(21) Numéro de dépôt: **81401824.8**

(22) Date de dépôt: **19.11.81**

(54) **Nouvelles vinyl-3 céphalosporines et leur préparation.**

(30) Priorité: **20.11.80 FR 8024635**

(43) Date de publication de la demande: **09.06.82 Bulletin 82/23**

(45) Mention de la délivrance du brevet: **12.03.86 Bulletin 86/11**

(84) Etats contractants désignés: **AT BE CH DE FR GB IT LI LU NL SE**

(56) Documents cités:
**FR - A - 2 081 451**
**FR - A - 2 137 899**
**US - A - 4 065 620**

**Le dossier contient des informations techniques présentées postérieurement au dépôt de la demande et ne figurant pas dans le présent fascicule.**

(73) Titulaire: **RHONE-POULENC SANTE, Les Miroirs 18 Avenue d'Alsace, F-92400 Courbevoie Cedex (FR)**

(72) Inventeur: **Farge, Daniel, 30 rue des Pins Sylvestres, F-94320 Thiais (FR)**
Inventeur: **Le Roy, Pierre, 2 Allée des Cerisiers, F-94320 Thiais (FR)**
Inventeur: **Moutonnier, Claude, 3 rue Auguste Rodin, F-92350 Le Plessis-Robinson (FR)**
Inventeur: **Peyronel, Jean-François, 36 parc d'Ardenay, F-91110 Palaiseau (FR)**

(74) Mandataire: **Gaumont, Robert et al, RHONE-POULENC RECHERCHES Service Brevets Pharma 25, Quai Paul Doumer, F-92408 Courbevoie Cedex (FR)**

**EP 0 053 538 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne de nouvelles vinyl-3 céphalosporines de formule générale:

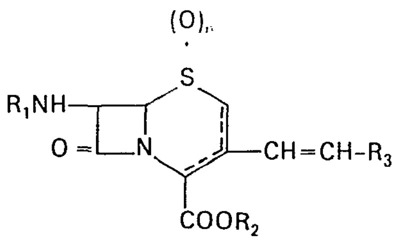
(I)

leurs sels et leur préparation.

Les demandes de brevet français 2 081 451 et 2 137 899 décrivent la préparation de dérivés de vinyl-3 céphalosporines. Dans ces produits la chaîne vinyle ne porte pas de substituant acyloxy ou halogène.

Les produits de formule générale (I), dans laquelle n égale 0 ou 1, se présentent sous forme bicyclooctène-2 ou -3 (selon la nomenclature des Chemical Abstracts) lorsque n = 0 ou sous forme bicyclooctène-2 lorsque n = 1, le substituant sur l'atome de carbone en position -3 du bicyclooctène présentant la stéréoisomérie cis ou trans et

1-a) le symbole $R_1$ représente un atome d'hydrogène, un radical de formule générale:

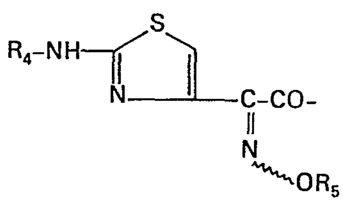
(II)

[dans laquelle $R_4$ est un atome d'hydrogène ou un radical protecteur d'amine (tel que t.butoxycarbonyle, trichloro-2,2,2 éthoxycarbonyle, chloracétyle, trichloracétyle, trityle, benzyle, dibenzyle, benzyloxycarbonyle, p.nitrobenzyloxycarbonyle, p.méthoxybenzyloxycarbonyle, formyle ou trifluoracétyle) et $R_5$ est un atome d'hydrogène, un radical alcoyle, vinyle, cyanométhyle ou un groupement protecteur d'oxime tel que trityle, tétrahydropyrannyle ou méthoxy-2 propyle-2], un radical benzhydryle, trityle, un radical acyle de formule générale:

$R_6CO-$ (III)

dans laquelle $R_6$ est un atome d'hydrogène ou un radical alcoyle (éventuellement substitué par un ou plusieurs atomes d'halogène ou par un radical phényle ou phénoxy) ou phényle, un radical de formule générale:

$R_7$ O CO- (IV)

{dans laquelle $R_7$ est un radical alcoyle ramifié non substitué ou alcoyle droit ou ramifié portant un ou plusieurs substituants [choisis parmi les atomes d'halogène et les radicaux cyano, trialcoylsilyle, phényle et phényle substitué (par un ou plusieurs radicaux alcoyloxy, nitro ou phényle)], vinyle, allyle ou quinolyle} ou bien un radical nitrophénylthio, ou bien $R_1NH-$ est remplacé par un radical méthylène amino dans lequel le radical méthylène est substitué par un groupement dialcoylamino ou aryle (lui-même éventuellement substitué par un ou plusieurs radicaux méthoxy ou nitro) et

le symbole $R_2$ représente un atome d'hydrogène ou un radical facilement éliminable par voie enzymatique de formule générale:

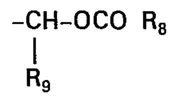
(V)

(dans laquelle $R_8$ représente un radical alcoyle ou le radical cyclohexyle et $R_9$ représente un atome d'hydrogène ou un radical alcoyle) ou un radical méthoxyméthyle, t.butyle, benzhydryle, nitrobenzyle ou p.méthoxybenzyle, ou bien

1-b) le symbole $R_1$ représente un atome d'hydrogène, un radical alcanoyle contenant 1 à 8 atomes de carbone, alcanoyle contenant 2 à 8 atomes de carbone substitué (par des atomes de chlore ou de brome), azidoacétyle, cyanoacétyle, un radical acyle de formule générale:

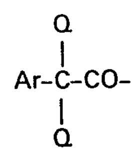
(VI)

dans laquelle chaque Q est H ou méthyle et Ar représente un radical thiényle-2, thiényle-3, furyle-2, furyle-3, pyrrolyle-2, pyrrolyle-3 ou phényle [éventuellement substitué par des atoms d'halogène ou des radicaux trifluorométhyle, hydroxy, alcoyle (contenant 1 à 3 atomes de carbone), alcoyloxy (contenant 1 à 3 atomes de carbone), cyano ou nitro, dont au moins l'un est situé en méta ou en para du phényle];

un radical acyle de formule générale:

$Ar-X-CH_2-CO-$ (VII)

dans laquelle X est l'oxygène ou le soufre et Ar est défini comme ci-dessus ou Ar-X- représente pyridyl-4 thio;

un radical acyle répondant à la formule générale:

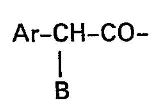
(VIII)

dans laquelle Ar est défini comme précédemment et B représente un radical amino, amino protégé (par un groupement benzyloxycarbonyle, alcoyloxycarbonyle, cyclopentyloxycarbonyle, cyclohexyloxycarbonyle, benzhydryloxycarbonyle, trityle ou trichloro-2,2,2 éthoxycarbonyle), un radical sulfo, un radical hydroxy ou carboxy [éventuellement protégés par estérification respectivement avec un acide alcanoïque ou un alcool (contenant 1 à 6 atomes de carbone)], azido, cyano ou carbamoyle;
un radical (sydnone-3)-2 alcanoyle (dont la partie alcanoyle contient 1 à 3 atomes de carbone);
un radical de formule générale:

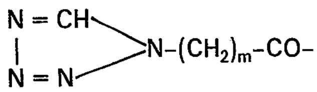 (IX)

dans laquelle m est 0 à 2;
ou un radical amino-5 adipoyle [dans lequel le groupement amino est éventuellement protégé par un radical alcanoyle (contenant 1 à 3 atomes de carbone et éventuellement substitué par un atome de chlore) et dans lequel le groupe carboxy est protégé par un groupe benzhydryle, trichloro-2,2,2 éthyle, t.alcoyle (contenant 4 à 6 atomes de carbone) ou nitrobenzyle]
ou bien $R_1NH$ est remplacé par un groupement imide cyclique d'un acide dicarboxylique,
le symbole $R_2$ représente un atome d'hydrogène ou un radical t.alcoyle contenant 4 à 6 atomes de carbone, t.alcényle contenant 6 ou 7 atomes de carbone, t.alcynyle contenant 6 ou 7 atomes de carbone, benzyle, méthoxybenzyle, nitrobenzyle, trichloro-2,2,2 éthyle, benzhydryle, succinimidométhyle ou phtalimidométhyle et
le symbole $R_3$ représente un atome d'halogène tel que chlore, brome ou iode;
2- Le symbole $R_1$ représente un radical de formule générale (II) dans laquelle le symbole $R_5$ représente un radical de formule générale:

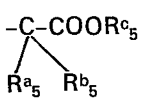 (IIa)

(dans laquelle $R^a_5$ et $R^b_5$ soit sont identiques ou différents et représentent des atomes d'hydrogène ou des radicaux alcoyle soit forment ensemble un radical alcoylène contenant 2 ou 3 atomes de carbone, et $R^c_5$ représente un atome d'hydrogène ou un radical protecteur d'acide tel que méthoxyméthyle, t.butyle, benzhydryle, benzyle, nitrobenzyle et p.méthoxybenzyle);
le symbole $R_2$ est défini comme ci-avant en (1-a), et
le symbole $R_3$ représente un radical de formule générale:

$R'_3-SO_2O-$ (X) ou $R''_3-COO-$ (XI)

(dans lesquelles $R'_3$ représente un radical alcoyle, trifluorométhyle, trichlorométhyle ou phényle non substitué ou substitué par un atome d'halogène ou par un radical alcoyle ou nitro, et $R''_3$ est défini comme $R'_3$ ou représente un radical acylméthyle, acyl-2 éthyle, acyl-2 propyle, alcoyloxycarbonylméthyle, alcoyloxycarbonyl-2 éthyle ou alcoyloxycarbonyl-2 propyle) ou un atome d'halogène (tel que chlore, brome et iode).

Il est entendu que les portions ou radicaux alcoyles ou acyles cités ci-dessus (ou qui seront cités ci-après) sont (sauf mention spéciale) droits ou ramifiés et contiennent 1 à 4 atomes de carbone, et que les mélanges des isomères bicyclooctène-2 et -3 et/ou cis et trans entrent dans le cadre de l'invention.

Dans ce qui suit la stéréoisomérie trans est désignée par E et la stéréoisomérie cis est désignée par Z.

Par ailleurs, le groupement $-OR_5$ du radical de formule générale (II) peut se trouver dans l'une des positions syn ou anti et ces isomères et leurs mélanges entrent aussi dans le cadre de l'invention.

La forme syn peut être représentée par la formule:

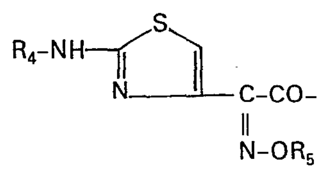 (IIb).

La forme anti peut être représentée par la formule:

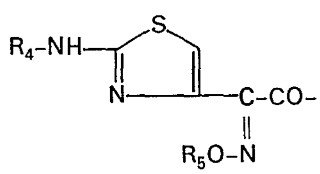 (IIc)

Lorsque $R^a_5$ et $R^b_5$ sont différents il existe également des diastéréoisomères. Ces isomères et leurs mélanges entrent aussi dans le cadre de la présente invention.

Ia) Selon l'invention, les produits de formule générale (I) {pour lesquels, n étant défini comme précédemment, $a'_1$) les symboles $R_1$ et $R_2$ sont définis comme dans la formule générale (I) en (1-a) à l'exception pour $R_1$ de représenter un atome d'hydrogène, ou bien le symbole $R_1$ représente un radical alcanoyle contenant 1 à 8 atomes de carbone, alcanoyle contenant 2 à 8 atomes de carbone substitué par des atomes de chlore ou de brome, un radical acyle de formule générale (VI) dans laquelle Q est H ou méthyle et Ar représente un radical thiényle-2, thiényle-3, furyle-2, furyle-3, pyrrolyle-2, pyrrolyle-3 ou phényle [éventuellement substitué par des atomes d'halogène ou des radicaux hydroxy, alcoyle (contenant 1 à 3 atomes de carbone) ou alcoyloxy (contenant 1 à 3 atomes de carbone), dont au moins l'un est situé en méta ou en para du phényle], un radical acyle de formule générale (VII), un radical acyle répondant à la formule générale (VIII) dans laquelle Ar est défini comme précédemment et B représente un radical amino protégé [par un groupement benzyloxycarbonyle, alcoyloxycarbonyle, cyclopentyloxycarbonyle, cyclohexyloxycarbonyle, benzhydryloxycarbonyle,

trityle ou trichloro-2,2,2 éthoxycarbonyle], un radical sulfo, un radical hydroxy ou carboxy [éventuellement protégés par estérification avec un acide alcanoïque ou un alcool (contenant 1 à 6 atomes de carbone)], ou un radical amino-5 adipoyle [dans lequel le groupement amino est éventuellement protégé par un radical alcanoyle (contenant 1 à 3 atomes de carbone et éventuellement substitué par un atome de chlore) et dans lequel le groupe carboxy est protégé par un groupe benzhydryle, trichloro-2,2,2 éthyle, t.alcoyle (contenant 4 à 6 atomes de carbone) ou nitrobenzyle] ou bien $R_1NH$ est remplacé par un groupement imide cyclique d'un acide dicarboxylique et $R_2$ est défini comme dans la formule générale (I) en (1-b) et le symbole $R_3$ est défini comme dans la formule générale (I) en (1-),

b'$_1$) les symboles $R_1$, $R_2$ et $R_3$ sont définis comme dans la formule générale (I) en (2-)}

peuvent être préparés par action d'une forme activée d'un acide $R'_3SO_3H$ ou $R''_3COOH$, du type:

$(R'_3SO_2)_2O$ (XII)

$R'_3SO_2Hal$ (XIII)

$(R''_3CO)_2O$ (XIV)

$R''_3COHal$ (XV)

(dans lesquelles R' et R'' sont définis comme précédemment et Hal représente un atome d'halogène), ou d'un agent d'halogénation, sur un produit de formule générale:

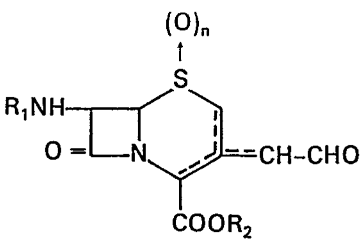

(XVI)

[dans laquelle, n étant défini comme précédemment, le produit se présente sous forme oxoéthyl-3 bicyclooctène-2 ou -3 ou oxoéthylidène-3 bicyclooctane lorsque n = 0 et sous forme oxoéthyl-3 bicyclooctène-2 ou oxoéthylidène-3 bicyclooctane lorsque n = 1, $R_1$ est défini comme ci-dessus (à l'exception de représenter un radical de formule générale (II) dans laquelle $R_4$ est hydrogène) et $R_2$ est défini comme ci-dessus (à l'exception de représenter l'hydrogène)] ou sur un mélange de ses isomères, suivie éventuellement de la réduction du sulfoxyde obtenu et, le cas échéant, de l'élimination des groupements protecteurs de la fonction amine du radical de formule générale (II) et/ou éventuellement des fonctions acides lorsque l'on veut obtenir un produit de formule générale (I) pour lequel les fonctions amine et/ou acide sont libres.

Il est entendu que, lorsque $R_1$ est un radical de formule générale (II) dans laquelle $R_5$ est un atome d'hydrogène, il est nécessaire de protéger l'oxime par un groupement tel qu'indiqué précédemment, qui pourra par la suite être éliminé dans les conditions indiquées ci-après.

On opère généralement en présence d'une base tertiaire de formule générale:

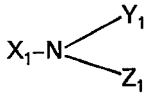

(XVII)

dans laquelle $X_1$, $Y_1$ et $Z_1$ représentent des radicaux alcoyle ou phényle, ou éventuellement deux d'entre eux forment un cycle avec l'atome d'azote auquel ils sont rattachés (par exemple en présence de triéthylamine ou de diméthylaniline), dans un solvant organique chloré (par exemple dichlorométhane), dans un ester (par exemple acétate d'éthyle), dans un éther (par exemple dioxanne, tétrahydrofuranne), dans un amide (par exemple diméthylacétamide, diméthylformamide ou hexaméthylphosphorotriamide), dans l'acétonitrile ou la N-méthylpyrrolidone, ou dans un mélange de tels solvants ou directement dans un solvant basique comme la pyridine ou bien, lorsque $R_3$ est autre qu'un atome d'halogène, on peut opérer en milieu hydroorganique en présence d'un agent alcalin de condensation (par exemple bicarbonate alcalin, soude ou potasse) à une température comprise entre -78°C et la température de reflux du mélange réactionnel.

Eventuellement on opère sous azote.

Lorsque l'on veut préparer un produit de formule générale (I) dans laquelle $R_3$ est un atome d'halogène, les agents d'halogénation peuvent être choisis parmi des dérivés halogénés du phosphore, notamment les

- composés d'addition d'halogène et de triarylphosphite, ou bien
- le trichlorure de phosphore, l'oxychlorure de phosphore, le pentachlorure de phosphore, le dichlorotriphénylphosphorane ou le catéchyltrichlorophosphorane lorsque $R_3$ est un atome de chlore, ou
- le tribromure de phosphore, l'oxybromure de phosphore, le pentabromure de phosphore ou le catéchyltribromophosphorane lorsque $R_3$ est un atome de brome.

Lorsque l'on utilise le trichlorure (ou le tribromure) de phosphore, on fait agir ce réactif sur un produit de formule générale (XVI) dans laquelle n = 0.

Le catéchyltrichloro (ou tribromo) phosphorane, qui peut être préparé in situ, peut être obtenu selon la méthode décrite par H. Gross et U. Karsch, J. Prakt. Chem., 29, 315 (1965).

Les composés d'addition d'halogène et de triarylphosphite, qui peuvent être formés in situ, sont décrits par H. N. Rydon et B. L. Tonge, J. Chem. Soc., 3043 (1956), par J. Michalski et coll., J. Org. Chem., 45, 3122 (1980) ou dans le brevet belge 881 424 et peuvent être préparés selon les méthodes mentionnées dans ces documents.

La préparation des dérivés halogénés de formule générale (I) s'effectue en milieu anhydre à

partir d'un produit de formule générale (XVI) dans laquelle $R_1$ est protégé s'il contient une fonction acide.

Lorsque le radical $R_1$ est susceptible de former un iminohalogénure il est important de ne pas utiliser un excès de réactif d'halogénation.

La réduction du S-oxyde peut être effectuée dans les conditions décrites dans la demande de brevet allemand 2 637 176.

Le cas échéant, l'élimination des radicaux protecteurs de la fonction amine et des fonctions acides s'effectue en une seule étape ou en 2 étapes.

A titre d'exemple:

1) L'élimination des groupements protecteurs d'amines s'effectue

- lorsqu'il s'agit d'un radical t.butoxycarbonyle, trityle, p.méthoxybenzyloxycarbonyle ou formyle: par traitement en milieu acide. De préférence on utilise l'acide trifluoracétique en opérant à une température comprise entre 0 et 20°C, ou bien on utilise l'acide formique, l'acide orthophosphorique ou polyphosphorique purs ou en présence d'eau, à température comprise entre 20 et 60°C, ou encore l'acide paratoluènesulfonique ou méthylsulfonique dans l'acétone ou dans l'acétonitrile à une température comprise entre 20°C et la température de reflux du mélange réactionnel. Dans ces conditions le produit de formule générale (I) peut être obtenu sous forme de trifluoroacétate, de solvate avec l'acide formique, de méthylsulfonate, de phosphate ou de paratoluène sulfonate, dont on peut libérer la fonction amine par toute méthode connue en soi pour obtenir une amine à partir de l'un de ses sels sans toucher au reste de la molécule. On opère notamment par mise en contact avec une résine échangeuse d'ions ou par action d'une base organique
- lorsqu'il s'agit d'un radical trichloro-2,2,2 éthoxycarbonyle ou p.nitrobenzyloxycarbonyle: par réduction (notamment traitement par le zinc dans l'acide acétique)
- lorsqu'il s'agit d'un radical chloracétyle ou trichloracétyle:par application de la méthode décrite dans le brevet français publié sous le no 2 243 199
- lorsqu'il s'agit d'un radical benzyle, dibenzyle ou benzyloxycarbonyle: par hydrogénation catalytique
- lorsqu'il s'agit d'un radical trifluoracétyle: par traitement en milieu basique.

2) L'élimination des groupements protecteurs de radicaux carboxy s'effectue

- lorsqu'il s'agit d'un groupement t.butyle, p.méthoxybenzyle ou benzhydryle: par traitement en milieu acide, dans les conditions décrites ci-dessus pour l'élimination du radical trityle protecteur d'amino. Dans le cas du radical benzhydryle, on peut opérer en présence d'anisole
- lorsqu'il s'agit d'un groupement méthoxyméthyle:par traitement en milieu acide dilué
- lorsqu'il s'agit d'un groupement nitrobenzyle: par réduction (notamment traitement par le zinc dans l'acide acétique ou hydrogénolyse).

3) L'élimination du groupement protecteur de l'oxime s'effectue

- lorsqu'il s'agit de groupement trityle ou tétrahydropyrannyle: par acidolyse, par exemple par l'acide trifluoracétique, l'acide formique aqueux ou non, ou l'acide paratoluènesulfonique
- lorsqu'il s'agit du groupement méthoxy-2 propyle-2: selon la méthode décrite dans le brevet belge 875 379.

Ib) Selon l'invention, lorsque l'on prépare par le procédé Ia) les produits de formule générale (I) définis ci-dessus en a'1) et b'1) et pour lesquels $R_3$ est un atome de chlore ou de brome, selon les conditions opératoires en peut isoler l'intermédiaire dihalogéné de formule générale:

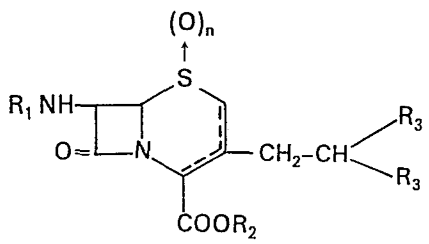

(XVIa)

(dans laquelle $R_1$, $R_2$, $R_3$ et n sont définis comme ci-dessus, étant entendu que $R_2$ ne représente pas l'atome d'hydrogène, lorsque n = 0 le produit se présente sous forme bicyclooctène-2 ou -3 et lorsque n = 1 le produit se présente sous forme bicyclooctène-2) qui peut être alors déhydrohalogéné.

Lorsque l'on veut isoler l'intermédiaire dihalogéné, on opère par action d'un agent d'halogénation dans un solvant organique tel qu'un solvant chloré (par exemple dichlorométhane, chloroforme, tétrachlorure de carbone, dichloro-1,2 éthane), un éther (par exemple éther éthylique, oxyde de propylène, tétrahydrofuranne, dioxanne), un amide (par exemple diméthylacétamide, diméthylpropionamide, diméthylformamide, N-acétylmorpholine, N-acétylpipéridine, N-méthylpyrrolidone) ou dans un mélange de tels solvants, à une température un peu moins élevée que pour préparer le dérivé halogénovinyle correspondant, c'est-à-dire comprise entre −78 et 30°C. Il est également possible d'opérer en présence d'une base telle que la pyridine, dans l'un des solvants cités ci-dessus, à une température comprise entre −78 et 0°C.

La déhydrohalogénation s'effectue en présence d'une base tertiaire telle que définie précédemment, d'une amine aromatique (par exemple pyridine, picoline, quinoléine) ou d'une base minérale (telle que la soude, la potasse, un carbonate ou un bicarbonate alcalin ou alcalino-terreux), en milieu organique ou hydroorganique dans les solvants cités précédemment, à une température

comprise entre -20°C et la température de reflux du mélange réactionnel.

Il n'est pas absolument nécessaire d'avoir purifié l'intermédiaire dihalogéné pour procéder à sa déhydrohalogénation.

Les produits de formule générale (XVI) pour lesquels n = 0 et $R_1$ et $R_2$ sont définis comme précédemment en a'$_1$) ou comme dans la formule générale (I) en 2-, à l'exception pour $R_1$, $R_2$ et $R_4$ de représenter un atome d'hydrogène, peuvent être obtenus par hydrolyse d'une énamine de formule générale:

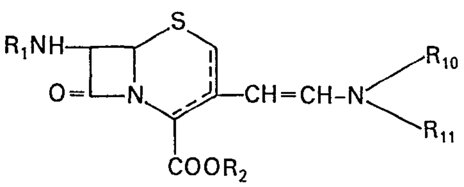
(XVIII)

ou du mélange de ses isomères, [dans laquelle $R_1$ et $R_2$ sont définis comme ci-dessus et $R_{10}$ et $R_{11}$, qui sont identiques ou différents, représentent des radicaux alcoyle (éventuellement substitués par un radical hydroxy, alcoyloxy, amino, alcoylamino ou dialcoylamino) ou phényle, ou forment ensemble avec l'atome d'azote auquel ils sont rattachés un hétérocycle saturé à 5 ou 6 chaînons contenant éventuellement un autre hétéroatome choisi parmi l'azote, l'oxygène ou le soufre et éventuellement substitué par un radical alcoyle, étant entendu que l'énamine de formule générale (XVIII) se présente sous forme bicyclooctène-2 ou -3 et que le substituant sur l'atome de carbone en position -3 du bicyclooctène présente la stéréoisomérie cis ou trans].

De préférence on hydrolyse une énamine de formule générale (XVIII) dans laquelle $R_{10}$ et $R_{11}$ représentent un radical méthyle.

On opère généralement dans un acide organique (par exemple acide formique, acide acétique) ou minéral (par exemple acide chlorhydrique, acide sulfurique) en présence ou non d'un solvant, en milieu aqueux ou organique, à une température comprise entre -20°C et la température de reflux du mélange réactionnel, puis traite éventuellement par une base minérale (bicarbonate alcalin) ou organique (amine tertiaire ou pyridine).

Lorsque l'on opère en milieu organique, l'hydrolyse est réalisée par addition d'eau au mélange réactionnel; lorsque l'on opère en présence d'un solvant, il n'est pas nécessaire que le solvent soit miscible à la phase aqueuse acide: le contact est alors réalisé par agitation vive.

Parmi les solvants utilisables peuvent être cités les solvants chlorés, l'acétate d'éthyle, le tétrahydrofuranne, l'acétonitrile, le diméthylformamide, les alcools. Il n'est pas absolument nécessaire de purifier le produit de formule générale (XVI) obtenu pour le mettre en œuvre pour la préparation des produits de formule générale (I).

Lorsque l'on veut préparer un produit de formule générale (XVI) dans laquelle $R_1$ contient un radical de formule générale (IIa) dont la fonction acide est libre, il est nécessaire d'utiliser une énamine de formule générale (XVIII) dans laquelle les radicaux protecteurs d'acide $R_2$ et $R^c_5$ (dans $R_1$) sont différents et éliminables sélectivement. L'élimination du radical protecteur $R^c_5$ s'effectue dans les conditions décrites précédemment.

Les produits de formule générale (XVI), pour lesquels n = 1, peuvent être obtenus par oxydation des produits de formule générale (XVI) pour lesquels n est égal à 0, par application de la méthode décrite dans la demande de brevet allemand DE 2 637 176.

Les produits de formule générale (XVIII) pour lesquels $R_{10}$ et $R_{11}$ ont les définitions données précédemment (à l'exception de représenter alcoyle substitué par hydroxy, amino ou alcoylamino) peuvent être obtenus par action d'un produit éventuellement préparé in situ de formule générale:

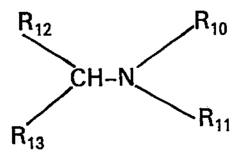
(XIX)

{dans laquelle $R_{10}$ et $R_{11}$ sont définis comme précédemment et $R_{12}$ et $R_{13}$, qui sont identiques ou différents, soit représentent des groupements de formule générale:

$$-X_2R_{14} \quad \text{(XX)}$$

(dans laquelle $X_2$ est un atome d'oxygène et $R_{14}$ représente un radical alcoyle ou phényle), soit représentent l'un un radical de formule générale (XX) dans laquelle $X_2$ est oxygène ou soufre et l'autre un radical amino de formule générale:

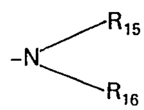
(XXI)

[dans laquelle $R_{15}$ et $R_{16}$ sont définis comme $R_{10}$ et $R_{11}$ dans la formule générale (XIX)], soit encore représentent chacun des radicaux de formule générale (XXI)} sur un dérivé de céphalosporine de formule générale:

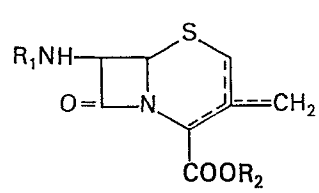
(XXII)

dans laquelle, $R_1$ et $R_2$ étant définis comme en a'$_1$ ou comme en (2-) pour la formule générale (I) (à l'exception pour $R_1$, $R_2$ et $R_4$ de représenter un

atome d'hydrogène), le dérivé se présente sous forme méthyl-3 bicyclooctène-2 ou -3 ou méthylène-3 bicyclooctane.

On opère généralement dans un solvant organique tel que le diméthylformamide, l'hexaméthylphosphorotriamide, le diméthylacétamide, l'acétonitrile, l'acétate d'éthyle, le dioxanne ou un solvant chloré (par exemple dichloro-1,2 éthane) ou dans un mélange de tels solvants à une température comprise entre 20°C et la température de reflux du mélange réactionnel.

Lorsque l'on choisit un produit de formule générale (XIX) dans laquelle le radical (XXI) est différent de $-N\ R_{10}R_{11}$, il est préférable de choisir un tel produit de manière que l'amine $HN\ R_{15}R_{16}$ soit plus volatile que $HN\ R_{10}R_{11}$.

Les produits de formule générale (XVIII) dans laquelle $R_{10}$ et $R_{11}$, qui sont identiques ou différents, représentent des radicaux alcoyle substitués par hydroxy, amino ou alcoylamino peuvent être obtenus par transénamination à partir d'un produit de formule générale (XVIII) dans laquelle $R_{10}$ et $R_{11}$ représentent des radicaux alcoyle, de préférence méthyle.

La réaction s'effectue par action d'une amine de formule générale:

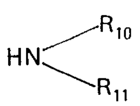

(XXIII)

(dans laquelle $R_{10}$ et $R_{11}$ ont les définitions correspondantes) sur le produit de formule générale (XVIII), et l'on opère dans des conditions analogues à celles décrites précédemment pour l'action d'un produit de formule générale (XIX) sur un dérivé de formule générale (XXII).

Les produits de formule générale (XIX) peuvent être préparés selon les méthodes décrites par H. Bredereck et coll., Chem. Ber. 101, 41 (1968), Chem. Ber. 101, 3058 (1968) et Chem. Ber. 106, 3725 (1973).

Les dérivés de la céphalosporine de formule générale (XXII) dans laquelle $R_1$ représente un radical de formule (II) peuvent être préparés à partir des produits de formule générale:

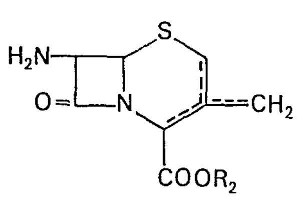

(XXIV)

[dans laquelle $R_2$ est défini comme ci-dessus et la position de la double liaison est définie comme pour le produit de formule générale (XXII)] par action d'un acide de formule générale:

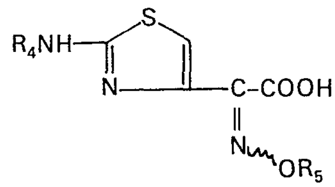

(XXV)

[dans laquelle les différents symboles sont définis comme précédemment à l'exception pour $R_4$ et $R^c_5$ (dans la définition de $R_5$) de représenter l'hydrogène], ou d'un dérivé réactif de cet acide. Il est entendu que l'acide de formule générale (XXV) sous forme syn, anti ou leurs mélanges, conduit respectivement aux produits de formule générale (XXII) de forme syn, anti ou leurs mélanges.

Généralement on effectue la condensation de l'acide de formule générale (XXV) sur l'amino-7 céphalosporine de formule générale (XXIV), dans un solvant organique tel que le diméthylformamide, l'acétonitrile, le tétrahydrofuranne, le chloroforme ou le dichlorométhane, en présence d'un agent de condensation tel qu'un carbodiimide (par exemple le dicyclohexylcarbodiimide), le N,N'-carbonyldiimidazole ou l'éthoxy-2 éthoxycarbonyl-1 dihydro-1,2 quinoléine, à une température comprise entre -20 et 40°C.

Lorsque l'on utilise un dérivé réactif de l'acide de formule générale (XXV), il est possible de mettre en œuvre l'anhydride, un anhydride mixte ou un ester réactif de formule générale:

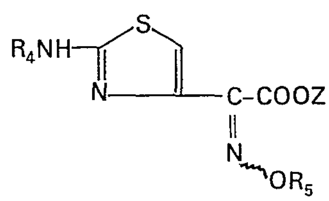

(XXVI)

[dans laquelle, les symboles $R_4$ et $R_5$ étant définis comme ci-dessus, Z représente un radical succinimido, benzotriazolyle-1, nitro-4 phényle, dinitro-2,4 phényle, pentachlorophényle ou phtalimido], ou bien des dérivés réactifs tels qu'un thioloester défini ci-après par la formule générale (XLV) ou un halogénure d'acide, par exemple le chlorure de l'acide de formule générale (XXV).

Lorsque l'on met en œuvre l'anhydride, un anhydride mixte ou un halogénure d'acide (qui peuvent être préparés in situ), on effectue la condensation dans un solvant organique inerte tel qu'un éther (par exemple tétrahydrofuranne ou dioxanne), un solvant chloré (par exemple chloroforme ou chlorure de méthylène), un amide (par exemple diméthylformamide ou diméthylacétamide) ou une cétone (par exemple l'acétone), ou dans des mélanges de tels solvants, en présence d'un accepteur d'acide tel qu'un époxyde (par exemple l'oxyde de propylène) ou tel qu'une base organique azotée comme la pyridine, la diméthylaminopyridine, la N-méthylmorpholine ou une trialcoylamine (par exemple triéthylamine), ou dans un milieu hydroorganique en présence d'un

agent alcalin de condensation tel que le bicarbonate de sodium, et l'on opère à une température comprise entre -40 et 40°C. Il est également possible de mettre en œuvre une céphalosporine de formule générale (XXIV) préalablement silylée par application de la méthode décrite dans la demande de brevet allemand 2 804 040.

Lorsque l'on met en œuvre un ester réactif de formule générale (XXVI) ou un thioloester, on opère généralement en présence d'une trialcoylamine (par exemple triéthylamine) dans un solvant organique tel que le diméthylformamide, à une température comprise entre 0 et 40°C.

Les dérivés de la céphalosporine de formule générale (XXII) et (XXIV), dans lesquelles $R_2$ représente un radical de formule générale (V), peuvent être obtenus par estérification de l'acide correspondant par toute méthode connue en soi pour préparer un ester à partir d'un acide, sans toucher au reste de la molécule.

Généralement on fait réagir un sel alcalin ou un sel d'amine tertiaire d'un produit de formule générale:

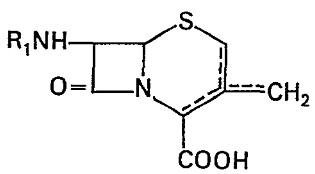

(XXVII)

(dans laquelle $R_1$ est défini comme ci-dessus) ou

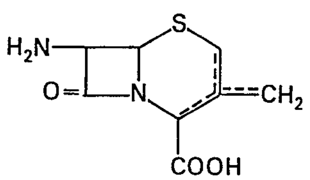

(XXVIII)

[dans lesquelles la position de la double liaison est définie comme pour les produits de formule générale (XXII) et (XXIV) et le cas échéant la fonction amine du radical $R_1$ est protégée], sur un halogénure de formule générale:

$$X\text{-}\underset{\displaystyle R_9}{\underset{|}{C}H}\text{-}OCO\ R_8 \qquad (XXIX)$$

dans laquelle $R_9$ et $R_{10}$ sont définis comme précédemment et X représente un atome d'halogène, dans un solvant inerte tel que le diméthylformamide, à une température comprise entre 0 et 30°C.

Les produits de formule générale (XXIX) peuvent être préparés selon la méthode décrite dans la demande de brevet allemand 2 350 230.

L'introduction des groupements protecteurs $R_1$ et/ou $R_2$ des produits de formule générale (XXII) pour lesquels $R_1$ et $R_2$ sont définis comme précédemment en 1-a) [à l'exception pour $R_1$ de représenter un radical de formule générale (II) et pour $R_2$ de représenter un radical de formule générale (V)] et des produits de formule générale (XXIV) pour lesquels $R_2$ est défini comme précédemment en 1-a) [à l'exception de représenter un radical de formule générale (V)] peut être effectuée sur une céphalosporine respectivement de formule générale (XXIV), (XXVII) ou (XXVIII) selon l'une des méthodes décrites dans les références suivantes:

- lorsque $R_1$ est un radical formyle: selon J. C. Sheehan et coll., J. Amer. Chem. Soc. 80, 1156 (1958),
- lorsque $R_1$ est acétyle, chloracétyle, trichloracétyle, phénylacétyle, phénoxyacétyle ou benzoyle: selon E. H. Flynn, Cephalosporins and Penicillins, Ac. Press (1972),
- lorsque $R_1$ est un radical t.butoxycarbonyle: selon L. Moroder et coll., Hoppe Seyler's Z. Physiol. Chem. 357, 1651 (1976),
- lorsque $R_1$ est trichloro-2,2,2 diméthyl-1,1 éthoxycarbonyle: selon J. Ugi et coll., Angew. Chem. Int. Ed. Engl. 17 (5), 361 (1978),
- lorsque $R_1$ est trichloro-2,2,2 éthoxycarbonyle, chloro-2 diméthyl-1,1 éthoxycarbonyle, cyano-2 diméthyl-1,1 éthoxycarbonyle, triméthylsilyl-2 éthoxycarbonyle, benzyloxycarbonyle, p.méthoxybenzyloxycarbonyle, diméthoxy-3,5 benzyloxycarbonyle, p.nitrobenzyloxycarbonyle, vinyloxycarbonyle: par action d'un chloroformiate en milieu hydroorganique en présence d'un bicarbonate alcalin, ou selon le brevet belge 788 885,
- lorsque $R_1$ est diphénylméthoxycarbonyle: par action de l'azidoformiate correspondant en milieu hydroorganique, en présence d'un bicarbonate alcalin,
- lorsque $R_1$ est (biphénylyl-4)-2 isopropyloxycarbonyl: par analogie avec la méthode décrite dans Helv. Chim. Acta, 51, 924 (1968),
- lorsque $R_1$ est quinolyl-8 oxycarbonyle ou allyloxycarbonyle: par action du carbonate correspondant en milieu hydroorganique basique,
- lorsque $R_1$ est o.nitrophénylthio ou p.nitrophénylthio: par analogie avec la méthode décrite par L. Zervas et coll., J. Amer. Chem. Soc. 85, 3660 (1963),
- lorsque $R_1NH$ est remplacé par diméthylaminométhylèneamino: par analogie avec la méthode décrite par J. F. Fitt, J. Org. Chem. 42 (15), 2639 (1977),
- lorsque $R_1NH$ est remplacé par nitro-4 benzylidèneamino ou diméthoxy-3,4 benzylidèneamino: selon la méthode décrite par R. A. Firestone, Tetrahedron Lett., 375 (1972),
- lorsque $R_2$ est méthoxyméthyle: selon S. Seki et coll., Tetrahedron Lett., 33, 2915 (1977),
- lorsque $R_2$ est t.butyle: selon R. J. Stedman, J. Med. Chem. 9, 444 (1966)
- lorsque $R_2$ est benzhydryle: selon la demande de brevet néerlandais 7 303 263,
- lorsque $R_2$ est benzyle, nitrobenzyle ou p.méthoxybenzyle: selon R. R. Chauvette et coll., J. Org. Chem. 38 (17), 2994 (1973).

Les dérivés de céphalosporine de formule générale (XXII) dans laquelle $R_1$ et $R_2$ sont définis comme précédemment en 1-b) peuvent être préparés par acylation d'une amino-7 céphalosporine de formule générale (XXIV) selon les méthodes décrites dans le brevet US 4 065 620.

Les acides de formule générale (XXV) dans laquelle $R_5$ est hydrogène, alcoyle ou trityle peuvent être préparés selon la méthode décrite dans le brevet BE 850 662.

Les acides de formule générale (XXV) dans laquelle $R_5$ est un radical vinyle peuvent être préparés selon la méthode décrite dans le brevet BE 869 079.

Les acides de formule générale (XXV) dans laquelle $R_5$ est un radical cyanométhyle peuvent être préparés selon la méthode décrite dans la demande de brevet allemand 2 812 625.

Les acides de formule générale (XXV) dans laquelle $R_5$ représente un radical de formule (IIa) peuvent être préparés selon la méthode décrite dans les brevets belges 864 810, 865 298, 876 541 et 876 542.

Les acides de formule générale (XXV) dans laquelle $R_5$ est un radical protecteur peuvent être préparés par protection de l'oxime d'un tel acide dans lequel $R_5$ est hydrogène, par toute méthode connue qui n'altère pas le reste de la molécule. La protection s'effectue notamment par les groupements trityle, tétrahydropyrannyle ou méthoxy-2 propyle-2.

II) Selon l'invention, les produits de formule générale (I), dans laquelle n est défini comme précédemment, $R_3$ est un atome d'halogène tel que défini dans la formule générale (I), $R_1$ représente un atome d'hydrogène et $R_2$ est défini comme dans la formule générale (I) peuvent être obtenus par élimination du radical $R_1$, ou éventuellement élimination simultanée des radicaux $R_1$ et $R_2$, d'un produit de formule générale (I) [dans laquelle $R_1$ est défini comme dans la formule générale (I) en 1-a) à l'exception de représenter un radical de formule générale (II) ou représente un radical amino-5 adipoyle dont les fonctions amine et acide sont protégées, ou un radical de formule générale (VI) ou (VII) tel que défini pour $R_1$ en 1-b) dans la formule générale (I) et $R_2$ a une définition correspondante].

L'élimination du radical protecteur $R_1$ s'effectue par toute méthode connue pour libérer une fonction amine sans toucher au reste de la molécule.

A titre d'exemple, on peut citer les méthodes suivantes:

- lorsque $R_1$ représente trityle, benzhydryle, trichloracétyle, chloracétyle, t.butoxycarbonyle, trichloréthoxycarbonyle, benzyloxycarbonyle, p.méthoxybenzyloxycarbonyle ou p.nitrobenzyloxycarbonyle: selon les méthodes citées ci-dessus pour la libération du radical amino du produit de formule générale (I). L'élimination du radical t.butoxycarbonyle s'effectue avantageusement par utilisation de l'acide p.toluènesulfonique ou méthanesulfonique dans l'acétonitrile à une température comprise entre 0 et 50°C,
- lorsque $R_1$ représente formyle, chloro-2 diméthyl-1,1 éthoxycarbonyle, cyano-2 diméthyl-1,1, éthoxycarbonyle, diméthoxy-3,5 benzyloxycarbonyle, diphénylméthoxycarbonyle, (biphénylyl-4)-2 isopropyloxycarbonyle, vinyloxycarbonyle, allyloxycarbonyle, quinolyl-8 oxycarbonyle, o.nitrophénylthio, p.nitrophénylthio, ou lorsque $R_1NH$ est remplacé par diméthylaminométhylène-amino, diméthoxy-3,4 benzylidène-amino ou nitro-4 benzylidène-amino: par hydrolyse en milieu acide,
- lorsque $R_1$ représente trichloro-2,2,2 éthyle ou trichloro-2,2,2 diméthyl-1,1 éthoxycarbonyle: par traitement par le zinc dans l'acide acétique,
- lorsque $R_1$ représente acétyle, benzoyle, phénylacétyle, phénoxyacétyle ou amino-5 adipoyle protégé: selon la méthode décrite dans le brevet belge BE 758 800,
- lorsque $R_1$ représente triméthylsilyléthoxycarbonyle: selon la méthode décrite par H. Gerlach, Helv. Chim. Acta 60 (8), 3039 (1977),
- lorsque $R_1$ représente p.nitrobenzyloxycarbonyle: par hydrogénolyse en présence de palladium.

III) Selon l'invention, les produits de formule générale (I) [dans laquelle n est défini comme précédemment, $R_1$ $R_2$ et $R_3$ sont définis comme précédemment en 2-, ou bien $R_1$ est un radical de formule générale (II) ou un radical tel que défini en 1-b) à l'exception de représenter l'atome d'hydrogène, $R_2$ a une définition correspondante et $R_3$ est un atome d'halogène] peuvent être préparés par action d'un acide représenté par la formule générale:

$$R_1OH \qquad (XXX)$$

dans laquelle $R_1$ est défini comme ci-dessus, ou d'un dérivé réactif de cet acide, sur un produit de formule générale (I) dans laquelle $R_1$ est un atome d'hydrogène et $R_2$ et $R_3$ sont définis comme précédemment en 1- ou 2-, ou le cas échéant sur un mélange des isomères de ce produit, suivie éventuellement de la réduction de l'oxyde obtenu puis éventuellement de l'élimination des radicaux protecteurs.

On opère par analogie avec la méthode décrite précédemment pour l'obtention d'un produit de formule générale (XXII) à partir de produits de formules générales (XXIV) et (XXV), ou selon les méthodes citées dans le brevet US 4 065 620.

Il est entendu que les conditons de protection de $R_1$ sont les mêmes que pour la réaction des produits (XXIV) avec les produits (XXV).

Le cas échéant la réduction de l'oxyde, ainsi que l'élimination des radicaux protecteurs de la fonction amine et des fonctions acides, peuvent être effectuées dans les conditions décrites pour obtenir le produit de formule générale (I) à partir d'un produit de formule générale (XVI).

IV) Selon l'invention, les produits de formule générale (I) dans laquelle n = 1 peuvent être préparés par oxydation du produit correspondant de fomule générale (I) dans laquelle n = 0.

L'oxydation peut être effectuée par toute méthode connue qui n'altère pas le reste de la molécule, notamment par application de la méthode décrite dans le brevet allemand DE 2 637 176.

V) Les produits de formule générale (I) [pour lesquels, n étant défini comme précédemment, $R_1$ et $R_3$ sont définis comme précédemment en 2- étant entendu que le radical $R^c{}_5$ du groupement de formule générale (IIa) contenu dans $R_1$ est autre que l'atome d'hydrogène, ou $R_1$ est défini comme en 1-a) et $R_3$ est un atome d'halogène, et $R_2$ est un radical de formule générale (V)], peuvent être obtenus par estérification du produit correspondant de formule générale (I) dans laquelle $R_2$ est un atome d'hydrogène, par toute méthode connue pour préparer un ester à partir d'un acide sans toucher au reste de la molécule.

On opère généralement par action d'un sel alcalin ou d'un sel d'amine tertiaire du produit de formule générale (I) sur un halogénure de formule générale (XXIX), dans les conditions décrites précédemment pour la préparation de produits de formules générales (XXII) ou (XXIV) dans lesquelles $R_2$ est un radical de formule générale (V).

VI) Selon l'invention, les produits de formule générale (I) dans laquelle n est défini comme précédemment,

$R_1$, $R_2$ et $R_3$ sont définis comme en 2- (à l'exception pour $R_4$ de représenter chloracétyle ou trichloracétyle) ou bien

$R_1$ représente un radical de formule générale (II) définie comme en 1-a) (à l'exception pour $R_4$ de représenter chloracétyle ou trichloracétyle et pour $R_5$ de représenter vinyle), $R_2$ est défini comme en 1-a) et

$R_3$ est défini comme précédemment en 1-, peuvent également être préparés par action d'une thiourée de formule générale:

$R_4NH\text{-}CS\text{-}NH_2$ (XXXI)

dans laquelle $R_4$ est défini comme ci-dessus, sur un produit ou un mélange des isomères d'un produit de formule générale:

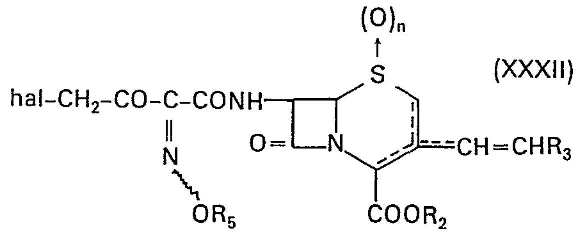

(XXXII)

dans laquelle $R_2$, $R_3$, $R_5$ et n sont définis comme ci-dessus et hal représente un atome de chlore ou de brome, suivie éventuellement de la réduction du sulfoxyde obtenu (lorsque n = 1), et éventuellement de l'élimination des radicaux protecteurs.

On opère généralement en milieu aqueux, organique ou hydroorganique, par exemple dans des solvants ou des mélanges de solvants tels que les alcools (méthanol, éthanol), les cétones (acétone), les solvants chlorés (chloroforme, chlorure d'éthylène), les nitriles (acétonitrile), les amides (diméthylformamide, diméthylacétamide), les éthers (tétrahydrofuranne, dioxanne), les esters (acétate d'éthyle) ou les acides (acide acétique, acide formique), en présence ou non d'une base telle que la soude, la potasse, les carbonates, les carbonates acides des métaux alcalins, les sels d'acides carboxyliques et de métaux alcalins (formiate de sodium, acétate de sodium) ou les amines tertiaires (triéthylamine, triméthylamine ou pyridine), à une température comprise entre -30 et 60°C.

Lorsque l'on opère en présence d'une base, selon la nature de celle-ci et la quantité introduite, on isole ou non l'intermédiaire de formule générale:

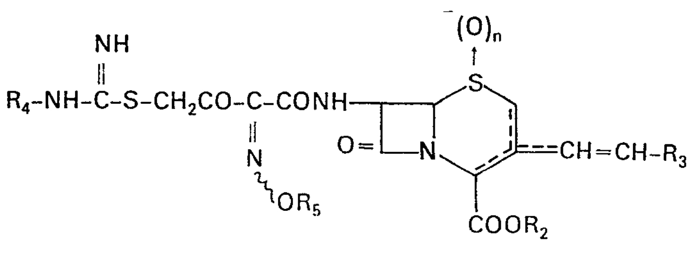

(XXXIII)

(dans laquelle $R_2$, $R_3$, $R_4$, $R_5$ et n sont définis comme ci-dessus), qui peut être alors cyclisé en milieu acide.

La réduction du sulfoxyde et l'élimination des radicaux protecteurs s'effectuent dans les conditions décrite précédemment.

Les produits de formule générale (XXXII) dans laquelle $R_5$ est défini comme ci-dessus à l'exception de représenter un atome d'hydrogène peuvent être obtenus par action d'un halogénure d'acide de formule générale:

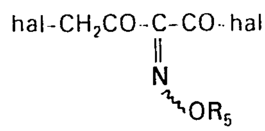

(XXXIV)

dans laquelle hal et hal' sont des atomes de chlore ou de brome et $R_5$ est défini comme ci-dessus (étant entendu que lorsque $R_5$ contient une fonc-

tion acide, cette dernière est protégée) sur une amino-7 céphalosporine de formule générale (I) dans laquelle $R_1$ représente un atome d'hydrogène et $R_2$ a la définition donnée ci-dessus, suivie de la réduction du sulfoxyde obtenu (lorsque n = 1) et de l'élimination des radicaux protecteurs.

La réaction s'effectue généralement en milieu hydroorganique, par exemple eau-éther (tétrahydrofuranne, dioxanne), eau-cétone (acétone) ou eau-solvant chloré (chloroforme, chlorure de méthylène), en présence d'un agent alcalin de condensation tel qu'un bicarbonate alcalin (par exemple bicarbonate de sodium) à une température comprise entre -40 et 40°C.

Il est également possible d'opérer par analogie avec la méthode décrite dans la demande de brevet français 2 399 418.

Les produits de formule générale (XXXIV) peuvent être obtenus par halogénation d'un produit de formule générale:

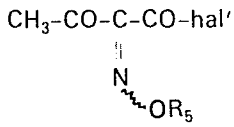

(XXXV)

dans laquelle $R_5$ et hal' sont définis comme ci-dessus pour la formule générale (XXXIV) par toute méthode connue en soi pour la préparation de dérivés halogénés, qui n'altère pas le reste de la molécule.

Lorsque l'on veut obtenir un produit de formule générale (XXXIV) dans laquelle hal représente un atome de brome, on fait agir le brome en présence d'un catalyseur, soit un catalyseur acide tel que l'acide bromhydrique, l'acide chlorhydrique, les acides sulfoniques (acide méthanesulfonique, acide p.toluènesulfonique anhydre ou acide benzènesulfonique), soit en présence de lumière ultra-violette.

Lorsque l'on veut obtenir un produit de formule générale (XXXIV) dans laquelle hal est un atome de chlore, on fait agir le chlore en présence d'un catalyseur tel que cité ci-dessus ou le chlorure de sulfuryle.

L'halogénation s'effectue dans un solvant organique tel qu'un solvant chloré (par exemple dichlorométhane, chloroforme, tétrachlorure de carbone, dichloroéthane ou trichloroéthane) ou un éther (par exemple éther éthylique ou dioxanne) ou dans un mélange de tels solvants, à une température comprise entre -40°C et la température de reflux du mélange réactionnel.

Les produits de formule générale (XXXV) peuvent être préparés à partir des acides et des esters correspondants, selon la méthode décrite dans la demande de brevet français 2 414 508.

Les esters peuvent être eux-mêmes préparés par application de la méthode décrite par R. Bucourt et coll., Tetrahedron, 34, 2233 (1978).

Les acides de formule générale:

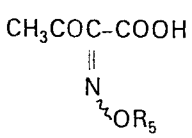

(XXXVa)

(dans laquelle $R_5$ est un radical $-C\,R^a_5\,R^b_5-COOR^c_5$ pour lequel $R^c_5$ est autre qu'un atome d'hydrogène) peuvent être préparés par action d'un produit de formule générale:

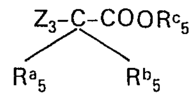

(XXXVI)

dans laquelle $R^a_5$, $R^b_5$ et $R^c_5$ sont définis comme précédemment à l'exception pour $R^c_5$ de représenter un atome d'hydrogène, et $Z_3$ représente un atome d'halogène ou un radical sulfate ou sulfonate, sur un produit de formule générale:

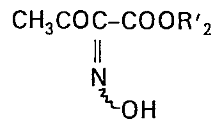

(XXXVb)

dans laquelle $R'_2$ est un radical protecteur d'acide tel que défini précédemment pour $R_2$, puis élimination du radical protecteur $R'_2$.

La réaction s'effectue généralement en présence d'une base minérale ou organique (par exemple la soude, la potasse, l'hydrure de sodium, le carbonate de potassium ou une base azotée telle que la triéthylamine) dans un solvant organique tel qu'un solvant chloré (par exemple dichlorométhane, dichloroéthane), un éther (par exemple tétrahydrofuranne, dioxanne), une cétone (par exemple acétone) ou un amide (par exemple diméthylformamide).

Il est nécessaire que les radicaux protecteurs d'acide ($R'_2$ et $R^c_5$) soient différents et éliminables sélectivement.

L'élimination du radical protecteur R'2 s'effectue dans les conditions décrites précédemment.

Les produits de formule générale (XXXII) dans laquelle $R_5$ est un atome d'hydrogène peuvent être obtenus par nitrosation d'un produit de formule générale:

(XXXVII)

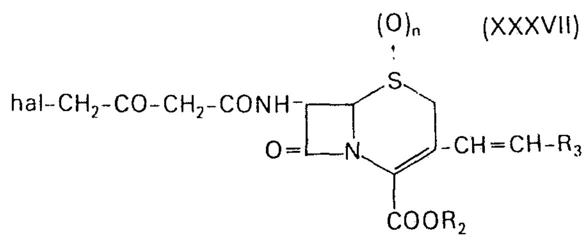

dans laquelle $R_2$, $R_3$, hal et n sont définis comme ci-dessus, par analogie avec la méthode décrite dans la demande de brevet français 2 399 418, puis éventuellement réduction du sulfoxyde et élimination des radicaux protecteurs.

Les produits de formule générale (XXXII) dans laquelle $R_5$ est un radical protecteur peuvent être obtenus par protection de l'oxime d'un produit de formule générale (XXXII) pour lequel $R_5$ est un atome d'hydrogène.

Les produits de formule générale (XXXVII) peuvent être obtenus à partir d'une amino-7 céphalosporine de formule générale (I) dans laquelle $R_2$

a la définition donnée ci-dessus et $R_1$ représente un atome d'hydrogène par action d'un produit de formule générale:

$$hal-CH_2-CO-CH_2-CO-hal \quad (XXXIVa)$$

dans laquelle hal est défini comme précédemment (qui peut être formé in situ), en opérant dans les conditions décrites précédemment pour condenser un produit de formule générale (XXXIV) avec un produit de formule générale (I) ou par analogie avec la méthode décrite dans la demande de brevet français 2 399 418.

Les nouveaux produits de formule générale (I) sont utiles comme intermédiaires pour la préparation de thiovinyl-3 céphalosporines de formule générale:

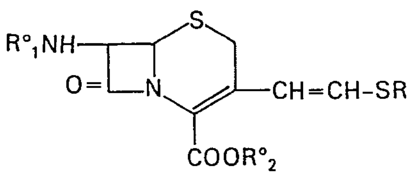

(XXXVIII)

dans laquelle

$\alpha_1$) le symbole R est choisi parmi:

1) alcoyle, L-amino-2 carboxy-2 éthyle, phényle,

2) pyridyle-2, pyridyle-3 ou pyridyle-4 éventuellement N-oxydés,

3) pyrimidinyle-2, pyridazinyle-3 substitué en position -6 (par un radical alcoyle, méthoxy, amino ou acylamino) et éventuellement N-oxydé ou tétrazolo[4,5-b]pyridazinyle-6,

4) dioxo-5,6 tétrahydro-1,4,5,6 triazine-1,2,4 yle-3 substitué en position -4, triazol-1,3,4 yle-5 ou alcoyloxycarbonyl-2 triazol-1,3,4 yle-5 substitués en position -1:

a) par un radical alcoyle contenant 1 à 4 atomes de carbone non substitué ou substitué par un radical alcoyloxy, alcoylthio, phényle, formyle, carbamoyle, alcoylcarbamoyle, dialcoylcarbamoyle, acyle, alcoyloxycarbonyle ou thiazolidinyle-2,

b) par un radical allyle, dihydroxy-2,3 propyle; dihydroxy-1,3 propyle-2; formyl-2 hydroxy-2 éthyle; formyloxy-3 hydroxy-2 propyle; bis-formyloxy-2,3 propyle ou bis-formyloxy-1,3 propyle-2,

c) par un radical alcoyle contenant 2 à 4 atomes de carbone, substitué par hydroxy, carbamoyloxy, acyloxy (dont la partie acyle peut être substituée par un radical amino, alcoylamino ou dialcoylamino), alcoylsulfinyle, alcoylsulfonyle, amino, alcoylamino, dialcoylamino, sulfoamino, alcoylsulfonylamino, sulfamoylamino, acylamino (dont la partie acyle est éventuellement substituée par hydroxy, amino, alcoylamino ou dialcoylamino), alcoyloxycarbonylamino, uréido, alcoyluréido, dialcoyluréido,

d) par un radical répondant à l'une des formules générales

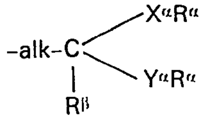

(XXXIXa)

ou 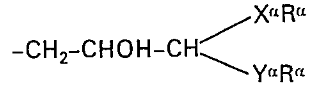 (XXXIXb)

ou 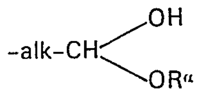 (XXXIXc)

dans laquelle alk est un radical alcoylène contenant 1 à 4 atomes de carbone, $X^\alpha$ et $Y^\alpha$ sont identiques et représentent des atomes d'oxygène ou de soufre, et $R^\alpha$ représente un radical alcoyle, ou bien $X^\alpha$ et $Y^\alpha$ sont identiques ou différents et représentent des atomes d'oxygène ou de soufre, et les radicaux $R^\alpha$ forment ensemble un radical alcoylène contenant 2 ou 3 atomes de carbone, et $R^\beta$ représente un atome d'hydrogène, un radical alcoyle contenant 1 à 3 atomes de carbone,

e) par un radical alcoyle contenant 1 à 5 atomes de carbone substitué par un radical alcoyloxyimino ou hydroxyimino,

5) dialcoyl-1,4 dioxo-5,6 tétrahydro-1,4,5,6 triazine-1,2,4 yle-3, alcoyl-1 dioxo-5,6 tétrahydro-1,4,5,6 triazine-1,2,4 yle-3 ou alcoyl-2 dioxo-5,6 tétrahydro-1,2,5,6 triazine-1,2,4 yle-3,

6) triazol-1,3,4 yle-5, triazol-1,2,3 yle-5 ou alcoyl-1 triazol-1,2,4 yle-5 non substitué ou substitué en position -3 par alcoyloxycarbonyle,

7) a) thiadiazol-1,3,4 yle-5 non substitué ou substitué par un radical alcoyle, trifluorométhyle, alcoyloxy, alcoylthio, hydroxyalcoylthio dont la partie alcoyle contient 2 à 4 atomes de carbone, alcoylsulfonyle, hydroxy, hydroxyalcoyle, carboxy, carboxyalcoyle, amino, alcoylamino, dialcoylamino, aminoalcoyle, alcoylaminoalcoyle, dialcoylaminoalcoyle, acylamino ou acylaminoalcoyle,

b) thiadiazol-1,2,4 yle-5 substitué par un radical alcoyle ou alcoyloxy,

8) a) oxadiazol-1,3,4 yle-5 non substitué ou substitué par un radical alcoyle, trifluorométhyle, phényle, aminoalcoyle, alcoylaminoalcoyle, dialcoylaminoalcoyle ou acylaminoalcoyle

b) oxazolyle-2 ou alcoyl-4 oxazolyle-2,

9) tétrazolyle-5 non substitué ou substitué en position -1 par

a) un radical alcoyle contenant 1 à 4 atomes de carbone non substitué ou substitué par alcoyloxy, sulfo, carboxy, formyle ou sulfamoyle,

b) un radical alcoyle contenant 2 à 4 atomes de carbone substitué par hydroxy, amino, alcoylamino, dialcoylamino, acylamino, carboxyalcoylamino, sulfamoylamino, sulfoamino, uréido, alcoyluréido ou dialcoyluréido,

c) un radical alcoyle contenant 1 à 5 atomes de carbone substitué par hydroxyimino ou alcoyloxyimino,

d) un radical phényle, dihydroxy-2,3 propyle, dihydroxy-1,3 propyle-2; formyl-2 hydroxy-2 éthyle; formyloxy-3 hydroxy-2 propyle; bisformyloxy-2,3 propyle ou bisformyloxy-1,3 propyle-2, ou

e) un radical de formule générale (XXXIXa) pour lequel $R^\beta$ est un atome d'hydrogène, ou un radical de formule générale (XXXIXb),

10) dioxo-5,6 tétrahydro-1,4,5,6 triazine-1,2,4 yle-3 substitué en position -1, dioxo-5,6 tétrahydro-1,2,5,6 triazine-1,2,4 yle-3 substitué en position -2, triazol-1,2,4 yle-5 ou alcoyloxycarbonyl-3 triazol-1,2,4 yle-5 substitués en position -1

a) par un radical alcoyle substitué par un radical alcoyloxy, alcoylthio, phényle, formyle, carbamoyle, alcoylcarbamoyle, dialcoylcarbamoyle, hydroxyalcoylcarbamoyle (dont la partie alcoyle contient 2 à 4 atomes de carbone), acyle alcoyloxycarbonyle ou thiazolidinyle-2,

b) par un radical allyle, dihydroxy-2,3 propyle; dihydroxy-1,3 propyle-2; formyl-2 hydroxy-2 éthyle; formyloxy-3 hydroxy-2 propyle; bis-formyloxy-2,3 propyle ou bis-formyloxy-1,3 propyle-2,

c) par un radical alcoyle contenant 2 à 4 atomes de carbone, substitué par hydroxy, carbamoyloxy, acyloxy (dont la partie acyle peut être substituée par un radical amino, alcoylamino ou dialcoylamino), alcoylsulfinyle, alcoylsulfonyle, amino, alcoylamino, dialcoylamino, sulfoamino, alcoylsulfonylamino, sulfamoylamino, acylamino (dont la partie acyle est éventuellement substituée par hydroxy, amino, alcoylamino ou dialcoylamino), alcoyloxycarbonylamino, uréido, alcoyluréido, dialcoyluréido,

d) par un radical répondant à l'une des formules générales (XXXIXa), (XXXIXb) ou (XXXIXc) telles que définies précédemment,

e) par un radical alcoyle contenant 2 à 5 atomes de carbone substitués par un radical alcoyloxyimino ou hydroxyimino,

11) dioxo-5,6 hydroxyalcoylcarbamoylalcoyl-4 tétrahydro-1,4,5,6 triazine-1,2,4 yle-3 dont la portion hydroxyalcoyle contient 2 à 4 atomes de carbone,

12) alcoyl-1 dioxo-5,6 tétrahydro-1,4,5,6 triazine-1,2,4 yle-3 substitué en position -4, alcoyl-1 dioxo-5,6 tétrahydro-1,2,5,6 triazine-1,2,4 yle-3 substitué en position -2, alcoyl-2 dioxo-5,6 tétrahydro-1,2,5,6 triazine-1,2,4 yle-3 substitué en position -1 ou alcoyl-4 dioxo-5,6 tétrahydro-1,4,5,6 triazine-1,2,4 yle-3 substitué en position -1 par un radical formylalcoyle ou par un radical de formule générale (XXXIXa) pour lequel $R^{\beta}$ est un atome d'hydrogène,

le symbole $R°_1$ représente un radical de formule générale (II) [dans laquelle $R_5$ est hydrogène, alcoyle, vinyle, cyanométhyle ou un radical de formule générale (IIa), $R^a_5$ et $R^b_5$ sont définis comme précédemment et $R^c_5$ et $R_4$ sont des atoms d'hydrogène] et le symbole $R°_2$ représente un atome d'hydrogène ou un radical de formule générale (V), ou bien

$\beta_1$) le symbole R représente un radical alcoyle ou phényle,

le symbole $R°_1$ est défini comme $R_1$ précédemment en 1-b) et

le symbole $R°_2$ est défini comme $R_2$ précédemment en 1-b).

Il est entendu que, dans les produits de formule générale (XXXVIII), le substituant en position -3 du bicyclooctène présente la stéréoisomérie E ou Z, et, lorsque $R°_1$ est un radical de formule générale (II), celui-ci peut se présenter sous les formes syn ou anti. Les produits de formule générale (XXXVIII) existent également à l'état de mélanges de ces formes isomères.

Les produits de formule générale (XXXVIII) peuvent être obtenus à partir des produits de formule générale (I) en opérant comme suit:

I°) Les thiovinyl-3 céphalosporines de formule générale (XXXVIII) dans laquelle R est défini comme en $\alpha_1$ et $\beta_1$ à l'exception de contenir un substituant de formule générale (XXXIXc) peuvent être préparées par action d'un thiol (ou d'un de ses sels alcalins ou alcalino-terreux) de formule générale:

R–SH (XL)

[dans laquelle le radical R, qui est défini comme ci-dessus, est protégé à l'état d'acétal tel que défini par les formules générales (XXXIXa) et (XXXIXb) lorsque l'on veut obtenir une céphalosporine de formule générale (XXXVIII) dans laquelle R contient un radical formyle ou acylalcoyle], sur un dérivé de la céphalosporine ou un mélange des isomères de formule générale (I), [dans laquelle $R_1$ est un radical de formule générale (II) telle que définie précédemment en 1-a) ou 2- et $R_2$ a une définition correspondante, ou $R_1$ est défini comme précédemment en 1-b) et $R_2$ a une définition correspondante], suivie éventuellement de la réduction de l'oxyde obtenu et de l'élimination des radicaux protecteurs.

Il est entendu que, lorsque le radical R du produit de formule générale (XL) contient un groupement susceptible d'interférer avec la réaction il est préférable de protéger ce groupement, par toute méthode connue en soi et qui n'altère pas le reste de la molécule (notamment lorsque R contient un radical amino, alcoylamino, hydroxy ou carboxy).

Lorsqu'il s'agit des groupements amino, alcoylamino ou carboxy, la protection s'effectue dans les conditions décrites précédemment.

Lorsqu'il s'agit de groupements hydroxy, la protection s'effectue par les radicaux cités précédemment pour la protection de l'oxime, ou sous forme d'acétal cyclique pour la protection des radicaux dihydroxy-2,3 propyle ou dihydroxy-1,3 propyle-2 (par exemple sous forme de radicaux diméthyl-2,2 dioxolannyl-4 méthyle ou diméthyl-2,2 dioxannyle-5).

Il est également entendu que, lorsque $R_5$ représente un atome d'hydrogène, il est préférable de protéger l'oxime (dans les conditions décrites précédemment). Il est aussi préférable d'utiliser une céphalosporine dans laquelle le radical $R^c_5$ du groupement (IIa) est un radical protecteur d'acide.

Par ailleurs il est entendu que, lorsque le radical R du produit de formule générale (XL) comporte un radical hydroxy ou sulfo, il est préférable de mettre en œuvre un produit de formule générale (I) dans laquelle n = 0.

On opère généralement en présence d'une base telle qu'une pyridine ou une base organique tertiaire de formule générale (XVII).

On utilise par exemple la diisopropyléthylamine ou la diéthylphénylamine.

La présence d'une telle base n'est pas nécessaire lorsque l'on fait agir un sel alcalin ou alcalino-terreux du thiol de formule générale (XL).

La réaction s'effectue avantageusement dans un solvant organique, tel que le diméthylformamide, le tétrahydrofuranne, le méthanol, l'éthanol ou l'acétonitrile ou un mélange de tels solvants.

Il est également possible d'opérer en présence de bicarbonate alcalin dans un solvant tel que cité ci-dessus, éventuellement en présence d'eau.

On opère à une température comprise entre -20°C et la température de reflux du mélange réactionnel, la température chosie étant variable selon le thiol employé. De même, selon le thiol employé, la durée de réaction peut varier de 5 minutes à 48 heures.

Eventuellement on opère sous azote.

De préférence lorsque l'on veut utiliser un bicyclooctène-3 de formule générale (I) dans laquelle $R_1$ représente un radical de formule générale (II), on met en œuvre un tel produit pour lequel $R_2$ est autre que l'hydrogène.

L'élimination du radical protecteur de R peut être effectuée indifféremment avant ou après la réduction de l'oxyde, avant, simultanément ou après l'élimination des autres radicaux protecteurs.

La réduction de l'oxyde et l'élimination des groupements protecteurs s'effectuent selon les méthodes décrites précédemment.

Lorsque les radicaux dihydroxypropyle sont protégés à l'état d'acétals cycliques, l'élimination des radicaux protecteurs s'effectue par acidolyse (acide trifluoracétique, acide formique aqueux ou non, acide p.toluènesulfonique).

Lorsqu'on utilise l'acide formique, aqueux ou non, la libération des radicaux hydroxyles protégés à l'état d'acétal cyclique peut conduire au moins partiellement aux mono- ou diesters formiques correspondants, qui peuvent être séparés le cas échéant par chromatographie.

La libération des radicaux formylalcoyle ou acylalcoyle protégés sous forme de groupements de formule générale (XXXIXa) et (XXXIXb) [lorsque l'on veut obtenir un produit de formule générale (XXXVIII) dans laquelle R contient un radical formyle ou acylalcoyle] s'effectue

- en présence d'un acide sulfonique (acide méthanesulfonique ou acide p.toluènesulfonique par exemple) dans un solvant organique (acétonitrile ou acétone par exemple), éventuellement en présence d'eau et éventuellement en présence d'un réactif acétalisable tel que l'acétone, l'acide glyoxylique, le benzaldéhyde ou l'acide pyruvique, à une température comprise entre 20°C et la température de reflux du mélange réactionnel
- ou bien, lorsque le radical R est un radical dioxo-5,6 tétrahydro-1,4,5,6 triazine-1,2,4 yle-3, par action d'acide formique pur ou aqueux (contenant de préférence moins de 10% d'eau), soit en présence ou non de silice, soit par transacétalisation en présence d'un réactif acétalisable tel que défini ci-dessus.

Les thiols de formule générale (XL) (qui peuvent être mis en œuvre sous leur forme tautomère), peuvent être préparés par application de l'une des méthodes suivantes selon la signification du radical R:

- lorsque R est un radical pyridyle-3: selon la méthode décrite par H. M. Wuest et E. H. Sakal, J. Am. Chem. Soc., 73, 1210 (1951),
- lorsque R est un radical oxyde-1 pyridyle-3: selon la méthode décrite par B. Blank et coll., J. Med. Chem. 17, 1065 (1974),
- lorsque R est un radical oxyde-1 pyridyle-4: selon la méthode décrite par R. A. Y. Jones et coll., J. Chem. Soc., 2937 (1960),
- lorsque R est un radical pyridazinyle-3 substitué par alcoyle ou méthoxy et éventuellement N-oxydé: selon la méthode décrite dans le brevet belge 787 635,
- lorsque R est un radical pyridazinyle-3 substitué par amino et éventuellement N-oxydé: selon la méthode décrite dans le brevet belge 579 291,
- lorsque R est un radical pyridazinyle-3 substitué par acylamino et éventuellement N-oxydé: par application des méthodes décrites par M. Kumagai et M. Bando, Nippon Kagaku Zasshi, 84, 995 (1963) et par T. Horie et T. Ueda, Chem. Pharm. Bull., 11, 114 (1963),
- lorsque R est un radical tétrazolo [4,5-b] pyridazinyle-6: selon la méthode décrite dans le brevet belge 804 251,
- lorsque R est un radical dioxo-5,6 tétrahydro-1,4,5,6 triazine-1,2,4 yle-3 substitué en position-4 ou alcoyloxycarbonyl-2 triazol-1,3,4 yle-5 substitué en position -1 par un radical $R^{\gamma}$ choisi parmi:

a) un radical allyle, alcoyle (1 à 4 atomes de carbone, lui-même éventuellement substitué par un radical alcoyloxy, alcoylthio, phényle, carbamoyle, alcoylcarbamoyle, dialcoylcarbamoyle, acyle, alcoyloxycarbonyle ou thiazolidinyle-2),
b) un radical dihydroxy-2,3 propyle ou dihydroxy-1,3 propyle-2 (éventuellement protégé sous forme d'acétal cyclique),
c) un radical alcoyle [2 à 4 atomes de carbone, lui-même substitué par hydroxy, carbamoyloxy, dialcoylamino, alcoylsulfinyle, alcoylsulfonyle, alcoylsulfonylamino, sulfamoylamino, acylamino (éventuellement substitué), alcoyloxycarbonylamino, uréido, alcoyluréido, dialcoyluréido],
d) un radical de formule générale (XXXIXa) ou (XXXIXb),
e) un radical hydroxyiminoalcoyle ou alcoyloxyiminoalcoyle;
ou lorsque R est un radical dioxo-5,6 tétrahydro-1,4,5,6 triazine-1,2,4 yle-3 substitué en position -1, dioxo-5,6 tétrahydro-1,2,5,6 triazine-1,2,4 yle-3 substitué en position -2 ou alcoyloxycarbonyl-3 triazol-1,2,4 yle-5 substitué en position -1

par un radical $R^{\gamma}$ tel que défini ci-dessus (à l'exception de représenter un radical alcoyle non substitué) ou par un radical hydroxyalcoylcarbamoylalcoyle dont la portion hydroxyalcoyle contient 2 à 4 atomes de carbone, ou encore lorsque R est un radical dioxo-5,6 hydroxyalcoylcarbamoylalcoyl-4 tétrahydro-1,4,5,6 triazine-1,2,4 yle-3 dont la portion hydroxyalcoyle contient 2 à 4 atomes de carbone:
en faisant agir un oxalate d'alcoyle ou un halogénure de monooxalate d'alcoyle sur une thiosemicarbazide de formule générale:

$H_2N-CS-NH-NH-R^{\gamma}$ (XLIa)

$$H_2N-CS-\underset{}{\overset{R^{\gamma}}{\overset{|}{N}}}-NH_2$$ ou (XLIb)

$R^{\gamma 1}-NH-CS-NH-NH_2$ (XLIc)

(dans lesquelles $R^{\gamma}$ est défini comme ci-dessus et $R^{\gamma 1}$ représente un radical hydroxyalcoylcarbamoylalcoyle ou un radical $R^{\gamma}$).

On effectue la réaction en présence d'un alcoolate alcalin, par exemple l'éthylate ou le méthylate de sodium, ou le t.butylate de patassium, par application de la méthode décrite par M. Pesson et M. Antoine, Bull. Soc. Chim. France, 1590 (1970).

Il n'est pas absolument nécessaire de purifier le produit obtenu (ni de libérer les radicaux protégés) pour le mettre en œuvre pour la préparation des produits de formule générale (XXXVIII).

Les thiosemicarbazides de formule générale (XLIa) à (XLIc) peuvent être préparées selon l'une des méthodes décrites par K. A. Jensen et coll., Acta Chem. Sand., 22, 1 (1968), ou par application de la méthode décrite par Y. Kazakov et J. Y. Potovskii, Doklady Acad. Nauk. SSSR 134, 824 (1960), étant entendu que, lorsque $R^{\gamma}$ contient un radical amino, ce dernier est protégé.

La protection du radical amino et l'élimination du radical protecteur s'effectuent selon les méthodes habituelles qui n'altèrent pas le reste de la molécule. On utilise notamment le groupement t.butoxycarbonyle, qui peut être éliminé par hydrolyse acide.

– Lorsque R est un radical triazol-1,3,4 yle-5 substitué en position -1 par un radical alcoyle, allyle ou alcoyloxyalcoyle,
par un radical alcoyle (1 à 4 atomes de carbone) lui-même substitué comme défini ci-dessus en a) à l'exception d'un radical thiazolidinyle-2,
par un radical tel que défini ci-dessus en c), ou
par un radical alcoyloxyiminoalcoyle:
par application de l'une des méthodes décrites par M. Pesson et M. Antoine, Bull. Soc. Chim. France, 1590 (1970);

– lorsque R est un radical triazol-1,3,4 yle-5 substitué en position -1 par thiazolidinyl-2 alcoyle ou hydroxyiminoalcoyle:
par action respectivement de cystéamine ou d'hydroxylamine sur un dialcoyloxyalcoyl-1 mercapto-5 triazole-1,3,4 qui peut être obtenu par application de la méthode décrite par M. Kanaoka, J. Pharm. Soc. Japan, 75, 1149 (1955), à partir d'une dialcoyloxyalcoyl-4 thiosemicarbazide.

– Lorsque R est un radical triazol-1,3,4 yle-5 substitué en position -1 par dihydroxy-2,3 propyle ou dihydroxy-1,3 propyle-2 (éventuellement protégés sous forme d'acétal cyclique), ou représente un radical de formule générale (XXXIXa) ou (XXXIXb):
par application de la méthode décrite par M. Kanaoka, J. Pharm. Soc. Japan, 75, 1149 (1955).

– Lorsque R est un radical dioxo-5,6 tétrahydro-1,4,5,6 triazine-1,2,4 yle-3 substitué en position -4 ou un radical alcoyloxycarbonyl-2 triazol-1,3,4 yle-5 ou triazol-1,3,4 yle-5 substitués en position -1 par acyloxyalcoyle (éventuellement substitué): par acylation respectivement de la dioxo-5,6 hydroxyalcoyl-4 mercapto-3 tétrahydro-1,4,5,6 triazine-1,2,4, de l'alcoyloxycarbonyl-2 hydroxyalcoyl-1 mercapto-5 triazole-1,3,4 ou de l'hydroxyalcoyl-1 mercapto-5 triazole-1,3,4 dont le radical mercapto a été préalablement protégé [par exemple selon C. G. Kruse et coll., Tet. Lett., 1725 (1976)], par toute méthode connue pour acyler un alcool sans toucher au reste de la molécule, puis libération du groupement mercapto en milieu acide.

– Lorsque R est un radical dioxo-5,6 tétrahydro-1,4,5,6 triazine-1,2,4 yle-3 substitué en position -4 ou alcoyloxycarbonyl-2 triazol-1,3,4 yle-5 ou triazol-1,3,4 yle-5 substitués en position -1 par aminoalcoyle ou alcoylaminoalcoyle:
par libération de la fonction amine du produit correspondant, protégé par exemple par un groupement t.butoxycarbonyle.

– Lorsque R est un radical dioxo-5,6 tétrahydro-1,4,5,6 triazine-1,2,4 yle-3 substitué en position -4, alcoyloxycarbonyl-2 triazol-1,3,4 yle-5 ou triazol-1,3,4 yle-5 substitués en position -1 par sulfoaminoalcoyle: à partir du produit correspondant substitué par un radical t.butoxycarbonylaminoalcoyle, par analogie avec la méthode décrite dans le brevet belge 847 237.

– Lorsque R est un radical dialcoyl-1,4 dioxo-5,6 tétrahydro-1,4,5,6 triazine-1,2,4 yle-3 ou alcoyl-1 dioxo-5,6 tétrahydro-1,4,5,6 triazine-1,2,4 yle-3: selon la méthode décrite dans le brevet belge 830 455.

– Lorsque R est un radical alcoyl-2 dioxo-5,6 tétrahydro-1,2,5,6 triazine-1,2,4 yle-3 ou alcoyl-1 alcoyloxycarbonyl-3 triazol-1,2,4 yle-5: selon la méthode décrite par M. Pesson et M. Antoine, C. R. Acad. Sci., Ser C, 267, 25, 1726 (1968).

– Lorsque R est un radical triazol-1,2,3 yle-5: selon la méthode décrite dans la demande de brevet français 2 215 942.

– Lorsque R est un radical triazol-1,3,4 yle-5: selon la méthode décrite par M. Kanaoka, J. Pharm. Soc. Jap., 75, 1149 (1955).

– Lorsque R est un radical thiadiazol-1,3,4 yle-5 éventuellement substitué par alcoyle, alcoyloxy, alcoylthio, alcoylsulfonyle, amino, alcoylamino, dialcoylamino ou acylamino: selon les méthodes décrites dans le brevet belge 830 821.

– Lorsque R est un radical thiadiazol-1,3,4 yle-5 substitué par hydroxyalcoyle, aminoalcoyle, alcoylaminoalcoyle ou dialcoylaminoalcoyle: selon la méthode décrite dans la demande de brevet allemand 2 446 254.

– Lorsque R est un radical thiadiazol-1,3,4 yle-5 substitué par un radical carboxyalcoyle: par application de la méthode décrite dans la demande de brevet allemand 1 953 861.

– Lorsque R est un radical thiadiazol-1,3,4 yle-5 substitué par un radical trifluorométhyle: selon la méthode décrite dans la demande de brevet allemand 2 162 575.

– Lorsque R est un radical thiadiazol-1,3,4 yle-5 substitué par un radical carboxy: selon la méthode décrite dans la demande de brevet japonais 77 48666.

– Lorsque R est un radical thiadiazol-1,3,4 yle-5 substitué par un radical acylaminoalcoyle: selon la méthode décrite dans la demande de brevet japonais 76 80857.

– Lorsque R est un radical thiadiazol-1,3,4 yle-5 substitué par un radical hydroxyalcoylthio: par application de la méthode décrite par G. Nannini, Arz. Forsch., 27 (2), 343 (1977).

– Lorsque R est un radical thiadiazol-1,2,4 yle-5 substitué par alcoyle ou alcoyloxy: selon la méthode décrite dans la demande de brevet allemand 2 806 226 ou selon Chem. Ber., 90, 184 (1957).

– Lorsque R est un radical oxadiazol-1,3,4 yle-5 tel que défini précédemment en 8a): par application de la méthode décrite par E. Hoggarth, J. Chem. Soc., 4811 (1952).

– Lorsque R est un radical oxazolyle-2 ou alcoyl-4 oxazolyle-2: par application de la méthode décrite précédemment par C. Bradsher, J. Org. Chem., 32, 2079 (1967).

– Lorsque R est un radical tétrazolyle-5 éventuellement substitué en position -1 par alcoyle, hydroxyalcoyle ou phényle: selon les méthodes décrites dans le brevet belge 830 821.

– Lorsque R est un radical tétrazolyle-5 substitué en position -1 par alcoyloxyalcoyle: par addition d'azoture de sodium sur un isothiocyanatoalcoyloxyalcoyle en opérant dans un solvant organique tel que l'éthanol, à la température de reflux du mélange réactionnel.

L'isothiocyanatoalcoyloxyalcoyle peut être obtenu par application de la méthode décrite par E. Schmidt et coll., Chem. Ber., 73, 286 (1940).

– Lorsque R est un radical tétrazolyle-5 substitué en position -1 par un radical carboxyalcoyle: selon la méthode décrite dans le brevet belge 858 112.

– Lorsque R est un radical tétrazolyle-5 substitué en position -1 par un radical sulfoalcoyle: selon la méthode décrite dans le brevet belge 856 498 ou décrite par D. A. Berges et coll., J. Het. Chem., 15, 981 (1978).

– Lorsque R est un radical tétrazolyle-5 substitué en position -1 par un radical aminoalcoyle, alcoylaminoalcoyle, dialcoylaminoalcoyle: par application de la méthode décrite dans la demande de brevet allemand 2 738 711.

– Lorsque R est un radical tétrazolyle-5 substitué en position -1 par un radical sulfamoylalcoyle, sulfamoylaminoalcoyle ou sulfoaminoalcoyle: selon la méthode décrite dans le brevet belge 856 636.

– Lorsque R est un radical tétrazolyle-5 substitué par un radical acylaminoalcoyle ou thiadiazol-1,3,4 yle-5 substitué par hydroxy: selon la méthode décrite dans le brevet US 4 117 123.

– Lorsque R est un radical tétrazolyle-5 substitué en position -1 par un radical uréidoalcoyle, alcoyluréidoalcoyle ou dialcoyluréidoalcoyle: à partir du produit correspondant substitué par aminoalcoyle (dont le radical mercapto a été préalablement protégé), par traitement par un isocyanate alcalin, par un isocyanate d'alcoyle ou par un halogénure de dialcoylcarbamoyle, puis libération du groupement mercapto dans les conditions décrites dans le brevet belge 847 237.

– Lorsque R est un radical tétrazolyle-5 substitué en position -1 par un radical carboxyalcoylaminoalcoyle: selon la méthode décrite dans la demande de brevet allemand 2 715 597.

– Lorsque R est un radical tétrazolyle-5 substitué en position -1 par un radical dihydroxy-2,3 propyle: selon la méthode décrite dans le brevet US 4 064 242.

– Lorsque R est un radical tétrazolyle-5 substitué en position -1 par un radical dihydroxy-1,3 propyle-2: par addition d'azoture de sodium sur un isothiocyanate de diméthyl-2,2 dioxolanne-1,3 yle-5 (suivie éventuellement de la libération des groupements hydroxy).

– Lorsque R est un radical tétrazolyle-5 substitué en position -1 par un radical de formule générale (XXXIXa) ou (XXXIXb) tels que définis précédemment en 9e) ou un radical défini précédemment en 9c): par action d'azoture de sodium sur l'isothiocyanate correspondant, par analogie avec la méthode décrite par R. E. Orth, J. Pharm. Sci., 52 (9), 909 (1963), étant entendu que dans le cas où R contient un substituant hydroxy ou hydroxyiminoalcoyle, l'alcool ou l'oxime sont éventuellement protégés par exemple par un groupement tétrahydropyrannyle.

– Lorsque R est un radical triazol-1,2,4 yle-5 substitué en position -1 par un radical allyle ou alcoyloxyalcoyle, par un radical alcoyle (1 à 4 atomes de carbone) lui-même substitué comme défini ci-dessus en a) à l'exception d'un radical thiazolidinyle-2, par un radical hydroxyalcoylcarbamoylalcoyle dont la portion hydroxyalcoyle contient 2 à 4 atomes de carbone, par un radical tel que défini ci-dessu en c), ou par un radical alcoyloxyiminoalcoyle: par analogie avec les méthodes décrites par M. Pesson et M. Antoine, Bull. Soc. Chim. France, 1599 (1970) ou C. R. Acad. Sci., Ser. C, 267 (25), 1726 (1968).

– Lorsque R est un radical triazol-1,2,4 yle-5 substitué en position 1 par thiazolidinyl-2 alcoyle ou par hydroxyiminoalcoyle: par action respectivement de cystéamine ou d'hydroxylamine sur un dialcoyloxyalcoyl-1 mercapto-5 triazole-1,2,4 qui peut être obtenu par analogie avec la méthode décrite par M. Kanaoka, J. Pharm. Soc. Japan, 75,

1149 (1955), à partir d'une dialcoyloxyalcoyl-4 thiosemicarbazide.

- Lorsque R est un radical triazol-1,2,4 yle-5 substitué en position -1 par dihydroxy-2,3 propyle ou dihydroxy-1,3 propyle-2 (éventuellement protégés sous forme d'acétal cyclique), ou par un radical de formule générale (XXXIXa) ou (XXXIXb): par analogie avec la méthode décrite par M. Kanaoka, J. Pharm. Soc. Japan, 75, 1149 (1955).

- Lorsque R est un radical dioxo-5,6 tétrahydro-1,4,5,6 triazine-1,2,4 yle-3 substitué en position -1, dioxo-5,6 tétrahydro-1,2,5,6 triazine-1,2,4 yle-3 substitué en position -2 ou un radical alcoyloxycarbonyl-3 triazol-1,2,4 yle-5 ou triazol-1,2,4 yle-5 substitués en position -1, par acyloxyalcoyle (éventuellement substitué): par acylation respectivement de la dioxo-5,6 hydroxyalcoyl-1 mercapto-3 tétrahydro-1,4,5,6 triazine-1,2,4, de la dioxo-5,6 hydroxyalcoyl-2 mercapto-3 tétrahydro-1,2,5,6 triazine-1,2,4, de l'alcoyloxycarbonyl-3 hydroxyalcoyl-1 mercapto-5 triazole-1,2,4 ou de l'hydroxyalcoyl-1 mercapto-5 triazole-1,2,4 dont le radical mercapto a été préalablement protégé [par exemple selon C. G. Kruse et coll., Tet. Lett. 1725 (1976)], par toute méthode connue pour acyler un alcool sans toucher au reste de la molécule, puis libération du groupement mercapto en milieu acide.

- Lorsque R est un radical dioxo-5,6 tétrahydro-1,4,5,6 triazine-1,2,4 yle-3 substitué en position -1, dioxo-5,6 tétrahydro-1,2,5,6 triazine-1,2,4 yle-3 substitué en position -2 ou alcoyloxycarbonyl-3 triazol-1,2,4 yle-5 ou triazol-1,2,4 yle-5 substitués en position -1 par aminoalcoyle ou alcoylaminoalcolye: par libération de la fonction amine du produit correspondant, protégé par exemple par un groupement t.butoxycarbonyle.

- Lorsque R est un radical dioxo-5,6 tétrahydro-1,4,5,6 triazine-1,2,4 yle-3 substitué en position -1, dioxo-5,6 tétrahydro-1,2,5,6 triazine-1,2,4 yle-3 substitué en position -2, alcoyloxycarbonyl-3 triazol-1,2,4 yle-5 ou triazol-1,2,4 yle-5 substitués en position -1 par sulfoaminoalcoyle: à partir du produit correspondant substitué par exemple par un radical t.butoxycarbonylaminoalcoyle, par analogie avec la méthode décrite dans le brevet belge 847 237.

- Lorsque R est un radical alcoyl-1 dioxo-5,6 tétrahydro-1,4,5,6 triazine-1,2,4 yle-3 substitué en position 4, alcoyl-1 dioxo-5,6 tétrahydro-1,2,5,6 triazine-1,2,4 yle-3 substitué en position -2, alcoyl-2 dioxo-5,6 tétrahydro-1,2,5,6 triazine-1,2,4 yle-3 substitué en position -1 ou alcoyl-4 dioxo-5,6 tétrahydro-1,4,5,6 triazine-1,2,4 yle-3 substitué en position -1 par un radical formylalcoyle ou par un radical de formule générale (XXXIXa) dans laquelle $R^{\beta}$ représente un atome d'hydrogène: en faisant agir un oxalate d'alcoyle sur une thiosemicarbazide, par analogie avec la méthode décrite par M. Pesson et M. Antoine, C. R. Acad. Sci., 267 (25C), 904 (1968) ou C. R. Acad. Sci, 267 (25C), 1726 (1968).

II°) Les thiovinyl-3 céphalosporines de formule générale (XXXVIII) dans laquelle R ne contient pas de substituant de formule générale (XXXIXc) peuvent également être obtenues de la manière suivante:

On fait agir un thiol de formule générale (XL) (ou un de ses sels alcalins ou alcalino-terreux) tel que défini ci-dessus sur un produit ou un mélange d'isomères du produit de formule générale (I) [dans laquelle $R_1$ est défini comme dans la formule générale (I) en 1-a) à l'exception de représenter un radical de formule générale (II), ou représente un radical amino-5 adipoyle dont les fonctions amine et acide sont protégées, ou un radical de formule générale (VI) ou (VII) tel que défini en 1-b) et $R_2$ a une définition correspondante] puis on réduit éventuellement le sulfoxyde obtenu (lorsque n = 1) et on élimine éventuellement les radicaux protecteurs de R pour préparer un produit de formule générale:

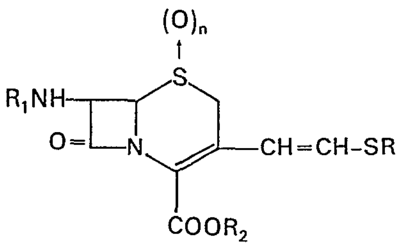

(XLII)

dans laquelle, n étant défini comme précédemment, $R_1$ et $R_2$ sont définis comme en II/-ci-dessus et R a une définition correspondante.

La réaction s'effectue dans les conditions décrites précédemment pour l'obtention d'un produit de formule générale (XXXVIII) à partir d'un produit de formule générale (I) et d'un thiol de formule générale (XL).

Il est entendu que le radical R du thiol est (le cas échéant) protégé comme décrit précédemment et que l'élimination des radicaux protecteurs peut être effectuée dans les conditions décrites précédemment. Il est cependant préférable de conserver les groupements protecteurs jusqu'à l'obtention du produit de formule générale (XXXVIII).

On prépare ensuite un produit de formule générale:

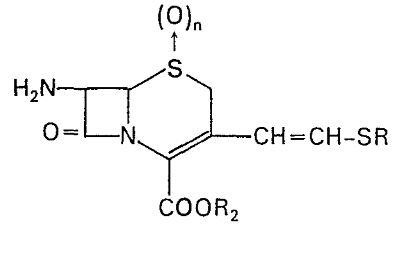

(XLIII)

dans laquelle R, $R_2$ et n sont définis comme ci-dessus, par élimination du radical $R_1$ d'un produit de formule générale (XLII) ou éventuellement élimination simultanée du radical protecteur $R_1$ et

des autres radicaux protecteurs de ce produit.

On opère dans les conditions décrites précédemment pour la préparation d'un produit de formule générale (I), dans laquelle $R_1$ est un atome d'hydrogène.

Il n'est pas nécessaire d'isoler le produit de formule générale (XLIII) pour l'utiliser dans la suite de la synthèse.

On prépare alors la thiovinyl-3 céphalosporine de formule générale (XXXVIII) dans laquelle R, $R^o_1$ et $R^o_2$ sont définis comme précédemment, par acylation d'une amino-7 céphalosporine de formule générale (XLIII) au moyen d'un acide représenté par la formule générale

$R^o_1$-OH (XLIV)

(dans laquelle $R^o_1$, qui est défini comme précédemment, est éventuellement protégé s'il comporte des radicaux pouvant interférer avec la réaction), ou d'un dérivé réactif de cet acide, dans les conditions décrites précédemment pour la préparation des produits de formule générale (XXIII) puis on réduit l'oxyde obtenu (lorsque n = 1) et élimine les radicaux protecteurs.

Il est entendu que

- les radicaux amino ou alcoylamino, qui existent dans certains radicaux R, doivent être protégé, et que
- les radicaux carboxy, hydroxy, formyle ou acylalcoyle contenus dans les radicaux R peuvent être protégés.

Lorsque $R^o_1$ contient un radical de formule générale (IIa) le radical $R^c_5$ est obligatoirement un radical protecteur.

La protection et l'élimination des radicaux protecteurs s'effectuent dans les conditions décrites précédemment.

La réduction de l'oxyde et l'élimination des autres radicaux protecteurs s'effectuent dans les conditions décrites précédemment.

Il est également entendu que lorsque R contient un substituant hydroxy, sulfo, sulfinyle ou sulfonyle, on préfère mettre en œuvre un produit de formule générale (XLIII) dans laquelle n = 0.

III°) Les thiovinyl-3 céphalosporines de formule générale (XXXVIII) dans laquelle R ne contient pas de substituant de formule générale (XXXIXc) peuvent également être obtenues par action d'un thioloester de formule générale:

$R'_1$-SR (XLV)

dans laquelle $R'_1$ soit représente un radical de formule générale (II), $R^c_5$ représentant un radical protecteur, soit est défini comme $R_1$ en 1-b) et R est défini comme ci-dessus [étant entendu que, lorsqu'il contient un substituant amino ou alcoylamino, celui-ci est protégé; lorsqu'il contient un substituant hydroxy ou carboxy celui-ci est libre ou protégé et lorsqu'il contient un substituant formyle ou acylalcoyle, celui-ci est protégé à l'état d'acétal de formule générale (XXXIXa) ou (XXXIXb)], sur une amino-7 céphalosporine de formule générale (I) dans laquelle $R_1$ est un atome d'hydrogène et $R_2$ a la définition correspondante, suivie de la réduction du sulfoxyde obtenu lorsque n = 1 et le cas échéant de l'élimination des radicaux protecteurs.

Il est également entendu que les radicaux $R'_1$ qui contiennent un groupement susceptible d'interférer avec la réaction sont préalablement protégés. Il en est de même pour l'oxime lorsque $R'_1$ représente un radical de formule générale (II) dans laquelle $R_5$ est un atome d'hydrogène.

Il est aussi préférable de mettre en œuvre un produit dans lequel $R'_1$ ne contient pas de substituant halogéné.

De même que pour les procédés décrits précédemment, lorsque R contient un substituant hydroxy, sulfo, sulfinyle ou sulfonyle, on préfère mettre en œuvre un produit de formule générale (I) dans laquelle n = 0.

La protection et l'élimination des radicaux protecteurs s'effectuent dans les conditions décrites précédemment.

La réaction du thioloester avec l'amino-7 céphalosporine de formule générale (I) s'effectue généralement en présence d'un accepteur d'acide tel qu'une base organique, plus particulièrement en présence d'une pyridine ou d'une base organique tertiaire de formule générale (XVII), notamment la triéthylamine, la NN-diisopropyl N-éthylamine, la diéthylphénylamine ou la N-méthylmorpholine.

La réaction s'effectue avantageusement dans un solvant organique tel qu'un amide (par exemple diméthylformamide, diméthylacétamide), un éther (par exemple tétrahydrofuranne, dioxanne), un solvant chloré (par exemple chloroforme, chlorure de méthylène), une cétone (par exemple acétone) ou un nitrile (par exemple acétonitrile), ou bien dans un mélange de ces solvants. Il est également possible d'opérer en présence d'un bicarbonate alcalin dans l'un des solvants cités ci-dessus, éventuellement en présence d'eau.

On opère à une température comprise entre -20°C et la température de reflux du mélange réactionnel. La réaction s'effectue éventuellement sous azote.

La réduction du S-oxyde s'effectue dans les conditions décrites précédemment.

Les thioloesters de formule générale (XLV) peuvent être préparés par action d'un acide ou d'un dérivé réactif d'un acide de formule générale:

$R'_1$OH (XLIVa)

sur un thiol de formule générale (XL) (ou sur un sel alcalin ou alcalino-terreux de ce thiol) suivie éventuellement de l'élimination des radicaux protecteurs.

Dans la formule générale (XLIVa), $R'_1$ représente un radical de formule générale (II) dans laquelle $R_4$ et $R_5$ (ou $R^c_5$) sont autres que l'hydrogè-

ne, ou bien $R'_1$ est défini comme $R_1$ en 1-b).

Il est entendu que les substituants amino ou alcoylamino de $R'_1$ et R sont protégé et que les substituants hydroxy ou carboxy sont libres ou protégés.

Il est également entendu que le radical R est protégé à l'état d'acétal lorsque l'on veut préparer un produit de formule générale (XXXVIII) pour lequel R contient un radical formyle ou acylalcoyle.

On opère dans les conditions décrites précédemment pour la préparation d'un produit de formule générale (XXII) à partir d'un produit de formule générale (XXIV) et d'un ester réactif de formule générale (XXVI).

Lorsque l'on veut obtenir un produit pour lequel R contient un radical carboxy ou sulfo il est préférable de faire agir un dérivé réactif de l'acide $R'_1OH$ sur le thiol correspondant.

Lorsque l'on veut obtenir un thioloester pour lequel $R'_1$ est un radical de formule générale (II) tel que défini pour $R^o_1$, on peut éliminer le radical t.butoxycarbonyle protecteur de l'aminothiazole par traitement en milieu acide anhydre. De préférence on emploie l'acide trifluoracétique en opérant entre 0 et 20°C. On peut éliminer le radical trityle protecteur de l'oxime par acidolyse, par exemple par l'acide trifluoracétique anhydre.

Le cas échéant, l'élimination du groupement trityle protecteur d'un substituant hydroxy du thioloester s'effectue dans les conditions décrites ci-dessus pour la libération de l'oxime.

Il est avantageux de n'éliminer les groupements protecteurs qu'après la réaction du thioloester sur le produit de formule générale (I) dans laquelle $R_1$ est l'atome d'hydrogène.

IV°) Les thiovinyl-3 céphalosporines de formule générale (XXXVIII) dans laquelle $R^o1$ représente un radical de formule générale (II) (tel que défini précédemment, à l'exception pour $R_5$ de représenter un radical vinyle) et R ne contient pas de substituant de formule générale (XXXIXc) peuvent être obtenus en opérant de la manière suivante:

On prépare un produit de formule générale:

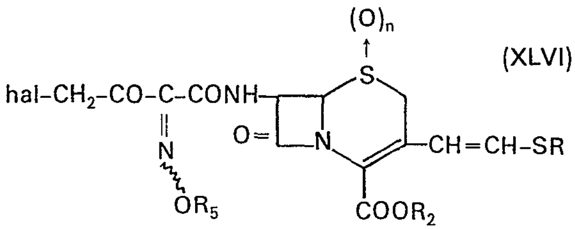

(XLVI)

dans laquelle R est défini comme ci-dessus, $R_5$ est défini comme dans la formule générale (XXXII) et $R_2$, hal et n sont définis comme précédemment, à partir d'un produit de formule générale (XLIII) ou d'un produit de formule générale:

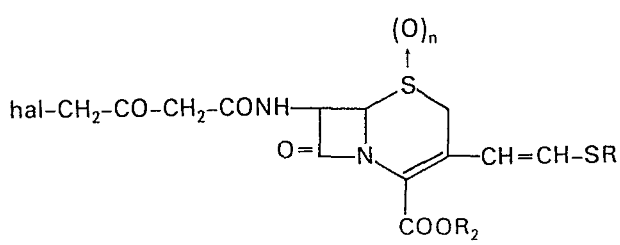

(XLVII)

(dans laquelle hal, $R_2$ et n sont définis comme précédemment et R est défini comme ci-dessus), par application des méthodes décrites précédemment pour la préparation du produit de formule générale (XXXII).

Lorsque l'on prépare le produit de formule générale (XLVI) à partir d'un produit de formule générale (XLIII) le radical R est préalablement protégé lorsqu'il contient un radical amino ou alcoylamino, et il est libre ou protégé lorsqu'il contient un radical hydroxy, carboxy, formyle ou acylalcoyle.

Lorsque l'on prépare le produit de formule générale (XLVI) à partir d'un produit de formule générale (XLVII), le radical R est préalablement protégé lorsqu'il contient un radical amino, alcoylamino ou formyle, et il est libre ou protégé lorsqu'il contient un radical hydroxy, carboxy ou acylalcoyle.

La protection et l'élimination des radicaux protecteurs s'effectuent dans les conditions décrites précédemment.

On fait agir une thiourée de formule générale (XXXI) sur le produit de formule générale (XLVI) dans les conditions décrites précédemment pour la préparation des produits de formule générale (I) à partir des produits de formule générale (XXXII), puis on réduit le cas échéant le sulfoxyde obtenu et élimine éventuellement les radicaux protecteurs.

Lorsque l'on veut obtenir un produit de formule générale (XXXVIII) dans laquelle R contient un radical formylalcoyle ou acylalcoyle, ce radical peut être protégé à l'état d'acétal, sous forme d'un radical de formule générale (XXXIXa) ou (XXXIXb) tel que défini précédemment.

La réduction du sulfoxyde et l'élimination des radicaux protecteurs s'effectuent dans les conditions décrites précédemment.

La céphalosporine de formule générale (XLVII) peut être préparée à partir d'une céphalosporine de formule générale (XLIII), par analogie avec la méthode décrite pour la préparation des produits de formule générale (XXXVII).

V°) Les thiovinylcéphalosporines de formule générale (XXXVIII) dans laquelle R représente un radical dioxo-5,6 tétrahydro-1,4,5,6 triazine-1,2,4 yle-3 substitué en position -1 ou 4, dioxo-5,6 tétrahydro-1,2,5,6 triazine-1,2,4 yle-3 substitué en position -2, ou bien triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitués en position -1, par un radical alcoyle contenant 2 à 4 atomes de carbone substitué par un groupement carbamoyloxy ou acyloxy (dont la partie acyle est éventuellement substituée par un radical amino, alcoylamino ou dialcoylamino), qui sont des dérivés fonctionnels du produit de formule générale (XXXVIII) dans laquelle R est un radical -®-alk'OH choisi parmi dioxo-5,6 hydroxyalcoyl-1 (ou -4) tétrahydro-1,4,5,6 triazine-1,2,4 yle-3, dioxo-5,6 hydroxyalcoyl-2 tétrahydro-1,2,5,6 triazine-1,2,4 yle-3, hydroxyalcoyl-1 triazol-1,3,4 (ou -1,2,4) yle-5, alcoyloxycarbonyl-2 hydroxyalcoyl-1 triazol-1,3,4 yle-5 ou alcoyloxycarbonyl-3 hydroxyalcoyl-1 triazol-1,2,4 yle-5 peuvent être obtenus par carbamatation ou estérification d'un produit de formule générale:

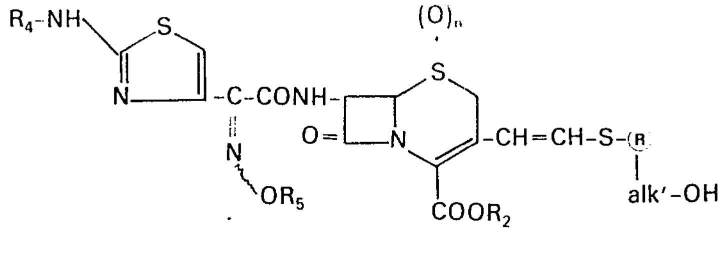

(XXXVIIIa)

(dans laquelle $R_2$, $R_4$, $R_5$, ®-alk'-OH et n sont définis comme précédemment à l'exception pour $R_4$ de représenter l'atome d'hydrogène) par toute méthode connue pour obtenir un ester ou un carbamate à partir d'un alcool sans toucher au reste de la molécule, puis s'il y a lieu, réduction du sulfoxyde obtenu et élimination des radicaux protecteurs.

L'estérification s'effectue à une température comprise entre -50°C et la température de reflux du mélange réactionnel, notamment par condensation de l'anhydride de l'acide (ou d'un autre dérivé réactif, par exemple halogénure) dans un solvant organique inerte tel qu'un éther (par exemple tétrahydrofuranne), un solvant chloré (par exemple chlorure de méthylène), ou un mélange de tels solvants, en présence d'une base azotée comme la pyridine, la diméthylamino-4 pyridine ou une trialcoylamine (triéthylamine) ou d'un agent alcalin de condensation (par exemple bicarbonate de sodium) puis, le cas échéant, réduction du S-oxyde obtenu et élimination des groupements protecteurs selon les méthodes décrites précédemment.

L'obtention du carbamate s'effectue par toute méthode connue qui n'altère pas le reste de la molécule. On opère notamment par action d'isocyanate de chlorosulfonyle ou de trichloracétyle dans un solvant organique inerte, par exemple le tétrahydrofuranne ou l'acétonitrile, à une température comprise entre -80 et 20°C, puis on élimine les groupements protecteurs.

VI°) Les thiovinyl-3 céphalosporines de formule générale (XXXVIII) dans laquelle R représente un radical dioxo-5,6 tétrahydro-1,4,5,6 triazine-1,2,4 yle-3 substitué en position -1 ou -4, dioxo-5,6 tétrahydro-1,2,5,6 triazine-1,2,4 yle-3 substitué en position -2, ou bien triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitués en position -1, par un radical alcoyle contenant 2 à 4 atomes de carbone substitué par un groupement sulfoamino, alcoylsulfonylamino, sulfamoylamino, acylamino (dont la partie acyle est éventuellement substituée par hydroxy, amino, alcoylamino ou dialcoylamino), alcoyloxycarbonylamino, uréido, alcoyluréido ou dialcoyluréido, ou représente un radical thiadiazol-1,3,4 yle-5 substitué par un radical acylamino ou acylaminoalcoyle, ou représente un radical oxadiazol-1,3,4 yle-5 substitué par un radical acylaminoalcoyle, ou représente un radical tétrazolyle-5 substitué en position -1 par un radical alcoyle contenant 2 à 4 atomes de carbone substitué par un groupement acylamino, sulfamoylamino, sulfoamino, uréido, alcoyluréido ou dialcoyluréido, et $R°_1$ et $R°_2$ ont les définitions correspondantes, qui sont tous des dérivés fonctionnels de l'amine qui leur correspond, peuvent être obtenus par traitement d'une amine de formule générale:

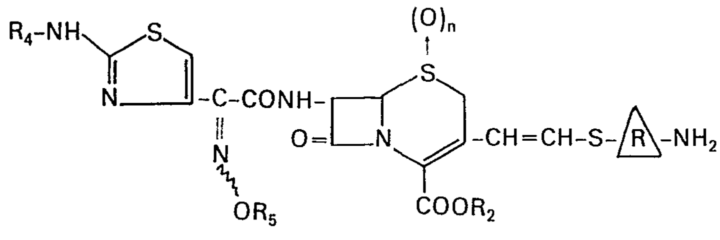

(XXXVIIIb)

dans laquelle $R_4$, $R_5$, $R_2$ et n sont définis comme précédemment en V°/, et -Ⓡ-$NH_2$ représente un radical dioxo-5,6 tétrahydro-1,4,5,6 triazine-1,2,4 yle-3 substitué en position -1 ou -4, dioxo-5,6 tétrahydro-1,2,5,6 triazine-1,2,4 yle-3 substitué en position -2, ou bien triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitués en position -1, par un radical aminoalcoyle dont la partie alcoyle contient 2 à 4 atomes de carbone,

ou un radical thiadiazol-1,3,4 yle-5 substitué par un radical amino ou aminoalcoyle, ou un radical oxadiazol-1,3,4 yle-5 substitué par un radical aminoalcoyle, ou un radical tétrazolyle-5 substitué en position -1 par un radical aminoalcoyle dont la partie alcoyle contient 2 à 4 atomes de carbone, par toute méthode connue en soi pour former une fonction amide, sulfamide, carbamate ou urée, sans toucher au reste de la molécule, puis le cas échéant réduction du sulfoxyde et élimination des groupements protecteurs.

Il est entendu que les produits qui contiennent un groupement sulfo, sulfonyle ou sulfamoyle sont préparés de préférence à partir d'un produit de formule générale (XXXVIIIb) dans laquelle n = 0.

Par ailleurs, lorsque l'on veut préparer un produit dont le radical R contient un groupement amino ou hydroxy, il est nécessaire de protéger ces radicaux dans le réactif utilisé. De même, lorsque $R_5$ représente l'atome d'hydrogène, il est nécessaire de protéger l'oxime.

Lorsque $R_5$ contient un groupement carboxy, celui-ci est libre ou protégé.

Lorsque l'on veut préparer un produit de formule générale (XXXVIII) dans laquelle le radical R contient un substituant alcoylsulfonylamino, sulfamoylamino, acylamino (substitué ou non), alcoyloxycarbonylamino ou dialcoyluréido, la réaction est effectuée avantageusement par action, respectivement, du dérivé chlorosulfonyle, du chlorure d'acide, du chloroformiate ou du chlorure de dialcoylcarbamoyle correspondant dans les conditions décrites précédemment pour la réaction du chlorure de l'acide de formule générale (XXV) sur l'amino-7 céphalosporine de formule générale (XXIV).

Lorsque l'on veut préparer un produit de formule générale (XXXVIII) dans laquelle le radical R contient un substituant sulfoamino, alcoylsulfonylamino ou acylamino (substitué ou non), on peut effectuer la réaction au moyen de l'anhydride de l'acide correspondant, dans les conditions décrites précédemment pour faire réagir le produit de formule générale (XXV) sous forme d'anhydride.

Lorsque l'on veut obtenir un produit de formule générale (XXXVIII) pour lequel R contient un radical acylamino (substitué ou non), il est également possible de faire agir l'acide correspondant, dans les conditions opératoires décrites précédemment pour l'emploi de l'acide de formule générale (XXV).

Lorsque l'on veut obtenir un produit de formule générale (XXXVIII) dans laquelle R contient un radical uréido ou alcoyluréido, on fait agir respectivement un isocyanate alcalin ou un isocyanate d'alcoyle sur le produit correspondant de formule générale (XXXVIIIb) en milieu hydroorganique ou organique (par exemple dans le tétrahydrofuranne) à une température comprise entre –20 et 60°C.

La réduction et l'élimination des radicaux protecteurs s'effectuent dans les conditions décrites précédemment.

VII°) Les thiovinyl-3 céphalosporines de formule générale (XXXVIII) dans laquelle R représente un radical dioxo-5,6 tétrahydro-1,4,5,6 triazine-1,2,4 yle-3 substitué en position -1 ou -4, dioxo-5,6 tétrahydro-1,2,5,6 triazine-1,2,4 yle-3 substitué en position -2, ou bien triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitués en position -1, par un radical thiazolidinyl-2 alcoyle, par un radical de formule générale (XXXIXc) ou par un radical hydroxyiminoalcyle ou alcoyloxyiminoalcoyle dont la partie iminoalcoyle contient 2 à 5 atomes de carbone ou bien représente un radical tétrazolyle-5 substitué en position -1 par un radical hydroxyiminoalcoyle ou alcoyloxyiminoalcoyle dont la partie iminoalcoyle contient 2 à 5 atomes de carbone, et $R°_1$ et $R°_2$ ont des définitions correspondantes, qui sont des dérivés d'addition du produit de formule générale (XXXVIII) dans laquelle R est l'un des hétérocycles cités ci-dessus substitué par un radical formylalcoyle (ou sa forme hydrate), peuvent être obtenus à partir d'un aldéhyde de formule générale:

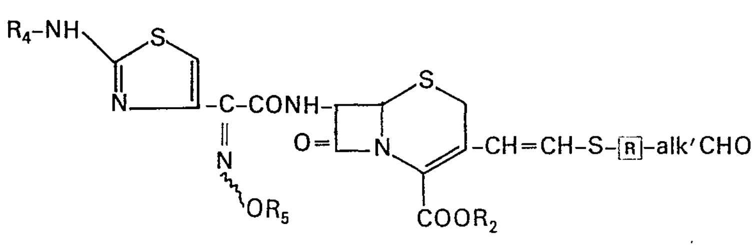

(XXXVIIIc)

dans laquelle $R_2$ et $R_4$ sont définis comme précédemment, $R_5$ est défini comme précédemment en V°) et -[R]-alk'CHO représente un radical dioxo-5,6 formylalcoyl-1 (ou -4) tétrahydro-1,4,5,6 triazine-1,2,4 yle-3, dioxo-5,6 formylalcoyl-2 tétrahydro-1,2,5,6 triazine-1,2,4 yle-3, formylalcoyl-1 triazol-1,3,4 (ou -1,2,4) yle-5, alcoyloxycarbonyl-2 formylalcoyl-1 triazol-1,3,4 yle-5, alcoyloxycarbonyl-3 formylalcoyl-1 triazol-1,3,4 yle-5 ou formylalcoyl-1 tétrazolyle-5, par addition respectivement de cystéamine, d'un alcool, d'hydroxylamine ou d'une alcoyloxyamine selon les méthodes connues pour former des dérivés d'addition de fonctions carbonylées, puis s'il y a lieu élimination des radicaux protecteurs.

La réaction s'effectue généralement dans un solvant organique à une température comprise entre 20°C et la température de reflux du mélange réactionnel.

Les solvants organiques sont choisis en fonction de la solubilité des produits. Lorsque l'on met on œuvre un produit de formule générale (XXXVIIIc) dans laquelle $R_4$, $R_2$ et $R^c_5$ (contenu dans $R_5$) sont autres que l'hydrogène, on utilise avantageusement des solvants tels que le tétrahydrofuranne, l'acétonitrile, les alcools, les cétones. Lorsque l'on met en œuvre un produit de formule générale (XXXVIIIc) dans laquelle $R_4$, $R_2$ et $R^c_5$ (contenu dans $R_5$) sont des atomes d'hydrogène, on opère avantageusement dans des solvants tels que la pyridine, le diméthylsulfoxyde ou le diméthylformamide.

Lorsque l'on veut préparer un produit de formule générale (XXXVIII) pour lequel le radical R contient un substituant de formule générale (XXXIXc), on opère en milieu acide.

VIII°) Les thiovinyl-3 céphalosporines de formule générale (XXXVIII) dans laquelle $R^o_2$ représente un radical de formule générale (V) dans laquelle $R_8$ et $R_9$ sont définis comme précédemment, et $R^o_1$ a une définition correspondante peuvent aussi être obtenues par estérification d'un produit de formule générale (XXXVIII) dans laquelle $R^o_2$ représente un atome d'hydrogène et dont la fonction amine et le cas échéant la fonction acide de $R^o_1$ ont été préalablement protégées, par toute méthode connue en soi pour préparer un ester à partir d'un acide sans toucher au reste de la molécule.

On opère notamment dans les conditions décrites précédemment pour la préparation de produits de formule générale (XXII) ou (XXIV) dans lesquelles $R_2$ est un radical de formule générale (V).

IX°) Les thiovinyl-3 céphalosporines de formule générale (XXXVIII) dans laquelle R ne contient pas de substituant de formule générale (XXXIXc) et dans laquelle $R^o_1$ contient un radical de formule générale (IIa) peuvent aussi être obtenues par action d'un produit de formule générale (XXXVI) sur une céphalosporine de formule générale:

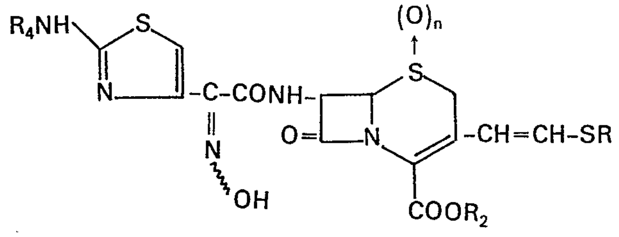

(XXXVIIId)

dans laquelle R, $R_2$, $R_4$ et n sont définis comme précédemment, à l'exception pour $R_4$ de représenter un atome d'hydrogène puis le cas échéant réduction du sulfoxyde obtenu et élimination des groupements protecteurs.

La fonction acide du produit de formule générale (XXXVI) peut être protégée par tout groupement protecteur défini précédemment pour la protection des radicaux carboxy.

Il est entendu que les groupements amino et alcoylamino qui existent dans certains radicaux R sont protégés et que les groupements formyle sont libres ou protégés.

La réaction s'effectue généralement en présence d'une base minérale ou organique (par exemple la soude, la potasse, l'hydrure de sodium, le carbonate de potassium ou une base azotée comme la triéthylamine), dans un solvant organique tel qu'un solvant chloré (par exemple dichlorométhane, dichloroéthane), un éther (par exemple tétrahydrofuranne, dioxanne), une cétone (par exemple acétone), un amide (par exemple diméthylformamide), éventuellement en présence d'eau.

La réduction du sulfoxyde et l'élimination des radicaux protecteurs s'effectuent dans les conditions décrites précédemment.

X°) Les thiovinyl-3 céphalosporines de formule générale (XXXVIII) dans laquelle R ne contient pas de substituant de formule générale (XXXIXc) peuvent également être obtenues à partir d'un produit ou d'un mélange des isomères du produit de formule générale (XVIa) par action d'un thiol de formule générale (XL) (ou d'un de ses sels alcalins ou alcalino-terreux) dans laquelle R qui est défini comme ci-dessus est protégé si nécessaire dans les conditions définies précédemment en I°), puis éventuellement réduction du sulfoxyde obtenu lorsque n = 1 et élimination des groupements protecteurs.

La réaction s'effectue dans les conditions décrites précédemment pour la préparation des produits de formule générale (XXXVIII) à partir d'un produit de formule générale (I) et d'un produit de formule générale (XL). Les conditions de protection (et de libération) des différents radicaux sont également les mêmes que pour le procédé décrit en I°).

Les produits de formules générales (XVIa), (XXXII) ou (XXXVII) dans lesquelles n = 1 peuvent être obtenus par oxydation des produits correspondants dans lesquels n = 0 selon la méthode décrite dans la demande de brevet DE 2 637 176.

Les isomères des produits de formule générales (I), (XVI), (XVIa), (XVIII), (XXXII), (XXXVII), (XXXVIII), (XLII), (XLIII), (XLVI) ou (XLVII) peuvent être séparés par chromatographie ou par cristallisation.

Les nouveaux produits de formule générale (I) dans laquelle existe un radical amino peuvent être transformés en sels d'addition avec acides. Selon les procédés indiqués, les produits sont éventuellement obtenus sous forme de trifluoroacétate, paratoluènesulfonate, méthanesulfonate, phosphate ou solvate avec l'acide formique. Les produits obtenus sous forme de ces sels peuvent être libérés et transformés en sels d'autres acides selon les méthodes habituelles.

Les produits de formule générale (I) qui contiennent un radical carboxy peuvent aussi être transformés en sels métalliques ou en sels d'addition avec les bases organiques azotées selon les méthodes connues en soi. Ces sels peuvent être obtenus par action d'une base métallique (par exemple alcaline ou alcalino-terreuse) ou d'une amine sur un produit de formule générale (I) dans un solvant approprié tel qu'un alcool, un éther ou l'eau ou par réaction d'échange avec un sel d'un acide organique. Le sel formé précipite après concentration éventuelle de sa solution, il est séparé par filtration ou décantation.

Comme exemples de sels peuvent être cités les sels avec les métaux alcalins (tels que les sels de potassium, de sodium ou de lithium) ou avec les métaux alcalino-terreux, les sels de bases azotées (sels de diméthylamine, de diéthylamine, de diisopropylamine, de dicyclohexylamine, de N-éthylpipéridine et de N-méthylmorpholine) et les sels d'addition avec les acides minéraux (tels que chlorhydrates ou bromhydrates) ou organiques (formiates, trifluoracétates, p.toluènesulfonates, naphthalènesulfonates ou oxalates).

Les dérivés de la céphalosporine de formule générale (XXXVIII) décrits en $\alpha_1$) et leurs sels pharmaceutiquement acceptables présentent des propriétés anti-bactériennes particulièrement intéressantes.

In vitro, ils se sont montrés actifs à une concentration comprise entre 0,5 et 10 µg/cm³ sur des souches de staphylocoques sensibles à la pénicilline G (Staphylococcus aureus Smith) et à une concentration comprise entre 0,01 et 2 µg/cm³ sur Escherichia coli souche NIHJ. De plus la majorité d'entre eux se sont montrés actifs à une concentration comprise entre 2 et 125 µg/cm³ sur Pseudomonas aeruginosa.

In vivo, ils se sont montrés actifs sur les infections expérimentales de la souris à Staphylococcus aureus Smith (sensible à la pénicilline G) à une dose comprise entre 0,5 et 15 mg/kg par jour par voie sous-cutanée, et à Escherichia coli souche NIHJ à des doses comprises entre 0,01 et 10 mg/kg par jour par voie sous-cutanée.

Par ailleurs, la $DL_{50}$ des produits de formule générale (XXXVIII) est comprise entre 1 g/kg et des doses supérieures à 2,5 g/kg par voie sous-cutanée chez la souris.

Les dérivés de la céphalosporine de formule générale (XXXVIII) tels que définis en β1) sont décrits pour leurs propriétés antibactériennes ou à titre d'intermédiaires pour la préparation de substances antibiotiques dans le brevet US 4 065 620.

D'un intérêt particulier sont les produits de formule générale (I) de forme E pour lesquels
$R_2$ représente un atome d'hydrogène ou un radical benzhydryle et
$R_3$ représente un atome d'halogène et $R_1$ représente un radical alcoyloxycarbonyle, un radical de formule générale (VIII) dans laquelle Ar est phényle et B est amino ou amino protégé ou un radical de formule générale (II) de forme syn pour lequel $R_4$ représente un radical trityle et $R_5$ représente un radical alcoyle, ou
$R_3$ représente un atome d'halogène ou un radical tosyloxy et $R_1$ représente un radical de formule générale (II) de forme syn pour lequel
$R_4$ représente un radical trityle et $R_5$ représente un radical de formule générale (IIa),
et plus spécialement ceux pour lesquels
soit $R_3$ représente un atome de chlore et $R_1$ représente un atome d'hydrogène, un radical t.butoxycarbonyle, un radical de formule générale (VIII) dans laquelle Ar est phényle et B est amino ou t.butoxycarbonylamino, ou un radical de formule générale (II) de forme syn dans laquelle $R_4$ est trityle et $R_5$ est méthyle,
soit $R_3$ représente un atome de chlore ou un radical tosyloxy et $R_1$ représente un radical de formule générale (II) de forme syn dans laquelle $R_4$ est trityle et $R_5$ représente un radical de formule générale (IIa).

Les exemples suivants, donnés à titre non limitatif, montrent comment l'invention peut être mise en pratique.

Dans ces exemples, les produits sont cités selon la nomenclature des Chemical Abstracts. Il est entendu que tous les produits qui sont cités présentent la stéréochimie donnée par la formule générale partielle

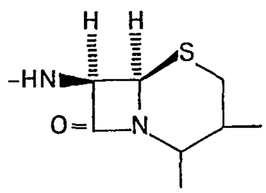

(XLVIII)

Exemple 1

A une solution refroidie à 5°C de 4,45 g de benzhydryloxycarbonyl-2 {[(t.butoxycarbonyl-2 propyl-2) oxyimino]-2 (tritylamino-2 thiazolyl-4)-2 acétamido}-7 formylméthyl-3 oxo-8 thia-5 aza-1 bicyclo [4.2.0] octène-2, isomère syn (produit 1a) dans 50 cm³ de pyridine, on ajoute 1,31 g de chlorure de p.toluènesulfonyle. On laisse remonter la température à 20°C en 30 minutes, agite pendant 1 heure à 20°C, verse dans 300 cm³ d'eau glacée, décante l'eau et reprend le produit pâteux insoluble dans 300 cm³ d'acétate d'éthyle. On lave par 100 cm³ d'acide chlorhydrique 1N, 100 cm³ d'une solution saturée de bicarbonate de sodium et 100 cm³ d'une solution saturée de chlorure de sodium, sèche sur sulfate de sodium, filtre et concentre à sec à 20°C sous 20 mm de mercure (2,7 kPa). Le produit ainsi obtenu est utilisé tel quel pour la suite de la synthèse. 1 g de ce produit est purifié par chromatographie sur une colonne de 20 g de gel de silice (0,05–0,2) (diamètre de la colonne: 1,7 cm) en éluant par un mélange de cyclohexane et d'acétate d'éthyle 80–20 (en volumes) et en recueillant des fractions de 50 cm³. Les fractions 4 à 12 contenant le produit pur sont évaporées à sec sous pression réduite (30 mm de mercure; 4 kPa) à 30°C pour donner 0,43 g de benzhydryloxycarbonyl-2 {[(t.butoxycarbonyl-2 propyl-2) oxyimino]-2 (tritylamino-2 thiazolyl-4)-2 acétamido}-7 oxo-8 (tosyloxy-2 vinyl)-3 thia-5

aza-1 bicyclo [4.2.0] octène-2, isomère syn, (produit 1) mélange des formes E et Z (dans des proportions - déterminées par RMN - de 75% de forme E et 25% de forme Z) sous la forme d'un solide jaune pâle.

Spectre infra-rouge ($CHBr_3$), bandes caractéristiques ($cm^{-1}$) 3400, 3260, 1790, 1720, 1680, 1630, 1595, 1580, 1520, 1490, 1450, 1380, 1370, 1190, 1180, 1070, 835, 750.

Spectre de RMN du proton (350 MHz, $CDCl_3$, δ en ppm, J en Hz) mélange d'isomères E et Z dans les proportions 75/25

forme E: 1,45 (s, 9H, $-C(CH_3)_3$); 1,62 et 1,67 (2s, 6H $=N-O-C(CH_3)_2-$); 2,48 (s, 3H, $-CH_3$ tosyl); 3,42 et 3,50 (2d, J = 18, 2H, $-S-CH_2-$); 5,08 (d, J = 4, 1H, -H en 6); 6,00 (dd, J = 4 et 9, 1H, -H en 7); 6,78 (s, 1H, H thiazole); 6,88 (mf, 1H, $-NH-C(C_6H_5)_3$); 6,90 et 6,97 (2d, J = 12, 2H, -CH=CH-S-); 6,92 (s, 1H, $-COO-CH(C_6H_5)_2$; 8,20 (d, J = 9, 1H, -CO-NH-)

forme Z: 6,20 et 6,47 (2d, J = 7, -CH=CH-S-Z); 2,45 (s, $-CH_3$ tosyl).

Le produit (1a) peut être obtenu de la manière suivante:

On agite pendant 1 heure à 20°C un mélange de 6,45 g de benzhydryloxycarbonyl-2 (diméthylamino-2 vinyl)-3 {[(t.butoxycarbonyl-2 propyl-2) oxyimino]-2 (tritylamino-2 thiazolyl-4)-2 acétamido}-7 oxo-8 thia-5 aza-1 bicyclo [4.2.0] octène-2, isomère syn, forme E (produit 1b) en solution dans 100 $cm^3$ d'acétate d'éthyle et 64 $cm^3$ d'acide chlorhydrique 1N. On décante, lave par 100 $cm^3$ d'eau, 100 $cm^3$ d'eau saturée de bicarbonate de sodium et 100 $cm^3$ d'eau saturée de chlorure de sodium, sèche sur sulfate de sodium, filtre et concentre à sec à 20°C sous 20 mm de mercure (2,7 kPa). On recueille 4,95 g de produit (1a) brut sous la forme d'une meringue brune.

Spectre infra-rouge ($CHBr_3$), bandes caractéristiques ($cm^{-1}$) 3400, 3270, 2720, 1770, 1725, 1685, 1525, 1495, 1450, 1370, 755, 700.

Spectre de RMN du proton (350 MHz, $CDCl_3$, δ en ppm, J en Hz) 1,45 (s, 9H, $-C(CH_3)_3$); 1,64 et 1,67 (2s, 6H, $(-CH_3)_2$); 3,25 et 3,54 (2d, J = 18, 2H, $-SCH_2-$); 3,50 et 3,73 (2d, J = 16, 2H, $-CH_2CHO$); 5,10 (d, J = 4, 1H, H en 6); 6,06 (dd, J = 4 et 9, 1H, H en 7); 6,77 (s, 1H, H du thiazole); 6,90 (s, 1H, -COOCH$<$); 8,22 (d, J = 9, 1H, -CONH-); 9,58 (s, 1H, -CHO).

Le produit (1b) peut être obtenu de la manière suivante:

A une solution à 80°C sous azote de 6 g de benzhydryloxycarbonyl-2 {[(t.butoxycarbonyl-2 propyl-2) oxyimino]-2 (tritylamino-2 thiazolyl-4)-2 acétamido}-7 méthyl-3 oxo-8 thia-5 aza-1 bicyclo [4.2.0] octène-2, isomère syn (produit 1c) dans 64 $cm^3$ de diméthylformamide, on ajoute sous agitation 1,9 $cm^3$ de t.butoxy bis-diméthylamino-méthane et poursuit la réaction pendant 15 minutes. On verse ensuite le mélange dans un mélange de 200 $cm^3$ d'acétate d'éthyle et 200 $cm^3$ d'eau, décante, lave par 3 fois 100 $cm^3$ d'eau et 100 $cm^3$ d'eau saturée de chlorure de sodium, sèche sur sulfate de sodium, filtre et concentre à sec à 20°C sous 20 mm de mercure (2,7 kPa). On obtient ainsi 6,5 g de produit (1b) brut sous la forme d'une meringue brune.

On purifie un échantillon (2,2 g) par chromatographie sur une colonne (diamètre 4 cm, hauteur 20 cm) de gel de silice Merck (0,04–0,06) en éluant par un mélange cyclohexane-acétate d'éthyle 65/35 (vol.) sous une pression de 40 kPa et en recueillant des fractions de 50 $cm^3$. Les fractions 14 à 24 sont concentrées à sec. On obtient 1,2 g de benzhydryloxycarbonyl-2 (diméthylamino-2 vinyl)-3 {[t.butoxycarbonyl-2 propyl-2) oxyimino]-2 (tritylamino-2 thiazolyl-4)-2 acétamido}-7 oxo-8 thia-5 aza-1 bicyclo [4.2.0] octène-2 isomère syn, forme E.

Spectre infra-rouge ($CHBr_3$), bandes caractéristiques ($cm^{-1}$) 3400, 3280, 1770, 1720, 1680, 1610, 1525, 1490, 1450, 1370, 940, 750, 735.

Spectre de RMN du proton (350 MHz, $CDCl_3$, δ en ppm, J en Hz) 1,45 (s, 9H, $-C(CH_3)_3$), 1,64 et 1,71 (2s, 6H, $=N-O-C(CH_3)_2-$); 2,93 (s, 6H, $-N(CH_3)_2$); 3,20 et 3,30 (2d, J = 14, 2H, $-S-CH_2-$); 5,18 (d, J = 4, 1H, -H en 6); 5,71 (dd, J = 4 et 9, 1H, -H en 7); 6,61 et 6,82 (2d, J = 14, 2H, -CH=CH-S-); 6,88 (s, 1H, H thiazole); 6,92 (s, 1H, $-COO-CH(C_6H_5)_2$); 6,92 (s large, 1H, $-NH-C(C_6H_5)_3$); 8,28 (d, J = 9, 1H, -CO-NH-).

Le benzhydryloxycarbonyl-2 {[(t.butoxycarbonyl-2 propyl-2) oxyimino]-2 (tritylamino-2 thiazolyl-4)-2 acétamido}-7 méthyl-3 oxo-8 thia-5 aza-1 bicyclo [4.2.0] octène-2, isomère syn peut être préparé de la manière suivante:

A une solution de 25,58 g d'acide (t.butoxycarbonyl-2 propyl-2 oxyimino)-2 (tritylamino-2 thiazolyl-4)-2 acétique préparé selon le brevet belge 876 541 dans un mélange de 250 $cm^3$ de dichlorométhane et 13,9 $cm^3$ de diméthylacétamide, on ajoute -10°C, en 30 minutes et sous agitation 40 $cm^3$ d'une solution 1,3 M de phosgène dans du chlorbenzène. On agite encore pendant 3 heures à -10°C et ajoute goutte à goutte en 1 heure 15 une solution de 16,29 g d'amino-7 benzhydryloxycarbonyl-2 méthyl-3 oxo-8 thia-5 aza-1 bicyclo [4.2.0] octène-2 dans 250 $cm^3$ de dichlorométhane. Après 1 heure 15 à -10°C, on lave le mélange par 200 $cm^3$ d'une solution aqueuse à 2% de bicarbonate de sodium et 200 $cm^3$ d'eau, sèche sur sulfate de sodium et concentre à sec à 30°C sous 20 mm de mercure (2,7 kPa). Le résidu est chromatographié sur une colonne de 200 g de gel de silice Merck (0,05–0,2) (diamètre de la colonne: 4 cm). On élue par un mélange cyclohexane - acétate d'éthyle 70–30 (en volumes) en recueillant des fractions de 120 $cm^3$. Les fractions 6 à 10 sont concentrées à sec à 30°C sous 20 mm de mercure (2,7 kPa). On recueille 8,5 g de benzhydryloxycarbonyl-2 [(t.butoxycarbonyl-2 propyl-2 oxyimino)-2 (tritylamino-2 thiazolyl-4)-2 acétamido]-7 méthyl-3 oxo-8 thia-5 aza-1 bicyclo [4.2.0] octène-2, isomère syn, sous la forme d'une meringue jaune.

Spectre infra-rouge ($CHBr_3$), bandes caractéristiques ($cm^{-1}$) 3400, 3270, 1790, 1725, 1685, 1525, 1500, 1450, 1380, 1370, 760, 740.

Exemple 2

On prépare une solution de composé d'addition du chlore et du triphénylphosphite en ajoutant, en 15 minutes, à 4 $cm^3$ d'une solution à 10% (poids/volume) de chlore dans du dichlorométhane refroidie à -5°C, une solution de 1,55 g de triphénylphosphite dans 5 $cm^3$ de dichlorométhane. Cette solution est ajoutée en 90 minutes à -10°C à une solution de 2,4 g de benzhydryloxycarbonyl-2 t.butoxycarbonylamino-7 oxo-8 (oxo-2 éthyl)-3 thia-5 aza-1 bicyclo [4.2.0] octène-2 (produit 2a) et de 0,4 $cm^3$ de pyridine dans 15 $cm^3$ de dichlorométhane. Le mélange réactionnel est lavé par 20 $cm^3$ d'eau distillée, 20 $cm^3$ de solution saturée de bicarbonate de sodium et par 2 fois 20 $cm^3$ d'eau distillée puis séché sur sulfate de magnésium et concentrée à sec sous pression réduite (30 mm de mercure; 4 kPa) à 30°C. On obtient 4,6 g d'un mélange équimoléculaire de benzhydryloxycarbonyl-2 t.butoxycarbonylamino-7 (dichloro-2,2 éthyl)-3 oxo-8 thia-5 aza-1 bicyclo [4.2.0] octène-2 (produit 2b) et de phosphate de triphényle. Ce mélange peut être utilisé sans purification supplémentaire pour la suite de la synthèse en le redissolvant dans 15 $cm^3$ de dichlorométhane sec et en ajoutant en 20 minutes à la solution refroidie à -10°C une solution de 1,22 g d'acide métachloroperbenzoïque à 85% dans 30 $cm^3$ de dichlorométhane. L'insoluble est éliminé par filtration et le filtrat est lavé par 2 fois 25 $cm^3$ de solution saturée de bicarbonate de sodium et 2 fois 25 $cm^3$ de solution saturée de chlorure de sodium puis séché sur sulfate de magnésium. Le résidu obtenu après évaporation du solvant sous pression réduite (30 mm de mercure; 4 kPa) à 35°C est chromatographié sur une colonne de silice (0,04-0,06) (diamètre de la colonne: 2 cm; hauteur: 30 cm) en éluant sous 50 kPa avec un mélange de cyclohexane et d'acétate d'éthyle (60-40 en volumes) et en recueillant des fractions de 30 $cm^3$. Les fractions 6 à 12 sont rassemblées et concentrées à sec sous pression réduite (30 mm de mercure; 4 kPa) à 40°C. On obtient 1,3 g d'une meringue jaune constituée principalement de benzhydryloxycarbonyl-2 t.butoxycarbonylamino-7 (dichloro-2,2 éthyl)-3 oxo-8 oxyde-5 thia-5 aza-1 bicyclo [4.2.0] octène-2 (produit 2c).

Spectre de masse: pic moléculaire m/e = 578.

Spectre de RMN du proton (350 MHz, $CDCl_3$, δ en ppm, J en Hz) 1,48 (s, 9H, $(CH_3)_3C-$); 3,10 (dd, J = 5 et 14, 1H, $CH_2-CHCl_2$); 3,66 (dd, J = 7 et 14, 1H, 2ème H du $CH_2-CHCl_2$); 3,41 et 3,96 (2d, J = 18, 2H, $-S(O)CH_2-$); 4,53 (d, J = 4, 1H, H en 6); 5,78 (d, J = 9, 1H, -CONH-); 5,83 (dd, J = 4 et 9, 1H, H en 7); 5,87 (dd, J = 5 et 7, 1H, $-CH_2CHCl_2$); 6,98 (s, 1H, $-CHAr_2$); 7,2 à 7,5 (m, 10H aromatiques).

On obtient le produit (2b) purifié en chromatographiant 4,6 g de mélange équimoléculaire de ce produit avec le phosphate de triphényle, tel qu'obtenu ci-dessus, sur une colonne de 30 g de gel de silice (0,2-0,06) en éluant par du dichlorométhane et en recueillant des fractions de 10 $cm^3$. Les fractions 2 à 11 sont réunies et concentrées à sec sous pression réduite (30 mm de mercure; 4 kPa) à 30°C. Le résidu obtenu est soumis à une nouvelle chromatographie sur une colonne de silice (0,04-0,06) (diamètre de la colonne: 2 cm; hauteur: 30 cm) en éluant sous 50 kPa par un mélange de cyclohexane et d'acétate d'éthyle (80-20 en volumes) et recueillant des fractions de 10 $cm^3$. Les fractions 7 et 8 contenant le produit pur sont concentrées à sec sous pression réduite (30 mm de mercure; 4 kPa) à 35°C et le produit séché sous pression réduite (0,2 mm de mercure; 0,027 kPa) à 25°C.

Spectre de masse: pic moléculaire m/e = 562.

Spectre de RMN du proton (350 MHz, $CDCl_3$, δ en ppm, J en Hz) 1,46 (s, 9H, $(CH_3)_3C-$); 3,20 et 3,28 (2dd, J = 9 et 14, 2H, $-CH_2-CHCl_2$); 3,65 (AB limite, J = 18, 2H, $-SCH_2-$); 5,0 (d, J = 4, 1H, H en 6); 5,25 (d, J = 9, 1H, -CONH-); 5,67 (dd, J = 4 et 9, 1H, H en 7); 5,98 (dd, J = 9 et 4, 1H, $-CH_2-CHCl_2$); 6,95 (s, 1H, $-CO_2CH(C_6H_5)_2$); 7,20 à 7,50 (m, 10 H aromatiques).

On agite à 25°C pendant 16 heures une solution de 3,3 g de produit (2c) et de 0,88 $cm^3$ de triéthylamine dans 50 $cm^3$ de tétrahydrofuranne sec puis dilue le mélange réactionnel par 200 $cm^3$ d'acétate d'éthyle. La solution organique est lavée par 5 fois 60 $cm^3$ d'eau distillée, séchée sur sulfate de sodium et concentrée à sec sous pression réduite (30 mm de mercure; 4 kPa) à 35°C. On obtient 3,1 g de benzhydryloxycarbonyl-2 t.butoxycarbonylamino-7 (chloro-2 vinyl)-3 oxo-8 oxyde-5 thia-5 aza-1 bicyclo [4.2.0] octène-2, forme E (produit 2) sous la forme d'une merinque orangée.

Spectre infra-rouge ($CHBr_3$), bandes caractéristiques ($cm^{-1}$) 3400, 1800, 1715, 1590, 1570, 1500, 1450, 1390, 1365, 1040, 960, 755.

Spectre de RMN du proton (350 MHz, $CDCl_3$, δ en ppm, J en Hz) 1,48 (s, 9H, $(CH_3)_3C-$); 3,20 et 3,94 (2d, J = 18, 2H, $-S(O)CH_2-$); 4,53 (d, J = 4, 1H, H en 6); 5,78 (d, J = 9, 1H, -CONH—); 5,86 (dd, J = 4 et 9, 1H, H en 7); 6,45 (d, J = 14, 1H, -CH=CHCl); 7,03 (s, 1H, $-CO_2CH(C_6H_5)_2$); 7,49 (d, J = 14, 1H, -CH=CHCl); 7,20 à 7,60 (m, 10H, aromatiques).

Le produit (2a) peut être obtenu de la façon suivante:

Une solution de 1,07 g de benzhydryloxycarbonyl-2 t.butoxycarbonylamino-7 (diméthylamino-2 vinyl)-3 oxo-8 thia-5 aza-1 bicyclo [4.2.0] octène-2, forme E (produit 2d) dans 10 $cm^3$ d'acétate d'éthyle est agitée pendant 1 heure à 25°C avec 5 $cm^3$ de solution aqueuse 1N d'acide chlorhydrique. La phase organique est décantée, lavée 4 fois par 50 $cm^3$ de solution aqueuse saturée de chlorure de sodium puis séchée sur sulfate de magnésium et filtrée. L'évaporation à sec du solvant sous pression réduite donne 1 g d'un produit dont le spectre IR montre qu'il s'agit principalement de produit (2a).

Rf = 0,57 [chromatoplaque de gel de silice; éluant: cyclohexane-acétate d'éthyle 60-40 (en volumes)]

Spectre infra-rouge (solution $CHBr_3$), bandes caractéristiques ($cm^{-1}$) 2840, 1785, 1720.

Spectre de RMN du proton (350 MHz, $CDCl_3$, δ en ppm, J en Hz) 1,47 (s, 9H, $(CH_3)_3C-O-$); 3,24 et

3,55 (AB, J = 18, 2H, $-SCH_2-$); 3,50 et 3,66 (AB, J = 16, 2H, $-CH_2CHO$); 4,98 (d, J = 4,5, 1H, H en 6); 5,25 (d, J = 9, 1H, -CONH-); 5,65 (dd, J = 4,5 et 9, 1H, H en 7); 6,87 (s, 1H, $-CO_2CH<$); 7,2 à 7,5 (massif, 10H, aromatiques); 9,54 (s, 1H, -CHO).

Le produit (2d) peut être obtenu en opérant de la manière suivante:

Une solution de 1,0 g de benzhydryloxycarbonyl-2 t.butoxycarbonylamino-7 méthyl-3 oxo-8 thia-5 aza-1 bicyclo [4.2.0] octène-3 (produit 2e) dans 100 $cm^3$ de N,N-dimethylformamide anhydre est chauffée à 80°C en atmosphère d'azote. On ajoute alors rapidement 0,86 $cm^3$ de bisdiméthylamino t.butoxyméthane. Le mélange réactionnel est maintenu à 80°C pendant 5 minutes puis versé dans 50 $cm^3$ d'acétate d'éthyle. Après addition de 25 $cm^3$ d'eau distillée, la phase organique est décantée, lavée par 4 fois 25 $cm^3$ d'eau distillée, séchée sur sulfate de magnésium et filtrée. On concentre à sec sous pression réduite (20 mm de mercure: 2,7 kPa) à 30°C et obtient 1,10 g d'un produit constitué principalement de produit (2d) sous la forme d'une meringue orangée.

Rf = 0,29; chromatoplaque de gel de silice [dichlorométhaneacétate d'éthyle 50-50 (en volumes)].

Spectre infra-rouge ($CHBr_3$), bandes caractéristiques ($cm^{-1}$) 3430, 3350, 2820, 1765, 1715, 1690, 1615, 1540, 1505, 1495, 1465, 1370, 1240, 940, 745, 600.

Spectre UV visible - Ethanol

$\lambda$ max = 390 nm $\varepsilon$ = 29 000 (c = $2.10^{-5}$M)

Spectre de masse: pic moléculaire = 535 fragments caractéristiques m/e = 378 et 379 (coupure du $\beta$-lactame).

Spectre de RMN du proton (350 MHz, $CDCl_3$, $\delta$ en ppm, J en Hz) 1,48 (s, $(CH_3)_3C-OCO-$, 9H); 2,89 (s, 6H, $(CH_3)_2N-$); 3,17 (AB, J = 14, 2H, $-S-CH_2-$céphème); 5,02 (d, J = 4, 1H, H en 6); 5,27 (dd, J = 4 et 9, 1H, H en 7); 5,60 (d, J = 9, 1H, -OCONH-); 6,71 (d, J = 14, 1H, $-CH=CH-N<$); 6,49 (d, J = 14, 1H, $-CH=CHN<$); 6,95 (s, 1H, $-CH(C_6H_5)_2$); 7,2 à 7,5 (massif, aromatiques, 10H).

Le produit (2e) peut être préparé par estérification de 3,2 g de t.butoxycarbonylamino-7 carboxy-2 méthyl-3 oxo-8 thia-5 aza-1 bicyclo [4.2.0] octène-3, (produit 2f), par 2,1 g de diphényldiazométhane entre 25 et 30°C pendant 16 heures. Après recristallisation dans un mélange cyclohexane-acétate d'éthylo 90-10 (en volumes) on obtient 2,3 g de produit (2e) sous la forme de cristaux blancs (F = 161°C).

Le produit (2f) peut être préparé par conversion de 8,28 g du t.butoxycarbonylamino-7 méthoxycarbonyl-2 méthyl-3 oxo-8 thia-5 aza-1 bicyclo [4.2.0] octène-2 (produit 2 g) en utilisant la méthode de R. B. Morin et coll., J. Amer. Chem. Soc., 91 (6), 1401 (1969). On obtient 5,4 g de produit (2f).

F = 200°C (déc.) (après recristallisation dans l'acétate d'éthyle).

Rf = 0,59 [chromatoplaque de gel de silice; éluant: mélange acétate d'éthyle-acétone-eau-acide formique 60-20-1-1 (en volumes)].

Le produit (2 g) peut être préparé en estérifiant 16,7 g de t.butoxycarbonylamino-7 carboxy-2 méthyl-3 oxo-8 thia-5 aza-1 bicyclo [4.2.0] octène-2 (produit 2 h) par une solution éthérée de diazométhane selon R. B. Morin et coll., J. Amer. Chem. Soc., 91 (6), 1401 (1969). On obtient 13,6 g de produit (2 g) sous la forme de cristaux blancs (F = 148°C).

Rf = 0,45 [chromatoplaque de gel de silice; éluant: cyclohexane-acétate d'éthyle 60-40 (vol.)]

Exemple 3

A une solution refroidie à 0°C de 3 g de benzhydryloxycarbonyl-2 {[(t.butoxycarbonyl-2 propyl-2) oxyimino]-2 (tritylamino-2 thiazolyl-4)-2 acétamido}-7 oxo-8 (tosyloxy-2 vinyl)-3 thia-5 aza-1 bicyclo [4.2.0] octène-2, insomère syn, (produit 3a, mélange à 75% de forme E et 25% de forme Z) dans 50 $cm^3$ de dichlorométhane, on ajoute goutte à goutte en 25 minutes une solution de 0,464 g d'acide m.chloroperbenzoïque à 90% dans 10 $cm^3$ de dichlorométhane. On agite pendant 1 heure à 0°C, dilue par 500 $cm^3$ d'acétate d'éthyle, lave par 2 fois 100 $cm^3$ d'une solution à 2% de bicarbonate de sodium, 2 fois 100 $cm^3$ d'eau et 100 $cm^3$ d'eau saturée de chlorure de sodium, sèche sur sulfate de sodium, filtre et concentre à sec à 20°C sous 20 mm de mercure (2,7 kPa). Le résidu est chromatographié sur une colonne de 60 g de gel de silice Merck (0,06-0,2) (diamètre de la colonne: 2 cm, hauteur: 20 cm), on élue par 1 litre d'un mélange cyclohexane-acétate d'éthyle (70-30 en volumes) en recueillant des fractions de 60 $cm^3$. Les fractions 5 à 14 sont concentrées à sec à 20°C sous 20 mm de mercure (2,7 kPa), on recueille 1,9 g de benzhydryloxycarbonyl-2 {[(t.butoxycarbonyl-2 propyl-2) oxyimino]-2 (tritylamino-2 thiazolyl-4)-2 acétamido}-7 oxo-8 oxyde-5 (tosyloxy-2 vinyl)-3 thia-5 aza-1 bicylo [4.2.0] octène-2, isomère syn, forme E (produit 3) sous la forme d'une merinque jaune.

Spectre infra-rouge ($CHBr_3$), bandes caractéristiques ($cm^{-1}$) 3380, 1800, 1720, 1680, 1590, 1580, 1510, 1490, 1445, 1375, 1190, 1175, 1070, 730

Spectre de RMN du proton (350 MHz), DMSO $d_6$, $\delta$ en ppm, J en Hz) 1,37 (s, 9H, $-C(CH_3)_3$); 1,45 et 1,46 (2s, 6H, $-O\ C(CH_3)_2-$); 2,44 (s, 3H, $-CH_3$ tosyle); 3,60 et 4,41 (2d, J = 18, 2H, $-SCH_2-$); 5,06 (d, J = 4, 1H, H en 6); 5,96 (dd, J = 4 et 9, 1H, H en 7); 6,75 (d, J = 13, 1H, -CH=CHS-); 6,73 (s, 1H, H du thiazole); 6,93 (s, 1H, -COOCH-); 7,48 et 7,84 (ab, 2H, J = 9); 8,16 (d, J = 9, 1H, -CONH-); 8,73 (s, 1H, $-NH\ C(C_6H_5)_3$).

Exemple 4

A une solution de 3,4 g de benzhydryloxycarbonyl-2 t.butoxycarbonylamino-7 (chloro-2 vinyl)-3 oxo-8 oxyde-5 thia-5 aza-1 bicyclo [4.2.0] octène-2, forme E dans 15 $cm^3$ d'acétonitrile à 40°C on ajoute en 15 minutes une solution de 2,2 g d'acide paratoluènesulfonique (hydrate) dans 15 $cm^3$ d'acétonitrile. Après 30 minutes à 40°C le mélange réactionnel est concentré à sec sous pression réduite (30 mm de mercure; 4 kPa) à 30°C. Le résidu est repris dans 100 cm³ d'acétate d'éthyle et la solution obtenue est lavée par 2 fois 50 cm³ de solution saturée de bicarbonate de sodium et 2 fois 50 cm³ d'eau distillée puis séchée sur sulfate de sodium et concentrée à sec sous pression réduite (30 mm de mercure; 4 kPa) à 35°C. Le résidu est séché sous pression réduite (0,2 mm de mercure; 0,03 kPa) à 25°C pendant 1 heure. On obtient 2,5 g d'amino-7 benzhydryloxycarbonyl-2 (chloro-2 vinyl)-3 oxo-8 oxyde-5 thia-5 aza-1 bicyclo [4.2.0] octène-2, forme E, brut sous la forme d'une meringue orangée. Ce produit est redissous dans 45 cm³ de dichlorométhane sec et on ajoute une solution de 2,5 g d'acide méthoxyimino-2 (tritylamino-2 thiazolyl-4)-2 acétique (isomère syn) et de 0,03 g de diméthylamino-4 pyridine dans 30 cm³ de dichlorométhane sec puis (après refroidissement vers 4°C) 1,3 g de N,N'-dicyclohexylcarbodiimide et 10 cm³ de dichlorométhane sec. Le mélange réactionnel est agité pendant 40 minutes vers 6°C puis 16 heures à 25°C. On filtre le précipité de N,N'-dicyclohexylurée, que l'on rince par 2 fois 5 cm³ de dichlorométhane sec. Le filtrat et les lavages rassemblés sont concentrés à sec sous pression réduite (30 mm de mercure; 4 kPa) à 30°C et le résidu obtenu est repris dans 200 cm³ d'acétate d'éthyle. La solution obtenue est lavée successivement par 50 cm³ d'acide chlorhydrique 0,2N, 2 fois 50 cm³ de solution demi-saturée de bicarbonate de sodium et 50 cm³ de solution saturée de chlorure de sodium puis séchée sur sulfate de sodium. Le résidu obtenu après évaporation à sec sous pression réduite (30 mm de mercure; 4 kPa) à 30°C est chromatographié sur une solonne de silice (0,04–0,06) (diamètre de la colonne: 4 cm, hauteur: 30 cm) en éluant sous une pression de 50 kPa par 800 cm³ de dichlorométhane puis par 1200 cm³ de mélange de dichlorométhane et d'acétate d'éthyle (95–5 en volumes) en recueillant des fractions de 60 cm³. Les fractions 12 à 25 contenant le produit pur sont rassemblées et concentrées à sec sous pression réduite. On obtient 1,7 g de benzhydryloxycarbonyl-2 (chloro-2 vinyl)-3 [méthoxyimino-2 (tritylamino-2 thiazolyl-4)-2 acétamido]-7 oxo-8 oxyde-5 thia-5 aza-1 bicyclo [4.2.0] octène-2, isomère syn, forme E, sous la forme d'une meringue de couleur crème.

Spectre infra-rouge ($CHBr_3$), bandes caractéristiques ($cm^{-1}$ 3380, 2820, 1800, 1725, 1680, 1595, 1585, 1570, 1515, 1495, 1450, 1210, 1040, 930, 750.

Spectre de RMN du proton (350 MHz, $CDCl_3$, δ en ppm, J en Hz) 3,22 et 3,92 (2d, J = 18, 2H, $-S-CH_2-$); 4,12 (s, 3H, $=N-O-CH_3$); 4,63 (d, J = 5, 1H, -H en 6); 6,21 (dd, J = 5 et 9, 1H, -H en 7); 6,44 et 7,50 (2d, J = 14, 2H, -CH=CHCl); 6,74 (s, 1H, -H thiazole); 7,01 (s, 1H, $-COO-CH(C_6H_5)_2$); 7,11 (mf, 1H, $-NH-C(C_6H_5)_3$); 7,2 à 7,6 (mt, aromatiques); 7,54 (d, J = 9, -CO-NH-).

Exemple 5

A une solution de 9,84 g d'amino-7 benzhydryloxycarbonyl-2 oxo-8 oxyde-5 (tosyloxy-2 vinyl)-3 thia-5 aza-1 bicyclo [4.2.0] octène-2, forme E (produit 5a) dans 170 cm³ de dichlorométhane, on ajoute 11,66 g d'acide [(t.butoxycarbonyl-2 propyl-2) oxyimino]-2 (tritylamino-2 thiazolyl-4)-2 acétique, isomère syn (préparé selon le procédé décrit dans le brevet belge 876 541) et 0,1 g de diméthylamino-4 pyridine. On ajoute 50 cm³ de diméthylformamide pour obtenir une solution limpide, refroidit entre 0°C et 5°C et ajoute en 15 minutes, sous agitation, une solution de 4,21 g de N,N'-dicyclohexylcarbodiimide dans 50 cm³ de dichlorométhane. On agite pendant 2 heures à 5°C et 2 heures à 20°C et concentre le mélange à 20°C sous 20 mm de mercure (2,7 kPa). Le résidu est repris dans 150 cm³ d'acétate d'éthyle, on filtre, lave par 3 fois 100 cm³ d'eau, 2 fois 100 cm³ d'eau demi-saturée de bicarbonate de sodium, sèche sur sulfate de sodium, filtre et concentre à sec à 20°C sous 20 mm de mercure (2,7 kPa). On chromatographie le résidu sur une colonne de 400 g de gel de silice Merck (0,06–0,2) (diamètre de la colonne: 4 cm, hauteur: 76 cm), on élue par 1 litre d'un mélange cyclohexane-acétate d'éthyle 80–20 (en volumes) et 4 litres d'un mélange 70–30 en recueillant des fractions de 250 cm³. On concentre à sec les fractions 6 à 11 à 20°C sous 20 mm de mercure (2,7 kPa) et recueille 6,95 g de benzhydryloxycarbonyl-2 {[(t.butoxycarbonyl-2 propyl-2) oxyimino]-2 (tritylamino-2 thiazolyl-4)-2 acétamido}-7 oxo-8 oxyde-5 (tosyloxy-2 vinyl)-3 thia-5 aza-1 bicyclo [4.2.0] octène-2, isomère syn, forme E (produit 5) sous la forme d'une meringue jaune dont les caractéristiques sont identiques à celles du produit de l'exemple 3.

Le produit (5a) peut être obtenu de la manière suivante:

On agite à 35°C pendant 2 heures une solution de 54,3 g de benzhydryloxycarbonyl-2 t.butoxycarbonylamino-7 oxo-8 oxyde-5 (tosyloxy-2 vinyl)-3 thia-5 aza-1 bicyclo [4.2.0] octène-2 (produit 5b), forme E et de 30,4 g d'acide p.toluènesulfonique hydraté dans 1,4 litre d'acétonitrile. On concentre à sec à 30°C sous 20 mm de mercure (2,7 kPa), reprend dans 1 litre d'acétate d'éthyle, lave par 2 fois 500 cm³ d'une solution demi-saturée de bicarbonate de sodium et 2 fois 500 cm³ d'une solution demi-saturée de chlorure de sodium, sèche sur sulfate de sodium et concentre à sec à 20°C sous 20 mm de mercure (2,7 kPa). Le résidu est trituré dans 200 cm³ d'éther. On obtient 28,13 g de produit (5a) sous la forme d'une poudre brun clair.

Rf = 0,32 chromatoplaque de gel de silice [dichlorométhaneméthanol 85–15 (vol.)].

Le produit (5b) peut être préparé de la manière suivante:

Dans une solution refroidie à -10°C de 180,56 g de benzhydryloxycarbonyl-2 t.butoxycarbonylamino-7 oxo-8 (tosyloxy-2 vinyl)-3 thia-5 aza-1 bicyclo [4.2.0] octène-2 (ou -3), (produit 5c, mélange des formes E et Z) dans 1,4 litre de dichlorométhane, on ajoute goutte à goutte en 2 heures une solution de 55,22 g d'acide m.chloroperbenzoïque à 85% dans 600 cm³ de dichlorométhane. Le mélange est lavé par 1,5 litre d'une solution à 5% de bicarbonate de sodium et 2 fois 1,5 litre d'eau, séché sur sulfate de sodium et concentré à

20°C sous pression réduite (20 mm de mercure) jusqu'au volume de 300 $cm^3$. Cette solution est chromatographiée sur une colonne de 3 kg de gel de silice Merck (0,05–0,2) (diamètre de la colonne: 9,2 cm; hauteur: 145 cm). On élue par des mélanges cyclohexane-acétate d'éthyle, successivement: 15 litres [80–20 (en volumes)] et 32 litres [70–30 (en volumes)] en recueillant des fractions de 600 $cm^3$. Les fractions 27 et 28 sont recueillies et concentrées à sec, on obtient 5,56 g de la forme Z du produit (5b).

Spectre infra-rouge ($CHBr_3$), bandes caractéristiques ($cm^{-1}$) 3420, 1800, 1720, 1505, 1380, 1370, 1195, 1180, 1050, 1010, 730.

Spectre de RMN du proton (350 MHz, $CDCl_3$, δ en ppm, J en Hz) 1,49 (s, 9H, $-C(CH_3)_3$); 2,44 (s, 3H, $-CH_3$); 3,36 et 4,04 (2d, J = 19, 2H, $-SCH_2-$); 4,44 (d, J = 4,5, 1H, H en 6); 5,73 (d, J = 9, 1H, -CONH-); 5,81 (dd, J = 4,5 et 9, 1H, H en 7); 6,42 (d, J = 7, 1H, $-CH=CH\ OSO_2-$); 6,46 (d, J = 7, 1H, $= CH\ OSO_2-$); 6,89 (s, 1H, $-COOCH<$); 7,77 (d, J = 9, 2H, H en ortho du tosyle).

Dans les fractions 29 à 34, on obtient 26 g du mélange des formes Z et E.

Enfin dans les fractions 35 à 58, on obtient 43 g de la forme E du produit (5b):

Spectre infra-rouge ($CHBr_3$), bandes caractéristiques ($cm^{-1}$) 3420, 1800, 1720, 1505, 1380, 1370, 1195, 1180, 1075, 935, 745.

Spectre de RMN du proton (350 MHz, $CDCl_3$, δ en ppm, J en Hz) 1,48 (s, 9H, $(CH_3)_3C-$); 2,46 (s, 3H, $-CH_3$); 3,16 et 3,81 (2d, J = 18, 2H, $-SCH_2-$); 4,46 (d, J = 4,5, 1H, H en 6); 5,73 (d, J = 9, 1H, -CONH-); 5,8 (dd, J = 9 et 4,5, 1H, H en 7); 6,83 (d, J = 13, 1H, $-CH=CH\ OSO_2-$); 6,83 (s, 1H, $-COOCH<$); 7,08 (d, J = 13, 1H, $=CH\ OSO_2-$); 7,73 (d, J = 9, 2H, H en ortho du tosyle).

Le produit (5c), mélange des formes E et Z, peut être obtenu de la manière suivante:

A une solution de 113,7 g de benzhydryloxycarbonyl-2 t.butoxycarbonylamino-7 (diméthylamino-2 vinyl)-3 oxo-8 thia-5 aza-1 bicyclo [4.2.0] octène-2 (forme E) (produit 5d) dans 1 litre de tétrahydrofuranne, on ajoute une solution de 50 $cm^3$ d'acide formique dans 500 $cm^3$ d'eau. On agite la solution homogène à 20°C pendant 20 minutes puis on la concentre au quart de son volume sous pression réduite (20 mm de mercure) à 20°C. On reprend le concentrat dans 2 litres d'acétate d'éthyle, lave par 2 fois 500 $cm^3$ d'une solution à 5% de bicarbonate de sodium, 2 fois 500 $cm^3$ d'eau et 2 fois 500 $cm^3$ d'une solution saturée de chlorure de sodium, sèche sur sulfate de sodium, filtre et évapore à sec à 20°C sous pression réduite (20 mm de mercure). On recueille 112,4 g de produit brut qui sont traités en solution dans 250 $cm^3$ de pyridine anhydre à 5°C par 57,2 g de chlorure de tosyle. Après 30 minutes à 5°C et 1 heure à 20°C, on verse la solution dans 1 litre d'un mélange eau-glace pilée. On sépare la phase aqueuse et lave l'insoluble par 300 $cm^3$ d'eau distillée. Le produit pâteux est mis en solution dans 200 $cm^3$ d'acétate d'éthyle, on lave par 2 fois 750 $cm^3$ d'acide chlorhydrique 1 N, 2 fois 750 $cm^3$ d'une solution à 5% de bicarbonate de sodium et 4 fois 750 $cm^3$ d'eau, sèche sur sulfate de sodium et concentre à sec sous pression réduite (20 mm de mercure) à 20°C. On obtient 121 g de produit constitué principalement de produit (5c) (mélange des formes E et Z) sous la forme d'une meringue brune brute.

Le produit (5d) est préparé comme décrit ci-dessus à l'exemple 2 pour le produit (2d).

Exemple 6

Une solution de 6 g de benzhydryloxycarbonyl-2 t.butoxycarbonylamino-7 (chloro-2 vinyl)-3 oxo-8 oxyde-5 thia-5 aza-1 bicyclo [4.2.0] octène-2, forme E dans 150 $cm^3$ de dichlorométhane et 4,06 $cm^3$ de diméthylacétamide est refroidie à -10°C, et on ajoute en 5 minutes sous agitation une solution de 2,02 g de trichlorure de phosphore dans 15 $cm^3$ de dichlorométhane. Le mélange est encore laissé 30 minutes sous agitation, puis il est concentré à sec sous pression réduite (30 mm de mercure; 4 kPa) à 30°C. Le résidu est dilué dans 250 $cm^3$ d'acétate d'éthyle, la phase organique est lavée par deux fois 200 $cm^3$ d'une solution saturée de bicarbonate de sodium, séchée sur sulfate de magnésium, filtrée et concentrée à sec sous pression réduite (20 mm de mercure; 2,7 kPa) à 30°C. Le résidu obtenu est chromatographié sur une colonne de 60 g de gel de silice Merck (0,2–0,06) (diamètre de la colonne: 3 cm). On élue par un mélange acétate d'éthyle-cyclohexane 30–70 (en volumes) en recueillant des fractions de 60 $cm^3$. Les fractions 2 à 4 sont concentrées à sec sous pression réduite. On obtient ainsi 4,1 g de benzhydryloxycarbonyl-2 t.butoxycarbonylamino-7 (chloro-2 vinyl)-3 oxo-8 thia-5 aza-1 bicyclo [4.2.0] octène-2, forme E sous forme d'une merinque jaune.

Rf = 0,47 [chromatoplaque de gel de silice; éluant: acétate d'éthyle-cyclohexane; 30–40 (en volumes)].

Exemple 7

A une solution de 4,1 g de benzhydryloxycarbonyl-2 t.butoxycarbonylamino-7 (chloro-2 vinyl)-3 oxo-8 thia-5 aza-1 bicyclo [4.2.0] octène-2, forme E dans 41 $cm^3$ d'acétonitrile on ajoute en 30 secondes 4,1 $cm^3$ d'acide méthanesulfonique et on laisse pendant 5 minutes sous agitation à 20°C. On verse alors le mélange dans 200 $cm^3$ d'une solution saturée de bicarbonate de sodium, ajoute 250 $cm^3$ d'eau et extrait par 200 $cm^3$ de dichlorométhane. La phase organique est séchée sur sulfate de magnésium, filtrée et concentrée à 100 $cm^3$ sous pression réduite (30 mm de mercure; 4 kPa) à 25°C. On obtient ainsi une solution d'amino-7 benzhydryloxycarbonyl-2 (chloro-2 vinyl)-3 oxo-8 thia-5 aza-1 bicyclo [4.2.0] octène-2, que l'on fait réagir avec 1,95 g d'acide Dα-t.butoxycarbonylaminophénylacétique en présence de 1,9 g de dicyclohexylcarbodiimide pendant 90 minutes à 4°C. Le mélange réactionnel est alors filtré pour séparer la dicyclohexylurée précipitée, le filtrat est concentré à sec sous pression réduite (20 mm de mercure; 2,7 kPa) à 30°C et le résidu est chromatographié sur une colonne de silice

(0,04–0,06) (diamètre de la colonne: 3,5 cm, hauteur: 25 cm) en éluant sous 50 kPa par un mélange acétate d'éthyle-cyclohexane 35–65 (en volumes) en recueillant des fractions de 100 $cm^3$. Les fractions 4 à 6 contenant le produit sont concentrées à sec sous pression réduite (20 mm de mercure; 2,7 kPa) à 30°C. On obtient ainsi 2,1 g de (D,α-t.butoxycarbonylaminophénylacétamido)-7 benzhydryloxycarbonyl-2 (chloro-2 vinyl)-3 oxo-8 thia-5 aza-1 bicyclo [4.2.0] octène-2, forme E sous forme d'une meringue jaune.

Rf = 0,50 [chromatoplaque de gel de silice; éluant: acétate d'éthyle-cyclohexane; 40–60 (en volumes)].

Spectre de RMN du proton (250 MHz, $CDCl_3$, δ en ppm, J en Hz) 1,42 (s, 9H, $-C(CH_3)_3$); 3,39 (s, 2H, $-SCH_2-$); 4,93 (d, J = 4, 1H, H en 6); 5,21 (m, 1H, -CONHCH$<$); 5,60 (d, J = 6, 1H, -CONHCH$<$); 5,80 (dd, J = 4 et 9, H en 7); 6,37 (d, J = 14, 1H, -C= trans).

On dissout 2,1 g de benzhydryloxycarbonyl-2 (D,α-t.butoxycarbonylaminophénylacétamido)-7 (chloro-2 vinyl)-3 oxo-8 thia-5 aza-1 bicyclo [4.2.0] octène-2, forme E dans 5 $cm^3$ d'anisole et 25 $cm^3$ d'acide trifluoroacétique à 20°C. La solution jaune est immédiatement concentrée à sec sous pression réduite (2 mm de mercure, 270 Pa) à 30°C. Le résidu est dissous dans 15 $cm^3$ d'acétate d'éthyle et la solution est versée, sous agitation, dans 200 $cm^3$ d'éther isopropylique. Le précipité formé est séparé par filtration et séché sous vide (2 mm de mercure; 270 Pa) à 25°C. On obtient ainsi 1,5 g de trifluoroacétate de (Dα-aminophénylacétamido)-7 carboxy-2 (chloro-2 vinyl)-3 oxo-8 thia-5 aza-1 bicyclo [4.2.0] octène-2, forme E sous forme d'un solide blanchâtre.

Rf = 0,32 [chromatoplaque de gel de silice; éluant: acétate d'éthyle-acide formique-eau; 6-1-1 (en volumes)].

Spectre de RMN du proton (250 MHz, DMSO $d_6$, δ en ppm, J en Hz) 3,46 (s, large, 2H, $-SCH_2-$); 4,95 (d, J = 4, 1H, H en 6); 5,10 (s, 1H, $>$CHNH$_2$); 5,72 (dd, J = 4 et 7,5, 1H, H en 7); 6,51 et 7,29 (2d, J = 14, 2H, -CH=CH- trans); 9,57 (d, J = 7,5, 1H, -CONH-).

Les intermédiaires dihalogénés des produits selon l'invention peuvent être isolés de la manière suivante:

Exemple 8

En opérant selon le mode opératoire décrit dans l'exemple 2 on traite une solution de 2,3 g de benzhydryloxycarbonyl-2 [méthoxyimino-2 (tritylamino-2 thiazolyl-4)-2 acétamido]-7 oxo-8 (oxo-2 éthyl)-3 thia-5 aza-1 bicyclo [4.2.0] octène-2, isomère syn (produit 8a) dans 15 $cm^3$ de dichlorométhane refroidie à -40°C par 5,5 $cm^3$ d'une solution 0,5 M dans le dichlorométhane du composé d'addition du chlore et du triphénylphosphite. Le mélange réactionnel est agité pendant 3 heures à une température comprise entre -40°C et -20°C puis dilué par 200 $cm^3$ d'acétate d'éthyle. La solution organique décantée est lavée par 50 $cm^3$ de solution demi-saturée en bicarbonate de sodium et en chlorure de sodium puis 3 fois 50 $cm^3$ de solution saturée de chlorure de sodium. Les phases aqueuses sont réextraites par 50 $cm^3$ d'acétate d'éthyle et les solutions organiques rassemblées sont concentrées à sec sous pression réduite (30 mm de mercure; 4 kPa) à 30°C. Le résidu est agité pendant 16 heures avec 150 $cm^3$ d'oxyde d'isopropyle. Le précipité est essoré et lavé par 50 $cm^3$ d'oxyde d'isopropyle puis redissous dans 50 $cm^3$ de dichlorométhane et fixé sur 8 g de gel de silice (0,02–0,06) que l'on dépose sur une colonne de gel de silice (0,06–0,04) (diamètre de la colonne: 1,8 cm; hauteur: 35 cm). On élue sous une pression de 80 kPa par un mélange de cyclohexane et d'acétate d'éthyle (75–25 en volumes) en recueillant des fractions de 50 $cm^3$. Les fractions 5 à 7 contenant le produit pur sont réunies et concentrées à sec sous pression réduite (30 mm de mercure; 4 kPa) à 30°C. On obtient 0,25 g de benzhydryloxycarbonyl-2 (dichloro-2,2 éthyl)-3 [méthoxyimino-2 (tritylamino-2 thiazolyl-4)-2 acétamido]-7 oxo-8 thia-5 aza-1 bicyclo [4.2.0] octène-2, isomère syn (produit 8a) sous la forme d'un solide blanchâtre.

Spectre de RMN du proton (350 MHz, $CDCl_3$, δ en ppm, J en Hz) 3,17 + 3,33 (2dd, J = 14 et 9 + J = 14 et 5, 2H, $-CH_2$-exo); 3,62 (AB limite, J = 18, 2H, $-CH_2-S-$); 4,08 (s, 3H, $=N-O-CH_3$); 5,06 (d, J = 5, 1H, -H en 6); 5,94 à 6 (mt, 2H, -H en 7 et $-CHCl_2$); 6,76 (s, 1H, -H thiazole); 6,8 (d, J = 9, 1H, -CO-NH-); 6,92 (s, 1H, $-COO-CH(C_6H_5)_2$); 7,04 (mf, 1H, $-NH-C(C_6H_5)_3$); 7,15 à 7,5 (mt, aromatiques).

A une solution refroidie à -10°C de 0,2 g de produit (8a) dans 5 $cm^3$ de dichlorométhane, on ajoute d'un coup une solution de 0,05 g d'acide métachloroperbenzoïque (à 85%) dans 1 $cm^3$ de dichlorométhane. Après 30 minutes à -10°C le mélange réactionnel est dilué par 20 $cm^3$ d'acétate d'éthyle et lavé 2 fois par 10 $cm^3$ de solution saturée de bicarbonate de sodium et 2 fois 10 $cm^3$ de solution saturée de chlorure de sodium. Après séchage sur sulfate de sodium et concentration à sec sous pression réduite (30 mm de mercure; 4 kPa) à 30°C on obtient 0,15 g de benzhydryloxycarbonyl-2 (dichloro-2,2 éthyl)-3 [méthoxyimino-2 (tritylamino-2 thiazolyl-4)-2 acétamido]-7 oxo-8 oxyde-5 thia-5 aza-1 bicyclo [4.2.0] octène-2, isomère syn (produit 8) brut sous la forme d'une meringue orangée.

Spectre de RMN du proton (350 MHz, $CDCl_3$, δ en ppm, J en Hz) 3,14 + 3,56 (2dd, J = 15 et 5 + J = 15 et 7, 2H, $-CH_2$-exo); 3,39 et 3,96 (2d, J = 18, 2H, $-CH_2-\overset{O}{S}$); 4,09 (s, 3H, $=N-O-CH_3$); 4,58 (d, J = 5, 1H, -H en 6); 5,87 (dd, J = 5 et 7, 1H, $-CHCl_2$); 6,19 (dd, J = 5 et 9, 1H, -H en 7); 6,72 (s, 1H, -H thiazole); 6,94 (s, 1H, $-COO-CH(C_6H_5)_2$); 7,08 (mf, 1H, $-NH-C(C_6H_5)_3$); 7,10 à 7,60 (mt, aromatiques + -CO-NH).

Exemple de reference 1

Le produit de l'exemple 3 peut être utilisé de la manière suivante:

On chauffe à 60°C pendant 4 heures sous agitation et sous azote un mélange de 6,79 g de benzhydryloxycarbonyl-2 {[(t.butoxycarbonyl-2 propyl-2) oxyimino]-2 (tritylamino-2 thiazolyl-4)-2 acétamido}-7 oxo-8 oxyde-5 (tosyloxy-2 vinyl)-3 thia-5 aza-1 bicyclo [4.2.0] octène-2, isomère syn, forme E (produit 1A), 60 $cm^3$ de diméthylformamide, 1,68 g de (diméthoxy-2,2 éthyl)-4 dioxo-5,6 thioxo-3 perhydrotriazine-1,2,4 et 1,25 $cm^3$ de N,N-diisopropyléthylamine. Le résidu obtenu après ce traitement est chromatographié sur une colonne de 125 g de gel de silice Merck (0,06–0,2) diamètre de la colonne: 3 cm, hauteur: 43 cm). On élue par 1,5 litre d'un mélange cyclohexane-acétate d'éthyle 50–50 (en volumes) et 1 litre d'acétate d'éthyle en recueillant des fractions de 100 $cm^3$. Les fractions 16 à 21 sont concentrées à sec à 25°C sous 20 mm de mercure (2,7 kPa), on obtient 5,5 g de benzhydryloxycarbonyl-2 {[(t.butoxycarbonyl-2 propyl-2) oxyimino]-2 (tritylamino-2 thiazolyl-4)-2 acétamido}-7 {[(diméthoxy-2,2 éthyl)-4 dioxo-5,6 tétrahydro-1,4,5,6 triazine-1,2,4 yl-3] thio-2 vinyl}-3 oxo-8 oxyde-5 thia-5 aza-1 bicyclo [4.2.0] octène-2, isomère syn, forme E (produit 1B) sous la forme d'une meringue brune.

Spectre infra-rouge ($CHB_3$), bandes caractéristiques ($cm^{-1}$) 1800, 1720, 1690, 1585, 1510, 1495, 1445, 1370, 1080, 1060, 1040, 940, 750, 700.

Spectre de RMN du proton (350 MHz, DMSO $d_6$, δ en ppm, J en Hz) 1,35 (s, 9H, $-C(CH_3)_3$); 1,44 et 1,45 (2s, 6H, $=N-O-C(CH_3)_2-$); 3,32 (s, 6H, $(-OCH_3)_2$); 3,65 et 4,36 (2d, J = 18, 2H, $-S-CH_2-$); 3,95 (d, J = 5, 2H, $>N-CH_2-$); 4,56 (t, J = 5, 1H,

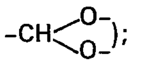

5,09 (d, J = 5, 1H, -H en 6); 5,95 (dd, J = 5 et 9, 1H, -H en 7); 6,78 (s, 1H, -H thiazole); 7 (s, 1H, $-COO-CH(C_6H_5)_2$); 7,02 et 7,1 (2d, J = 16, 2H, -CH=CH-S-); 8,23 (d, J = 9, 1H, -CO-NH-); 8,73 (s, 1H, $-NH-C(C_6H_5)_3$); 12,65 (s, 1H, =N-NH-CO- ou =N-N=C-OH).

On traite à -5°C pendant 1 heure ½, sous agitation, , 5,37 g de produit (1B) en solution dans 45 $cm^3$ de dichlorométhane et 1,79 $cm^3$ de N,N-diméthylacétamide par 0,79 $cm^3$ de trichlorure de phosphore. Le résidu obtenu après traitement est chromatographié sur une colonne de 80 g de gel de silice Merck (0,06–0,2) (diamètre de la colonne: 2 cm, hauteur: 20 cm), on élue par 250 $cm^3$ d'un mélange cyclohexane-acétate d'éthyle 40–60 (en volumes) et 1,5 litre d'un mélange 30–70 en recueillant des fractions de 100 $cm^3$. Les fractions 5 à 14 sont concentrées à sec à 25°C sous 20 mm de mercure, on obtient 4,02 g de benzhydryloxycarbonyl-2 {[(t.butoxycarbonyl-2 propyl-2) oxyimino]-2 (tritylamino-2 thiazolyl-4)-2 acétamido}-7 {[(diméthoxy-2,2 éthyl)-4 dioxo-5,6 tétrahydro-1,4,5,6 triazine-1,2,4 yl-3] thio-2 vinyl}-3 oxo-8 thia-5 aza-1 bicyclo [4.2.0] octène-2, isomère syn, forme E (produit 1C) sous la forme d'une meringue brun-clair.

Spectre infra-rouge (KBr), bandes caractéristiques ($cm^{-1}$) 1790, 1720, 1690, 1585, 1520, 1495, 1450, 1370, 1080, 940, 750, 700.

Spectre de RMN du proton (350 MHz, DMSO $d_6$, δ en ppm, J en Hz) 1,37 (s, 9H, $-C(CH_3)_3$); 1,42 (s, 6H, $=N-O-C(CH_3)_2-$); 3,31 (s, 6H, $(-OCH_3)_2$); 3,64 et 3,89 (2d, J = 18, 2H, $-S-CH_2-$); 3,95 (d, J = 5, 2H, $>N-CH_2$); 4,56 (t, J = 5, 1H,

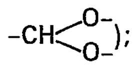

5,26 (d, J = 4, 1H, -H en 6); 5,77 (dd, J = 4 et 9, 1H, -H en 7); 6,71 (s, 1H, -H thiazole); 6,90 et 7,03 (2d, J = 16, 2H, -CH=CH-S-); 6,97 (s, 1H, $-COO-CH(C_6H_5)_2$); 8,80 (s, 1H, $-NH-C(C_6H_5)_3$); 9,39 (d, J = 9, 1H, -CO-NH-); 12,66 (s, 1H, =N-NH-CO- ou =N-N=C-OH).

On agite à 20°C pendant 20 minutes une solution de 3,89 g de produit (1C) dans 39 $cm^3$ d'acide trifluoracétique. On concentre à sec à 20°C sous 0,05 mm de mercure (0,007 kPa), reprend le résidu dans 100 $cm^3$ d'éther diéthylique, agite pendant 10 minutes et filtre. Le solide obtenu est traité à 50°C pendant 45 minutes par 80 $cm^3$ d'acide formique, on ajoute 16 $cm^3$ d'eau, maintient 30 minutes à 50°C et concentre à sec à 20°C sous 0,05 mm de mercure (0,007 kPa). On reprend le résidu par 3 fois 150 $cm^3$ d'acétone en évaporant à chaque fois à 20°C sous 30 mm de mercure (4 kPa) et chauffe le résidu à reflux sous agitation dans 100 $cm^3$ d'acétone. On filtre et recueille 2,15 g d'{(amino-2 thiazolyl-4-)-2 [(carboxy-2 propyl-2) oxyimino]-2 acétamido}-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo [4.2.0] octène-2, isomère syn, forme E (produit R1) sous la forme d'une poudre jaune.

Spectre infra-rouge (KBr), bandes caractéristiques ($cm^{-1}$) 3400, 3300, 3200, 2200, 1780, 1720, 1685, 1585, 1540, 1000.

Spectre de RMN du proton (350 MHz, $CF_3COOD$, δ en ppm, J en Hz) 1,85 et 1,86 (2s, 6H, $-CH_3$); 3,90 (s large, 2H, $-SCH_2-$); 5,20 (s, 2H, $-CH_2CHO$); 5,40 (d, J = 4, 1H, H en 6); 6,12 (d, J = 4, 1H, H en 7); 7,23 et 7,76 (2d, J = 16, 2H, -CH=CH-); 7,50 (s, 1H, H du thiazole); 9,73 (s, 1H, -CHO).

Exemple de reference 2

Le produit de l'exemple 2 peut être utilisé de la manière suivante:

Une solution de 0,54 g de benzhydryloxycarbonyl-2 t.butoxycarbonylamino-7 (chloro-2 vinyl)-3 oxo-8 oxyde-5 thia-5 aza-1 bicyclo [4.2.0] octène-2, forme E (produit 2A), 0,25 g de (diméthoxy-2,2 éthyl)-4 dioxo-5,6 thioxo-3 perhydrotriazine-1,2,4 et 0,19 $cm^3$ de N,N-diisopropyléthylamine dans 8 $cm^3$ de N,N-diméthylformamide sec est portée à 60°C pendant 4 heures puis agitée pendant 16 heures à 25°C et diluée par 100 $cm^3$ de dichlorométhane. La solution est lavée successivement par 2 fois 50 $cm^3$ de solution demi-saturée de chlorure de sodium, par 2 fois 50 $cm^3$ de solution demi-saturée de bicarbonate de sodium et 2 fois 50 $cm^3$ d'eau distillée. Après

séchage de la couche organique sur sulfate de sodium et concentration à sec sous pression réduite (30 mm de mercure; 4 kPa) à 35°C, le résidu est chromatographié sur une colonne de gel de silice (0,04–0,06) (diamètre de la colonne: 2 cm; hauteur: 30 cm) en éluant sous 50 kPa par de l'acétate d'éthyle et recueillant des fractions de 30 cm³. Les fractions 12 à 16 sont rassemblées et concentrées à sec sous pression réduite (30 mm de mercure; 4 kPa) à 35°C. On obtient 0,4 g de produit (2B) sous la forme d'un solide orangé amorphe. Ce produit est cristallisé dans 20 cm³ de mélange d'oxyde d'isopropyle et d'acétonitrile (75–25 en volumes). On obtient 0,15 g du produit cristallisé dont les caractéristiques sont les suivantes:

Spectre infra-rouge (KBr), bandes caractéristiques ($cm^{-1}$) 3410, 1790, 1715, 1580, 1490, 1450, 1155, 1115, 1075, 1040, 935, 750, 740, 695.

Spectre de RMN du proton (80 MHz, DMSO $d_6$, δ en ppm, J en Hz) 1,45 (s, 9H, $(CH_3)_3C-$); 3,62 et 4,42 (2d, J = 18,5, 2H, $-S(O)CH_2-$); 3,33 (s, 6H, $-CH(OCH_3)_2$); 3,97 (d, J = 5, 2H, $-CH_2CH(OCH_3)_2$); 4,55 (t, J = 5, 1H, $-CH(OCH_3)_2$); 5,04 (d, J = 4, 1H, H en 6); 5,80 (dd, J = 4 et 9, 1H, H en 7); 6,40 (d, J = 9, $CONH-C_7$); 6,98 (s, 1H, $-CH(C_6H_5)_2$); 7,08 (AB limite, 2H, -CH=CH-S-); 7,2 à 7,50 (m, 10H, aromatiques); 12,68 (s, 1H, -N=COH).

On ajoute 5 g de benzhydryloxycarbonyl-2 t.butoxycarbonylamino-7 {[(diméthoxy-2,2 éthyl)-4 dioxo-5,6 tétrahydro-1,4,5,6 triazine-1,2,4 yl-3] thio-2 vinyl}-3 oxo-8 oxyde-5 thia-5 aza-1 bicyclo [4.2.0] octène-2, forme E (produit 2B) à 80 cm³ d'un mélange acétonitrile-méthanol (85–15 en volumes) puis ajoute 6 g d'acide méthanesulfonique. Le mélange est devenu limpide. On laisse sous agitation pendant 5 heures à 20°C et on filtre le précipité blanc formé. Le précipité est lavé 2 fois par 10 cm³ d'acétonitrile, 2 fois par 25 cm³ d'acétate d'éthyle puis 2 fois par 25 cm³ d'éther éthylique. Après séchage sous vide (0,5 mm de mercure: 0,07 kPa) on obtient 4,4 g de méthanesulfonate du produit (2C) sous forme de cristaux blancs.

Spectre infra-rouge (KBr), bandes caractéristiques ($cm^{-1}$) 3300, 2200, 1800, 1720, 1590, 1495, 1455, 1220, 1125, 1085, 1080, 1045, 945, 760, 710, 620, 600, 560.

Spectre de RMN du proton (80 MHz, DMSO $d_6$, δ en ppm, J en Hz) 2,48 (s, 3H, $CH_3SO_3H$); 3,38 (s, 6H, $(-OCH_3)_2$); 3,74 et 4,45 (2d, J = 18, 2H, $-SCH_2-$); 3,98 (d, J = 5, 2H, $>NCH_2-$); 4,58 (t, J = 5, 1H, $CH(OCH_3)_2$); 5,06 (d, J = 4, 1H, H en 6); 5,38 (d, J = 4, 1H, H en 7); 7,0 (s, 1H, $-COOCH<$).

On agite pendant 10 minutes à 20°C un mélange de 0,14 g de méthanesulfonate d'amino-7 benzhydryloxycarbonyl-2 {[(diméthoxy-2,2 éthyl)-4 dioxo-5,6 tétrahydro-1,4,5,6 triazine-1,2,4 yl-3] thio-2 vinyl}-3 oxo-8 oxyde-5 thia-5 aza-1 bicyclo [4.2.0] octène-2, forme E (produit 2C), 30 cm³ de dichlorométhane et 10 cm³ d'une solution demi-saturée de bicarbonate de sodium, on décante, lave la phase organique par 15 cm³ d'une solution saturée de chlorure de sodium, sèche sur sulfate de sodium et filtre. La solution filtrée est additionnée de 0,156 g d'acide t.butoxycarbonylméthoxyimino-2 (tritylamino-2 thiazolyl-4)-2 acétique, isomère syn (préparé selon le brevet belge 865 298) et de 5 mg de diméthylamino-4 pyridine. On refroidit à 5°C, ajoute goutte à goutte en 3 minutes une solution de 50 mg de N,N'-dicyclohexylcarbodiimide dans 1 cm³ de dichlorométhane et laisse remonter la température à 20°C en 3 heures. On lave le mélange par 20 cm³ d'une solution à 2% de bicarbonate de sodium et 20 cm³ d'une solution demi-saturée de chlorure de sodium, sèche sur sulfate de sodium, filtre et concentre à sec à 20°C sous 20 mm de mercure (2,7 kPa). On chromatographie le résidu sur une colonne de gel de silice Merck (0,04–0,06) (diamètre de la colonne: 1,8 cm; hauteur: 9 cm); on élue par 200 cm³ d'un mélange cyclohexane-acétate d'éthyle 20–80 (en volumes) sous une pression de 40 kPa en recueillant des fractions de 10 cm³. On concentre à sec les fractions 6 à 14 à 20°C sous 20 mm de mercure (2,7 kPa) et recueille 50 mg de benzhydryloxycarbonyl-2 [t.butoxycarbonylméthoxyimino-2 (tritylamino-2 thiazolyl-4)-2 acétamido]-7 {[(diméthoxy-2,2 éthyl)-4 dioxo-5,6 tétrahydro-1,4,5,6 triazine-1,2,4 yl-3] thio-2 vinyl}-3 oxo-8 oxyde-5 thia-5 aza-1 bicyclo [4.2.0] octène-2, isomère syn, forme E (produit 2D), sous la forme d'une merinque de couleur crème dont les caractéristiques infra-rouge et RMN sont les suivantes:

Spectre infra-rouge ($CHBr_3$), bandes caractéristiques ($cm^{-1}$) 1800, 1720, 1690, 1590, 1520, 1495, 1450, 1370, 1080, 1065, 1040, 945, 755, 700.

Spectre de RMN du proton (350 MHz, DMSO $d_6$, δ en ppm, J en Hz) 1,40 (s, 9H, $-C(CH_3)_3$); 3,24 (s, 6H, $(-OCH_3)_2$); 3,60 et 4,25 (2d, J = 18, 2H, $-SCH_2-$); 3,95 (d, J = 6, 2H, $>NCH_2-$); 4,52 (s, 2H, $=NOCH_2-$); 4,54 (t, J = 6, 1H, $-CH(OCH_3)_2$); 5,08 (d, J = 4, 1H, H en 6); 5,91 (dd, J = 4 et 9, 1H, H en 7); 6,82 (s, 1H, H du thiazole); 6,97 (s, 1H, $-COOCH<$); 6,96 et 7,0 (2d, J = 16, 2H, -CH=CH-); 8,68 (d, J = 9, 1H, -CONH-); 8,74 (s, 1H, $-NH C(C_6H_5)_3$; 12,35 (s, 1H, =NNHCO- ou =NN=C-OH).

On traite à -5°C sous agitation pendant 1 heure 15 une solution de 12,7 g de produit (2D) dans 100 cm³ de dichlorométhane et 4,34 cm³ de N,N-diméthylacétamide par 1,91 cm³ de trichlorure de phosphore. On dilue le mélange par 600 cm³ d'acétate d'éthyle, lave par 2 fois 200 cm³ d'une solution à 2% de bicarbonate de sodium et 2 fois 200 cm³ d'une solution demi-saturée de chlorure de sodium, sèche sur sulfate de sodium, filtre et concentre à sec à 30°C sous 20 mm de mercure (2,7 kPa). Le produit mis en solution dans 50 cm³ de dichlorométhane est fixé sur 25 g de gel de silice Merck (0,06–0,2) et la poudre obtenue est déposée sur une colonne de 175 g de gel de silice (diamètre de la colonne: 3 cm, hauteur: 60 cm). On élue par 1,2 litre d'un mélange cyclohexane-acétate d'éthyle 60–40 (en volumes) puis par 2,8

litres d'un mélange 40-60 (en volumes) en recueillant des fractions de 100 $cm^3$. Les fractions 13 à 28 sont réunies et évaporées à sec à 20°C sous 20 mm de mercure (2,7 kPa), on obtient 9,45 g de benzhydryloxycarbonyl-2 [t.butoxycarbonylméthoxyimino-2 (tritylamino-2 thiazolyl-4)-2 acétamido]-7 {[(diméthoxy-2,2 éthyl)-4 dioxo-5,6 tétrahydro-1,4,5,6 triazine-1,2,4 yl-3] thio-2 vinyl}-3 oxo-8 thia-5 aza-1 bicyclo [4.2.0] octène-2, isomère syn, forme E, sous la forme d'une meringue jaune-orange (produit 2E).

Spectre infra-rouge ($CHBr_3$), bandes caractéristiques ($cm^{-1}$) 3280, 1800, 1725, 1690, 1590, 1530, 1495, 1440, 1370, 1160, 1080, 945, 755, 700.

Spectre de RMN du proton (350 MHz, DMSO $d_6$, δ en ppm, J en Hz) 1,45 (s, 9H, $-C(CH_3)_3$); 3,25 (s, 6H, $(-OCH_3)_2$); 3,63 et 3,81 (2d, J = 18, 2H, $-SCH_2-$); 3,95 (d, J = 6, 2H, $>NCH_2-$); 4,52 (s, 2H, $=NOCH_2-$); 4,57 (t, J = 6, 1H, $-CH(OCH_3)_2$); 5,24 (d, J = 4, 1H, H en 6); 5,78 (dd, J = 4 et 9, 1H, H en 7); 6,78 (s, 1H, H du thiazole); 6,95 (s, 1H, -COOCH<); 6,93 et 7,0 (2d, J = 16, 2H, -CH=CH-); 8,80 (s, 1H, $-NH\ C(C_6H_5)_3$); 9,50 (d, J = 9, 1H, -CONH-); 12,62 (s, 1H, =NNHCO- ou =N-N=C-OH).

On agite à 20°C pendant 25 minutes une solution de 9,4 g de produit (2E) dans 95 $cm^3$ d'acide trifluoracétique et concentre à sec à 20°C sous 0,05 mm de mercure (0,007 kPa). Le résidu est trituré dans 200 $cm^3$ d'éther diéthylique, on filtre la suspension et obtient 6,7 g de poudre jaune. Le produit ainsi obtenu est mis en solution dans 190 $cm^3$ d'acide formique pur, on chauffe à 50°C pendant 40 minutes et concentre à sec à 30°C sous 0,05 mm de mercure (0,007 kPa). On reprend le résidu par 200 $cm^3$ d'acétone, concentre à sec à 20°C sous 30 mm de mercure (4 kPa) et répète l'opération une seconde fois. Le solide obtenu est traité par 300 $cm^3$ d'acétone à reflux pendant 15 minutes, on filtre à chaud et, après séchage, on obtient 4,45 g d'[(amino-2 thiazolyl-4)-2 carboxyméthoxyimino-2 acétamido]-7 carboxy-2 [(dioxo-5,6 formylméthyl-4 tétrahydro-1,4,5,6 triazine-1,2,4 yl-3)thio-2 vinyl]-3 oxo-8 thia-5 aza-1 bicyclo [4.2.0] octène-2, isomère syn, forme E (produit R2) sous la forme d'une poudre jaune.

Spectre infra-rouge (KBr), bandes caractéristiques ($cm^{-1}$) 3600, 2200, 1775, 1815, 1680, 1635, 1585, 1195, 945, 800, 720.

Spectre de RMN du proton (350 MHz, $CF_3COOD$, δ en ppm, J en Hz) 3,88 (s large, 2H, $-SCH_2-$); 5,12 (s, 2H, $=NOCH_2-$); 5,21 (s, 2H, $-CH_2CHO$); 5,39 (d, J = 4, 1H, H en 6); 6,10 (d, J = 4, 1H, H en 7); 7,24 et 7,75 (2d, J = 16, 2H, -CH=CH-); 7,52 (s, 1H, H du thiazole); 9,77 (s, 1H, -CHO).

Exemple de reference 3

Le produit de l'exemple 4 peut être utilisé de la manière suivante:

Une solution de 0,9 g de benzhydryloxycarbonyl-2 (chloro-2 vinyl)-3 [méthoxyimino-2 (tritylamino-2 thiazolyl-4-2 acétamido]-7 oxo-8 oxyde-5 thia-5 aza-1 bicyclo [4.2.0] octène-2, isomère syn, forme E (produit 3A), 0,23 g de (diéthoxy-2,2 éthyl)-1 dioxo-5,6 méthyl-4 thioxo-3 perhydrotriazine-1,2,4 et 0,19 $cm^3$ de N,N-diisopropyléthylamine dans 10 $cm^3$ de N,N-diméthylformamide est agitée pendant 16 heures à 25°C puis 4 heures à 60°C, puis refroidie et diluée par 100 $cm^3$ de dichlorométhane. La solution obtenue est lavée successivement par 2 fois 50 $cm^3$ de solution saturée de chlorure de sodium, 2 fois par 50 $cm^3$ de solution saturée de bicarbonate de sodium et 2 fois 50 $cm^3$ d'eau distillée puis séchée sur sulfate de sodium et concentrée à sec sous pression réduite (30 mm de mercure; 4 kPa) à 30°C. Le résidu est chromatographié sur une colonne de gel de silice (0,04-0,06) (diamètre de la colonne: 3 cm; hauteur: 30 cm) en éluant sous une pression de 50 kPa par 2,5 litres d'un mélange de cyclohexane et d'acétate d'éthyle (25-75 en volumes) en recueillant des fractions de 50 $cm^3$. Les fractions 19 à 42 contenant le produit pur sont réunies et concentrées à sec. On obtient 0,8 g de benzhydryloxycarbonyl-2 {[(diéthoxy-2,2 éthyl)-1 dioxo-5,6 méthyl-4 tétrahydro-1,4,5,6 triazine-1,2,4 yl-3] thio-2 vinyl}-3 [méthoxyimino-2 (tritylamino-2 thiazolyl-4)-2 acétamido]-7 oxo-8 oxyde-5 thia-5 aza-1 bicyclo [4.2.0] octène-2, isomère syn, forme E (produit 3B) sous la forme d'une meringue orangée dont les caractéristiques sont les suivantes:

Spectre infra-rouge (CHBr), bandes caractéristiques ($cm^{-1}$) 3380, 2820, 1800, 1715, 1680, 1585, 1515, 1495, 1445, 1050, 940, 750.

Spectre de RMN du proton (350 MHz, DMSO $d_6$, δ en ppm, J en Hz) 1,07 et 1,08 (2t, J = 7, 6H, $2-CH_3$); 3,28 (s, $>N-CH_3$); 3,40 à 3,70 (2mt, 4H, $(-O-CH_2-CH_3)_2$); 3,62 et 4,36 (2d, J = 18, 2H, $-S-CH_2-$); 3,85 (s, 3H, $=N-OCH_3$); 3,95 (AB limite, 2H, $>N-CH_2$); 4,85 (t, J = 6, 1H,

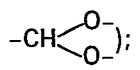

5,08 (d, J = 4, 1H, -H en 6); 5,87 (dd, J = 4 et 9, 1H, -H en 7); 6,80 (s, 1H, -H thiazole); 6,99 (s, 1H, $-COO-CH(C_6H_5)_2$); 7,05 et 7,18 (2d, J = 16, 2H, -CH=CH-S-); 8,79 (s, 1H, $-NH-C(C_6H_5)_3$); 9,12 (d, J = 9, 1H, -CO-NH-).

Rf = 0,3 chromatoplaque de gel de silice Merck [éluant: mélange de cyclohexane et d'acétate d'éthyle 20-80 en volumes].

On traite 2,3 g de produit (3B) par 0,44 $cm^3$ de trichlorure de phosphore à -5°C pendant 1 heure 15 minutes. On obtient 2 g de benzhydryloxycarbonyl-2 {[(diéthoxy-2,2, éthyl)-1 dioxo-5,6 méthyl-4 tétrahydro-1,4,5,6 triazine-1,2,4 yl-3] thio-2 vinyl}-3 [méthoxyimino-2 (tritylamino-2 thiazolyl-4)-2 acétamido]-7 oxo-8 thia-5 aza-1 bicyclo [4.2.0] octène-2, isomère syn, forme E (produit 3C) sous la forme d'une meringue jaune.

Spectre infra-rouge ($CHBr_3$), bandes caractéristiques ($cm^{-1}$) 3390, 2820, 1785, 1715, 1680, 1585, 1515, 1490, 1455, 1050, 940, 750, 740.

Spectre de RMN du proton (350 MHz, DMSO $d_6$, δ en ppm, J en Hz) 1,07 (t, J = 7, 6H, $2-CH_3$); 3,30 (s, $>N-CH_3$); 3,40 à 3,68 (2mt, 4H, $(-O-CH_2-$

$CH_3)_2)$; 3,68 et 3,92 (2d, J = 18, 2H, $-S-CH_2-$); 3,83 (s, 3H, $=N-OCH_3$); 3,95 (d, J = 6, 2H, >$N-CH_2-$); 4,85 (t, J = 6, 1H,

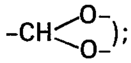);

5,27 (d, J = 4, 1H, -H en 6); 5,79 (dd, J = 4 et 9, 1H, -H en 7); 6,75 (s, 1H, -H thiazole); 6,96 (s, 1H, $-COO-CH(C_6H_5)_2$); 6,98 et 7,05 (2d, J = 16, 2H, $-CH=CH-S-$); 8,82 (s, 1H, $-NH-C_6H_5)_3$); 9,63 (d, J = 9, 1H, -CO-NH-).

Une solution de 1,9 g de produit (3C) dans 20 $cm^3$ d'acide formique est agitée pendant 30 minutes à 50°C puis diluée par 2 $cm^3$ d'eau et agitée pendant 10 minutes à 50°C. Après refroidissement à 20°C le mélange réactionnel est filtré et le filtrat concentré sous pression réduite (0,1 mm de mercure; 0,013 kPa) à 35°C. Le résidu est trituré avec 20 $cm^3$ d'éthanol que l'on évapore sous pression réduite (30 mm de mercure; 4 kPa) à 40°C. Cette opération est répétée 3 fois puis le résidu est repris par 30 $cm^3$ d'éthanol. Le solide est isolé sur filtre à vide et lavé par 3 fois 10 $cm^3$ d'éthanol et par 3 fois 10 $cm^3$ d'éther éthylique et séché. On obtient 0,96 g d'[(amino-2 thiazolyl-4)-2 méthoxyimino-2 acétamido]-7 carboxy-2 [(dioxo-5,6 formylméthyl-1 méthyl-4 tétrahydro-1,4,5,6 triazine-1,2,4 yl-3) thio-2 vinyl]-3 oxo-8 thia-5 aza-1 bicyclo [4.2.0] octène-2, isomère syn, forme E (produit R3) sous la forme d'un solide de couleur crème.

Spectre infra-rouge (KBr), bandes caractéristiques ($cm^{-1}$) 3700–2200, 1770, 1710, 1675, 1580, 1550, 1040.

Spectre de RMN du proton (350 MHz, $CF_3COOD$, δ en ppm, J en Hz) 3,66 (s, 3H, >$N-CH_3$); 3,85 (s large, 2H, $-S-CH_2-$); 4,31 (s, 3H, $=N-O-CH_3$); 5,13 (s large, 2H, >$N-CH_2-$); 5,38 (d, J = 4, 1H, -H en 6); 6,02 (d, J = 4, 1H, -H en 7); 7,22 et 7,73 (2d, J = 16, 2H, $-CH=CH-S-$); 7,48 (s, 1H, -H thiazole); 9,74 (s, 1H,

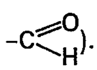).

La (diéthoxy-2,2 éthyl)-1 dioxo-5,6 méthyl-4 thioxo-3 perhydrotriazine-1,2,4 peut être préparée de la manière suivante:

8,8 g de (diéthoxy-2,2 éthyl)-1 éthoxalyl-1 méthyl-4 thiosemicarbazide sont traités par 3,08 g de t.butylate de potassium dans 60 $cm^3$ de t.butanol sec pendant 2 heures à 25°C. Le mélange réactionnel est dilué par 50 $cm^3$ d'éther. Le précipité de sel de potassium du produit attenu est isolé sur filtre, lavé par 10 $cm^3$ d'éther puis repris par 30 $cm^3$ d'eau. La solution obtenue est acidifiée à pH 3 par de l'acide chlorhydrique 4N. Le précipité est isolé sur filtre, lavé par 10 $cm^3$ d'eau et 10 $cm^3$ d'éther éthylique, puis séché sous pression réduite (2 mm de mercure; 0,27 kPa) à 20°C. On obtient 3,65 g de (diéthoxy-2,2 éthyl)-1 dioxo-5,6 méthyl-4 thioxo-3 perhydrotriazine-1,2,4 sous la forme de cristaux blancs F = 136–138°C.

La (diéthoxy-2,2 éthyl)-1 éthoxalyl-1 méthyl-4 thiosemicarbazide peut être obtenue de la manière suivante:

19,4 g de (diéthoxy-2,2 éthyl)-1 méthyl-4 thiosemicarbazide dans 200 $cm^3$ de dichlorométhane sec sont traités par 7,1 $cm^3$ de pyridine et 9,85 $cm^3$ de chlorure d'éthoxalyle pendant 1 heure à 2°C puis 16 heures à 25°C; après lavage par 100 $cm^3$ d'eau, séchage sur sulfate de magnésium et concentration à sec sous pression réduite (30 mm de mercure; 4 kPa) à 30°C, le résidu est cristallisé dans 40 $cm^3$ d'éther éthylique. On obtient 8,5 g de (diéthoxy-2,2 éthyl)-1 éthoxalyl-1 méthyl-4 thiosemicarbazide sous la forme de cristaux blancs (F = 122°C).

La (diéthoxy-2,2 éthyl)-1 méthyl-4 thiosemicarbazide peut être préparée de la manière suivante:

39,3 g de (diéthoxy-2,2 acétyl)-1 méthyl-4 thiosemicarbazide sont réduits par 18 g d'hydrure de lithium et d'aluminium dans 500 $cm^3$ de tétrahydrofuranne sec pendant 1 heure 30 à 25°C puis 1 heure au reflux. On obtient 26,4 g de (diéthoxy-2,2 éthyl)-1 méthyl-4 thiosemicarbazide sous la forme d'une huile incolore.

Spectre infra-rouge ($CCl_4$), bandes caractéristiques ($cm^{-1}$) 3360, 3200, 1550, 1240, 1130, 1060.

Spectre de masse m/e = 221, 175, 146.

La (diéthoxy-2,2 acétyl)-1 méthyl-4 thiosemicarbazide peut être préparée de la manière suivante:

31,5 g d'hydrazide de l'acide diéthoxyacétique sont traités dans 200 $cm^3$ d'éther éthylique par 14,6 g d'isocyanate de méthyle pendant 20 heures entre 20 et 30°C. La (diéthoxy-2,2 acétyl)-2 méthyl-4 thiosemicarbazide cristallise dans le milieu.

(Poids: 42, g; F = 116–118°C).

Les intermédiaires dihalogénés des produits selon l'invention peuvent être mis en œuvre de la manière suivante:

Exemple de reference 4

Une solution de 1,09 g de benzhydryloxycarbonyl-2 t.butoxycarbonylamino-7 (dichloro-2,2 éthyl)-3 oxo-8 oxyde-5 thia-5 aza-1 bicyclo [4.2.0] octène-2 (produit 4A), 0,49 g de (diméthoxy-2,2 éthyl)-4 dioxo-5,6 thioxo-3 perhydrotriazine-1,2,4 et 0,77 $cm^3$ de N,N-diisopropyléthylamine dans 20 $cm^3$ de N,N-diméthylformamide est agitée pendant 16 heures à 25°C et 4 heures à 60°C puis refroidie et diluée par 200 $cm^3$ de dichlorométhane. La solution obtenue est lavée successivement par 2 fois 100 $cm^3$ de solution saturée de chlorure de sodium, 2 fois 100 $cm^3$ de solution saturée de bicarbonate de sodium et 2 fois 100 $cm^3$ d'eau distillée puis séchée sur sulfate de sodium et concentrée à sec sous pression réduite; le résidu est chromatographié sur une colonne de gel de silice (0,04–0,06) (diamètre de la colonne: 3 cm, hauteur: 30 cm) en éluant sous une pression de 50 kPa par de l'acétate d'éthyle et recueillant des fractions de 50 $cm^3$ environ. Les fractions 7 à 15 contenant le produit pur sont rassemblées et évaporées à sec. On obtient 0,7 g de benzhydryloxycarbonyl-2 t.butoxycarbonylamino-7 {[(diméthoxy-2,2 éthyl)-4 dioxo-5,6 tétrahydro-1,4,5,6 triazine-1,2,4 yl-3] thio-2 vinyl}-3 oxo-8 oxyde-5 thia-5 aza-1 bicyclo [4.2.0] octène-2 (forme E)

dont les caractéristiques sont identiques à celles du produit (2B) décrit dans l'exemple de référence 2.

Ce produit peut être utilisé comme décrit précédemment à l'exemple de référence 2.

Exemple de reference 5

On ajoute à une solution de 0,09 g de benzhydryloxycarbonyl-2 (dichloro-2,2 éthyl)-3 [méthoxyimino-2 (tritylamino-2 thiazolyl-4)-2 acétamido]-7 oxo-8 oxyde-5 thia-5 aza-1 bicyclo [4.2.0] octène-2, isomère syn (produit 5A) et de 0,03 g de (diéthoxy-2,2 éthyl)-1 dioxo-5,6 méthyl-4 thioxo-3 perhydrotriazine-1,2,4 dans 2 cm³ de N,N diméthylformamide sec 0,04 cm³ de N,N-diisopropyléthylamine et agite le mélange réactionnel pendant 16 heures à 25°C puis 4 heures à 60°C. L'examen chromatographique met en évidence la formation du benzhydryloxycarbonyl-2 {[(diéthoxy-2,2 éthyl)-1 dioxo-5,6 méthyl-4 tetrahydro-1,4,5,6 triazine-1,2,4 yl-3] thio-2 vinyl}-3 [méthoxyimino-2 (tritylamino-2 thiazolyl-4)-2 acétamido]-7 oxo-8 oxyde-5 thia-5 aza-1 bicyclo [4.2.0] octène-2, isomère syn, forme E (produit R5).

Rf = 0,3 [chromatoplaque de gel de silice Merck; éluant: mélange de cyclohexane et d'acétate d'éthyle 20-80 (en volumes)].

Ce produit peut être alors utilisé comme décrit précédemment à l'exemple de référence 3.

**Revendications pour les Etats contractants: BE CH DE FR GB IT LI LU NL SE**

1. Une vinyl-3 céphalosporine caractérisée en ce qu'elle répond à la formule générale:

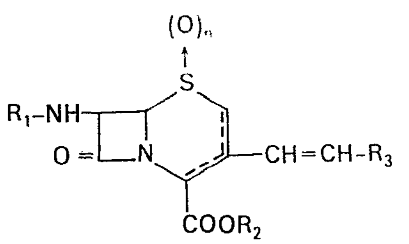

(I)

dans laquelle n est égal à 0 ou 1,
1°)a - le symbole $R_1$ représente
un atome d'hydrogène,
un radical de formule générale

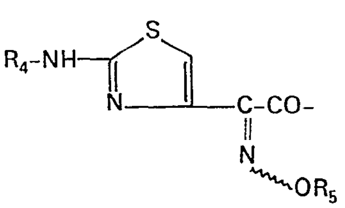

(II)

de forme syn ou anti,
(dans laquelle $R_4$ est un atome d'hydrogène ou un radical protecteur d'amine et $R_5$ est un atome d'hydrogène, un radical alcoyle, vinyle ou cyanométhyle ou un groupement protecteur d'oxime),
un radical benzhydryle, trityle,
un radical acyle de formule générale:

$R_6CO-$

dans laquelle $R_6$ est un atome d'hydrogène ou un radical alcoyle (éventuellement substitué par un ou plusieurs atomes d'halogène ou par un radical phényle ou phénoxy) ou phényle, ou bien
un radical de formule générale:

$R_7O\ CO-$

dans laquelle $R_7$ est un radical alcoyle ramifié non substitué ou un radical alcoyle droit ou ramifié portant un ou plusieurs substituants [choisis parmi les atomes d'halogène et les radicaux cyano, trialcoylsilyle, phényle, phényle substitué (par un ou plusieurs radicaux alcoyloxy, nitro ou phényle)], vinyle, allyle ou quinolyle, ou bien
un radical nitrophénylthio, ou bien
$R_1NH-$ est remplacé par un radical méthylèneamino dans lequel le radical méthylène est substitué par un groupement dialcoylamino ou aryle (lui-même éventuellement substitué par un ou plusieurs radicaux méthoxy ou nitro) et
le symbole $R_2$ représente un atome d'hydrogène, un radical facilement éliminable par voie enzymatique de formule générale:

$$\begin{array}{l}-CH-OCOR_8\\ \ \ |\\ \ \ R_9\end{array}$$

(dans laquelle $R_8$ représente un radical alcoyle ou le radical cyclohexyle, et $R_9$ représente un atome d'hydrogène ou un radical alcoyle) ou un radical méthoxyméthyle, t.butyle, benzhydryle, nitrobenzyle ou p.méthoxybenzyle, ou
1°)b - le symbole $R_1$ représente
- un atome d'hydrogène,
- un radical alcanoyle contenant 1 à 8 atomes de carbone, alcanoyle contenant 2 à 8 atomes de carbone substitué (par des atomes de chlore ou de brome),
- azidoacétyle, cyanoacétyle
- un radical acyle de formule générale:

$$\begin{array}{c}Q\\ |\\ Ar-C-CO-\\ |\\ Q\end{array}$$

{dans laquelle Q est H ou méthyle et Ar représente un radical thiényl-2, thiényl-3, furyle-2, furyle-3, pyrrolyle-2, pyrrolyle-3 ou phényle [éventuellement substitué par des atomes d'halogène ou des radicaux trifluorométhyle, hydroxy, alcoyle (contenant 1 à 3 atomes de carbone) alcoyloxy (contenant 1 à 3 atomes de carbone), cyano ou nitro, dont au moins l'un est situé en méta ou en para du phényle]}, un radical acyle de formule générale:

$Ar-X-CH_2-CO-$

dans laquelle X est l'oxygène ou le soufre et Ar

est défini comme ci-dessus, ou Ar-X- représente pyridyl-4 thio,
- un radical acyle de formule générale:

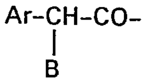

dans laquelle Ar est défini comme précédemment et B représente un radical amino, amino protégé (par un groupement benzyloxycarbonyle, alcoyloxycarbonyle, cyclopentyloxycarbonyle, cyclohexyloxycarbonyle, benzhydryloxycarbonyle, trityle ou trichloro-2,2,2 éthoxycarbonyle), un radical sulfo, un radical hydroxy ou carboxy [éventuellement protégé par estérification (respectivement avec un acide alcanoïque ou un alcool contenant 1 à 6 atomes de carbone)], azido, cyano ou carbamoyle,
ou un radical (sydnone-3)-2 alcanoyle (dont la partie alcanoyle contient 1 à 3 atomes de carbone)
ou un radical acyle de formule générale:

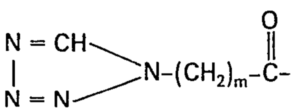

dans laquelle m est 0 à 2
ou un radical amino-5 adipoyle [dans lequel le groupement amino est éventuellement protégé par un radical alcanoyle (contenant 1 à 3 atomes de carbone et éventuellement substitué par un atome de chlore) et dans lequel le groupe carboxy est protégé par un radical benzhydryle, trichloro-2,2,2 éthyle, t.alcoyle (contenant 4 à 6 atomes de carbone) ou nitrobenzyle], ou bien $R_1NH$ est remplacé par un groupement imide cyclique d'un acide dicarboxylique,
le symbole $R_2$ représente un radical t.alcoyle contenant 4 à 6 atomes de carbone, t.alcényle contenant 6 ou 7 atomes de carbone, t.alcynyle contenant 6 ou 7 atomes de carbone, benzyle, méthoxybenzyle, nitrobenzyle, trichloro-2,2,2 éthyle, benzhydryle, succinimidométhyle ou phtalimidométhyle ou un atome d'hydrogène et
le symbole $R_3$ représente un atome d'halogène ou
2°) le symbole $R_1$ représente un radical de formule générale (II) dans laquelle $R_4$ est défini comme en 1°)a - et $R_5$ représente un radical de formule générale

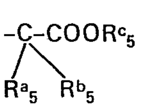

(IIa)

(dans laquelle
$R^a_5$ et $R^b_5$ qui sont identiques ou différents représentent des atomes d'hydrogène ou des radicaux alcoyle ou forment ensemble un radical alcoylène contenant 2 ou 3 atomes de carbone et $R^c_5$ représente un atome d'hydrogène ou un radical protecteur d'acide),
le symbole $R_2$ est défini comme précédemment en 1°)a - et
le symbole $R_3$ représente un radical de formule générale:

$R'_3$-$SO_2O$-

ou $R''_3$-COO-

dans lesquelles $R'_3$ représente un radical alcoyle, trifluorométhyle, trichlorométhyle ou phényle non substitué ou substitué (par un atome d'halogène ou un radical alcoyle ou nitro) et $R''_3$ est défini comme $R'_3$ ou représente un radical acylméthyle, acyl-2 éthyle, acyl-2 propyle, alcoyloxycarbonylméthyle, alcoyloxycarbonyl-2 éthyle ou alcoyloxycarbonyl-2 propyle, ou $R_3$ représente un atome d'halogène,
les portions ou radicaux alcoyles ou acyles cités ci-dessus étant (sauf mention spéciale) droits ou ramifiés et contenant 1 à 4 atomes de carbone, le produit se présentant sous forme bicyclooctène-2 ou -3 lorsque n = 0 et sous forme bicyclooctène-2 lorsque n = 1 et le substituant en -3 étant de forme E ou Z, ainsi que les mélanges de ses isomères et ses sels.

2. Un produit selon la revendication 1, caractérisé en ce que, n, $R_2$ et $R_3$ étant définis comme dans la revendication 1 en 2°), $R_1$ représente un radical de formule générale:

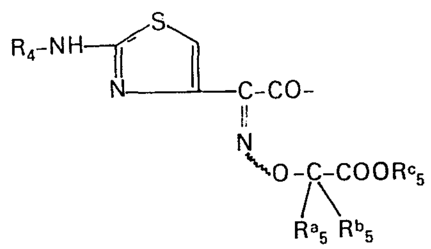

sous forme syn ou anti, dans laquelle $R^a_5$, $R^b_5$ et $R^c_5$ sont définis comme dans la revendication 1 et $R_4$ est un atome d'hydrogène ou un radical t.butoxycarbonyle, trichloro-2,2,2 éthoxycarbonyle, chloracétyle, trichloracétyle, trityle, benzyle, dibenzyle, benzyloxycarbonyle, p.nitrobenzyloxycarbonyle, p.méthoxybenzyloxycarbonyle, formyle ou trifluoracétyle.

3. Un produit selon la revendication 1 caractérisé en ce que, n, $R_2$ et $R_3$ étant définis comme dans la revendication 1 en 1°)a, $R_1$ représente un radical de formule générale:

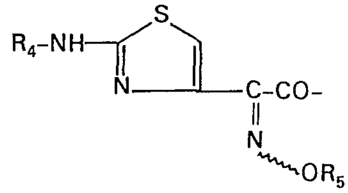

sous forme syn ou anti, dans laquelle $R_4$ est un atome d'hydrogène ou un radical t.butoxycarbonyle, trichloro-2,2,2 éthoxycarbonyle, chloracétyle, trichloracétyle, trityle, benzyle, dibenzyle, benzyoloxycarbonyle, p.nitrobenzyloxycarbonyle, p.méthoxybenzyloxycarbonyle, et $R_5$ est un atome d'hydrogène ou un radical alcoyle, vinyle,

cyanométhyle, trityle ou tétrahydropyrannyle ou méthoxy-2 propyle-2.

4. Un produit selon la revendication 1, caractérisé en ce que le radical protecteur $R^c_5$ est choisi parmi méthoxyméthyle, t.butyle, benzhydryle, nitrobenzyle, benzyle et p.méthoxybenzyle.

5. Un produit selon la revendication 1 caractérisé en ce que le radical $R_3$ représente un atome d'halogène choisi parmi le chlore, le brome et l'iode.

6. Un procédé de préparation d'un produit selon l'une des revendications 1, 2, 3, 4 ou 5 pour lequel, n étant défini selon la revendication 1, soit $R_1$, $R_2$ et $R_3$ sont définis comme dans la revendication 1 en 2°) ou dans la revendication 2, soit $R_1$ et $R_2$ sont définis comme dans la revendication 1 en 1°)a ou dans la revendication 3, à l'exception pour $R_1$ de représenter l'atome d'hydrogène, ou bien $R_1$ représente un radical alcanoyle contenant 1 à 8 atomes de carbone, alcanoyle contenant 2 à 8 atomes de carbone substitué (par des atomes de chlore ou de brome),
un radical acyle de formule générale:

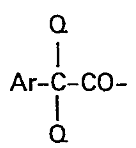

{dans laquelle Q est H ou méthyle et Ar représente un radical thiényle-2, thiényle-3, furyle-2, furyle-3, pyrrolyle-2, pyrrolyle-3 ou phényle [éventuellement substitué par des atomes d'halogène ou des radicaux hydroxy, alcoyle (contenant 1 à 3 atomes de carbone) ou alcoyloxy (contenant 1 à 3 atomes de carbone), dont au moins l'un est situé en méta ou en para du phényle]},
un radical acyle de formule générale:

$Ar-X-CH_2-CO-$

tel que défini dans la revendication 1,
un radical acyle répondant à la formule générale:

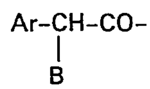

dans laquelle Ar est défini comme dans la revendication 1, et B représente un radical amino protégé (par un groupement benzyloxycarbonyle, alcoyloxycarbonyle, cyclopentyloxycarbonyle, cyclohexyloxycarbonyle, benzhydryloxycarbonyle, trityle ou trichloro-2,2,2 éthoxycarbonyle), un radical sulfo, un radical hydroxy ou carboxy (éventuellement protégé par estérification, respectivement avec un acide alcanoïque ou un alcool contenant 1 à 6 atomes de carbone), ou
un radical amino-5 adipoyle [dans lequel le groupement amino est éventuellement protégé par un radical alcanoyle (contenant 1 à 3 atomes de carbone et éventuellement substitué par un atome de chlore) et dans lequel le groupe carboxy est protégé par un groupe benzhydryle, trichloro-2,2,2 éthyle, t.alcoyle (contenant 4 à 6 atomes de carbone) ou nitrobenzyle] et $R_2$ est défini comme précédemment dans la revendication 1 en 1°)b et $R_3$ est défini comme dans la revendicatin 1 en 1°),
caractérisé en ce que l'on fait agir un agent d'halogénation ou un produit de formule générale:

$(R'_3SO_2)_2O$

$R'_3SO_2Hal$

$(R''_3CO)_2O$

ou $R''_3COHal$

dans lesquelles $R'_3$ et $R''_3$ sont définis comme dans la revendication 1 et Hal représente un atome d'halogène, sur un produit de formule générale:

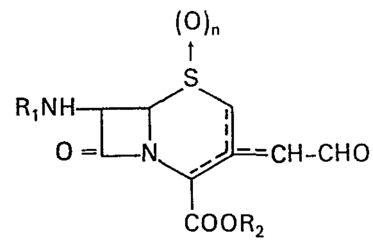

{dans laquelle, n étant défini comme dans la revendication 1, le produit se présente sous forme oxoéthyl-3 bicyclooctène-2 ou -3 oxoéthylidène-3 bicyclooctane lorsque n = 0 et sous forme oxoéthyl-3 bicyclooctène-2 ou oxoéthylidène-3 bicyclooctane lorsque n = 1, et $R_1$ est défini comme ci-dessus [à l'exception de représenter un radical de formule générale:

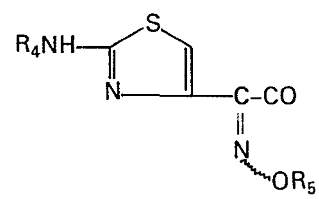

dans laquelle $R_4$ et/ou $R_5$ sont hydrogène, (et/ou $R^c_5$ dans $R_5$ est hydrogène lorsqu'on fait agir un agent d'halogénation)] et $R_2$ est défini comme ci-dessus à l'exception de représenter l'hydrogène}, ou sur un mélange de ses isomères, puis on réduit éventuellement le sulfoxyde obtenu, élimine éventuellement les groupements protecteurs, sépare éventuellement les isomères du produit obtenu et transforme éventuellement ce produit en un sel.

7. Un procédé selon la revendication 6, pour la préparation d'un produit selon l'une des revendications 1, 2, 3, 4 ou 5 pour lequel $R_3$ est un atome d'halogène choisi parmi le chlore, le brome et l'iode, caractérisé en ce que l'on fait agir un agent d'halogénation choisi parmi les composés d'addition de chlore, de brome ou d'iode et de triarylphosphite, le trichlorure ou le tribromure de phosphore, l'oxychlorure ou l'oxybromure de

phosphore, le pentachlorure ou le pentabromure de phosphore, le dichlorotriphénylphosphorane et le catéchyltrichloro (ou tribromo) phosphorane.

8. Un procédé selon la revendication 6 pour la préparation d'un produit selon l'une des revendications 1, 2, 3, 4 ou 5 pour lequel $R_3$ est un atome de chlore ou de brome caractérisé en ce que l'on isole l'intermédiaire dihalogéné de formule générale:

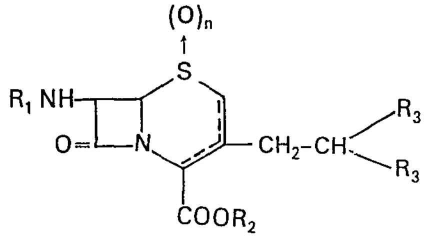

(ou un mélange de ses isomères)
qui se présente sous forme bicyclooctène-2 ou -3 lorsque n = 0 et sous forme bicyclooctène-2 lorsque n = 1, et dans laquelle $R_1$ et $R_2$ sont définis comme dans la revendication 6 pour l'aldéhyde de départ et $R_3$ est défini comme ci-dessus, puis on effectue la déhydrohalogénation de ce dérivé, réduit éventuellement le sulfoxyde obtenu, élimine éventuellement les radicaux protecteurs, sépare éventuellement ce produit en un sel.

9. Un dérivé dihalogéné de formule générale:

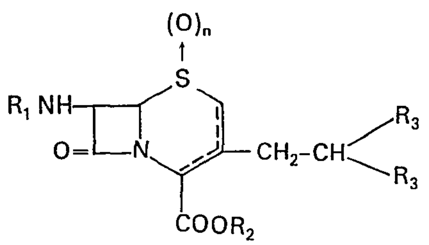

qui se présente sous forme bicyclooctène-2 ou -3 lorsque n = 0 et sous forme bicyclooctène-2 lorsque n = 1 et dans laquelle, $R_1$ étant défini comme dans la revendication 1, $R_2$ est défini comme dans la revendication 4 et $R_3$ représente un atome de chlore ou de brome, ainsi que les mélanges de ses isomères.

10. Un procédé de préparation d'un produit selon la revendication 1 pour lequel, $R_2$, $R_3$ et n étant définis comme précédemment, $R_1$ représente un atome d'hydrogène,
caractérisé en ce que l'on élimine le radical $R_1$ ou éventuellement en ce que l'on élimine simultanément les radicaux $R_1$ et $R_2$ d'un produit selon la revendication 1 pour lequel, $R_3$ et n étant définis comme dans la revendication 1,
$R_1$ et $R_2$ sont définis comme dans la revendication 1 en 1°)a, à l'exception pour $R_1$ de représenter soit l'hydrogène soit un radical de formule générale:

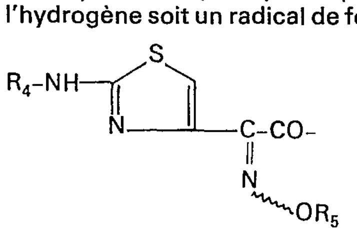

ou bien $R_1$ représente un radical amino-5 adipoyle dont les fonctions amine et acide sont protégées, ou un radical

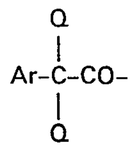

ou Ar-X-$CH_2$CO-

tel que défini dans la revendication 1 et $R_2$ est défini comme dans la revendication 1 en 1°)b, puis on sépare éventuellement le produit obtenu en ses isomères et le transforme éventuellement en un sel.

11. Un procédé de préparation d'un produit selon l'une des revendications 1, 2, 3, 4 ou 5 pour lequel, n, $R_2$ et $R_3$ étant définis selon la revendication 1, $R_1$ représente un radical de formule générale:

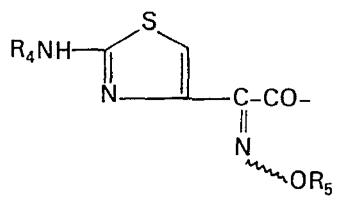

tel que défini respectivement en 2°) ou en 1°)a dans la revendication 1 ou dans la revendication 2 ou 3, ou $R_1$ est défini comme en 1°)b dans la revendication 1 à l'exception de représenter l'atome d'hydrogène, caractérisé en ce que l'on acyle au moyen d'un acide représenté par la formule générale:

$R_1$OH

(dans laquelle $R_1$ est défini comme ci-dessus étant entendu que les cas échéant les radicaux $R_4$ et/ou $R^c_5$ contenus dans $R_1$ sont autres que des atomes d'hydrogène), ou d'un dérivé réactif de cet acide, un produit selon la revendication 1 pour lequel $R_1$ représente l'hydrogène, ou éventuellement un mélange des isomères de ce produit, puis on réduit éventuellement l'oxyde obtenu, élimine éventuellement les radicaux protecteurs, sépare éventuellement les isomères du produit obtenu et le transforme éventuellement en un sel.

12. Procédé de préparation d'un produit selon l'une des revendications 1, 2, 3, 4 ou 5 pour lequel n est égal à 1, caractérisé en ce que l'on oxyde un produit selon la revendication 1, 2 ou 3 pour lequel n = 0 par toute méthode connue pour préparer un sulfoxyde à partir d'un sulfure sans toucher au reste de la molécule, puis sépare éventuellement les isomères du produit obtenu et le transforme éventuellement en un sel.

13. Procédé pour la préparation d'un produit selon l'une des revendications 1, 2, 3, 4 ou 5 pour lequel, n étant défini comme dans la revendication 1, $R_1$ est défini comme dans la revendication

1 en 2°) ou en 1°)a ou dans la revendication 2, 3 ou 4, $R_2$ représente un radical de formule générale:

$-CH\ OCO\ R_8$
|
$R_9$

tel que défini dans la revendication 1, et $R_3$ est défini comme dans la revendication 1 ou 5, caractérisé en ce que l'on
estérifie un produit selon l'une des revendications 1, 2 ou 3 [pour lequel, $R_1$, $R_3$ et n étant définis comme ci-dessus (étant entendu que lorsque $R_1$ contient un radical $R^c_5$ celui-ci est autre qu'un atome d'hydrogène), $R_2$ est un atome d'hydrogène], puis on réduit éventuellement l'oxyde obtenu, élimine éventuellement les radicaux protecteurs et sépare éventuellement le produit obtenu en ses isomères.

14. Procédé de préparation d'un produit selon l'une des revendications 1, 2, 3, 4 ou 5 pour lequel, n étant défini comme dans la revendication 1, $R_1$ représente un radical de formule générale:

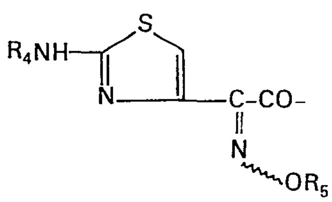

tel que défini respectivement dans la revendication 1 en 2°) ou en 1°)a ou dans les revendications 2, 3 ou 4 (à l'exception pour $R_4$ de représenter un radical chloracétyle ou trichloracétyle et/ou pour $R_5$ de représenter vinyle), $R_2$ a la définition correspondante donnée dans la revendication 1, et $R_3$ est défini comme dans la revendication 1 ou 5, caractérisé en ce que l'on fait agir une thiourée de formule générale:

$R_4NH-CS-NH_2$

dans laquelle $R_4$ est défini comme ci-dessus, sur un dérivé de céphalosporine de formule générale

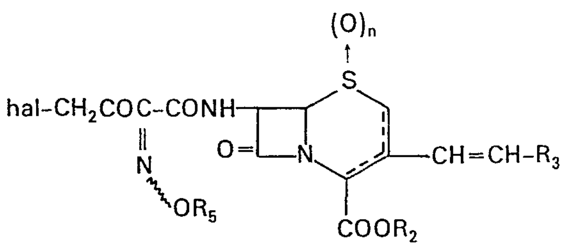

dans laquelle $R_3$ et n sont définis comme dans la revendication 1, $R_2$ et $R_5$ sont définis comme ci-dessus et hal représente un atome de chlore ou de brome, ou éventuellement sur un mélange des isomères de ce produit, puis on réduit éventuellement l'oxyde obtenu, élimine éventuellement les radicaux protecteurs, sépare éventuellement les isomères du produit obtenu et le transforme éventuellement en sel.

**Revendications pour l'Etat contractant: AT**

1. Procédé de préparation d'une vinyl-3 céphalosporine de formule générale:

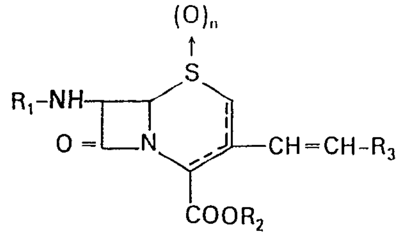
(I)

dans laquelle n est égal à 0 ou 1,
1°)a – le symbole $R_1$ représente
un atome d'hydrogène,
un radical de formule générale:

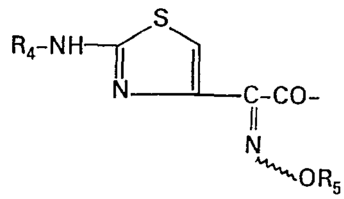
(II)

de forme syn ou anti,
(dans laquelle $R_4$ est un atome d'hydrogène ou un radical protecteur d'amine et $R_5$ est un atome d'hydrogène, un radical alcoyle, vinyle ou cyanométhyle ou un groupement protecteur d'oxime),
un radical benzhydryle, trityle,
un radical acyle de formule générale:

$R_6CO-$

dans laquelle $R_6$ est un atome d'hydrogène ou un radical alcoyle (éventuellement substitué par un ou plusieurs atomes d'halogène ou par un radical phényle ou phénoxy) ou phényle, ou bien
un radical de formule générale:

$R_7O\ CO-$

dans laquelle $R_7$ est un radical alcoyle ramifié non substitué ou un radical alcoyle droit ou ramifié portant un ou plusieurs substituants [choisis parmi les atomes d'halogène et les radicaux cyano, trialcoylsilyle, phényle, phényle substitué (par un ou plusieurs radicaux alcoyloxy, nitro ou phényle)], vinyle, allyle ou quinolyle, ou bien
un radical nitrophénylthio, ou bien
$R_1NH-$ est remplacé par un radical méthylèneamino dans lequel le radical méthylène est substitué par un groupement dialcoylamino ou aryle (lui-même éventuellement substitué par un ou plusieurs radicaux méthoxy ou nitro) et
le symbole $R_2$ représente un atome d'hydrogène, un radical facilement éliminable par voie enzymatique de formule générale:

$-CH-OCOR_8$
|
$R_9$

(dans laquelle $R_8$ représente un radical alcoyle ou le radical cyclohexyle, et $R_9$ représente un atome d'hydrogène ou un radical alcoyle) ou un radical méthoxyméthyle, t.butyle, benzhydryle, nitrobenzyle ou p.méthoxybenzyle, ou

1°)b - le symbole $R_1$ représente
- un atome d'hydrogène,
- un radical alcanoyle contenant 1 à 8 atomes de carbone, alcanoyle contenant 2 à 8 atomes de carbone substitué (par des atomes de chlore ou de brome),
- azidoacétyle, cyanoacétyle
- un radical acyle de formule générale:

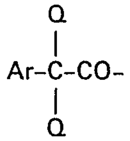

{dans laquelle Q est H ou méthyle et Ar représente un radical thiényl-2, thiényl-3, furyle-2, furyle-3, pyrrolyle-2, pyrrolyle-3 ou phényle [éventuellement substitué par des atomes d'halogène ou des radicaux trifluorométhyle, hydroxy, alcoyle (contenant 1 à 3 atomes de carbone) alcoyloxy (contenant 1 à 3 atomes de carbone), cyano ou nitro, dont au moins l'un est situé en méta ou en para du phényle]}, un radical acyle de formule générale:

$Ar-X-CH_2-CO-$

dans laquelle X est l'oxygène ou le soufre et Ar est défini comme ci-dessus, ou Ar-X- représente pyridyl-4 thio,
- un radical acyle de formule générale:

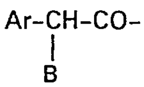

dans laquelle Ar est défini comme précédemment et B représente un radical amino, amino protégé (par un groupement benzyloxycarbonyle, alcoyloxycarbonyle, cyclopentyloxycarbonyle, cyclohexyloxycarbonyle, benzhydryloxycarbonyle, trityle ou trichloro-2,2,2 éthoxycarbonyle), un radical sulfo, un radical hydroxy ou carboxy [éventuellement protégés par estérification (respectivement avec un acide alcanoïque ou un alcool contenant 1 à 6 atomes de carbone)], azido, cyano ou carbamoyle,
ou un radical (sydnone-3)-2 alcanoyle (dont la partie alcanoyle contient 1 à 3 atomes de carbone)
ou un radical acyle de formule générale:

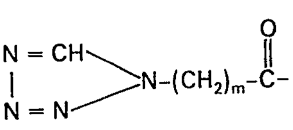

dans laquelle m est 0 à 2
ou un radical amino-5 adipoyle [dans lequel le groupement amino est éventuellement protégé par un radical alcanoyle (contenant 1 à 3 atomes de carbone et éventuellement substitué par un atome de chlore) et dans lequel le groupe carboxy est protégé par un radical benzhydryle, trichloro-2,2,2 éthyle, t.alcoyle (contenant 4 à 6 atomes de carbone) ou nitrobenzyle], ou bien $R_1NH$ est remplacé par un groupement imide cyclique d'un acide dicarboxylique,
le symbole $R_2$ représente un radical t.alcoyle contenant 4 à 6 atomes de carbone, t.alcényle contenant 6 ou 7 atomes de carbone, t.alcynyle contenant 6 ou 7 atomes de carbone, benzyle, méthoxybenzyle, nitrobenzyle, trichloro-2,2,2 éthyle, benzhydryle, succinimidométhyle ou phtalimidométhyle ou un atome d'hydrogène et le symbole $R_3$ représente un atome d'halogène ou

2°) le symbole $R_1$ représente un radical de formule générale (II) dans laquelle $R_4$ est défini comme en 1°)a et $R_5$ représente un radical de formule générale

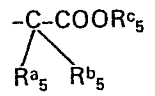 (IIa)

(dans laquelle
$R^a_5$ et $R^b_5$ qui sont identiques ou différents représentent des atomes d'hydrogène ou des radicaux alcoyle ou forment ensemble un radical alcoylène contenant 2 ou 3 atomes de carbone et $R^c_5$ représente un atome d'hydrogène ou un radical protecteur d'acide),
le symbole $R_3$ représente un atome d'halogène ou un radical de formule générale:

$R'_3-SO_2O-$

ou $R''_3-COO-$

dans lesquelles $R'_3$ représente un radical alcoyle, trifluorométhyle, trichlorométhyle ou phényle non substitué ou substitué (par un atome d'halogène ou un radical alcoyle ou nitro) et $R''_3$ est défini comme $R'_3$ ou représente un radical acylméthyle, acyl-2 éthyle, acyl-2 propyle, alcoyloxycarbonylméthyle, alcoyloxycarbonyl-2 éthyle ou alcoyloxycarbonyl-2 propyle,
les portions ou radicaux alcoyles ou acyles cités ci-dessus étant (sauf mention spéciale) droits ou ramifiés et contenant 1 à 4 atomes de carbone, le produit se présentant sous forme bicyclooctène-2 ou -3 lorsque n = 0 et sous forme bicyclooctène-2 lorsque n = 1 et le substituant en -3 étant de forme E ou Z, ainsi que les mélanges de ses isomères et ses sels, caractérisé en ce que A - pour la préparation d'un produit dans la forme duquel soit $R_1$ et $R_2$ sont définis comme en 1°)a, à l'exception pour $R_1$ de représenter l'atome d'hydrogène, ou bien $R_1$ représente un radical alcanoyle contenant 1 à 8 atomes de carbone, alcanoyle contenant 2 à 8 atomes de carbone substitué (par des atomes de chlore ou de brome),
un radical acyle de formule générale:

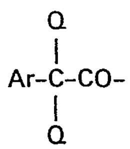

{dans laquelle Q est H ou méthyle et Ar représente un radical thiényle-2, thiényle-3, furyle-2, furyle-3, pyrrolyle-2, pyrrolyle-3 ou phényle [éventuellement substitué par des atomes d'halogène ou des radicaux hydroxy, alcoyle (contenant 1 à 3 atomes de carbone) ou alcoyloxy (contenant 1 à 3 atomes de carbone), dont au moins l'un est situé en méta ou en para du phényl]},
un radical acyle de formule générale:

$Ar-X-CH_2-CO-$

tel que défini ci-dessus,
un radical acyle répondant à la formule générale:

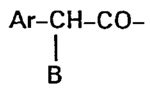

dans laquelle Ar est défini comme ci-dessus, et B représente un radical amino protégé (par un groupement benzyloxycarbonyle, alcoyloxycarbonyle, cyclopentyloxycarbonyle, cyclohexyloxycarbonyle, benzhydryloxycarbonyle, trityle ou trichloro-2,2,2 éthoxycarbonyle), un radical sulfo, un radical hydroxy ou carboxy (éventuellement protégé par estérification, respectivement avec un acide alcanoïque ou un alcool contenant 1 à 6 atomes de carbone), ou
un radical amino-5 adipoyle [dans lequel le groupement amino est éventuellement protégé par un radical alcanoyle (contenant 1 à 3 atomes de carbone et éventuellement substitué par un atome de chlore) et dans lequel le groupe carboxy est protégé par un groupe benzhydryle, trichloro-2,2,2 éthyle, t.alcoyle (contenant 4 à 6 atomes de carbone) ou nitrobenzyle] et
$R_2$ est défini comme précédemment en 1°)b et
$R_3$ est défini comme en 1°),
on fait agir un agent d'halogénation ou un produit de formule générale:

$(R'_3SO_2)_2O$ $R'_3SO_2Hal$

$(R''_3CO)_2O$ ou $R''_3COHal$

dans lesquelles $R'_3$ et $R''_3$ sont définis comme précédemment et Hal représente un atome d'halogène, sur un produit de formule générale:

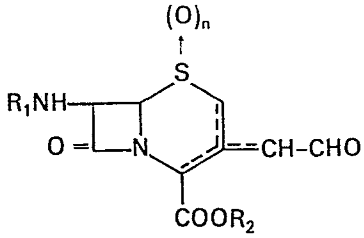

{dans laquelle le produit se présente sous forme oxoéthyl-3 bicyclooctène-2 ou -3 ou oxoéthylidène-3 bicyclooctane lorsque n = 0 et sous forme oxoéthyl-3 bicyclooctène-2 ou oxoéthylidène-3 bicyclooctane lorsque n = 1, et $R_1$ est défini comme ci-dessus [à l'exception de représenter un radical de formule générale:

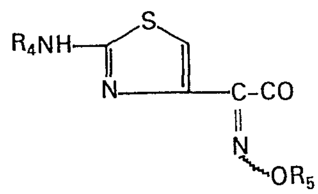

dans laquelle $R_4$ et/ou $R_5$ sont hydrogène, (et/ou $R^c_5$ dans $R_5$ est hydrogène lorsqu'on fait agir un agent d'halogénation)] et $R_2$ est défini comme ci-dessus à l'extension de représenter l'hydrogène}, ou sur un mélange de ses isomères, puis on réduit éventuellement le sulfoxyde obtenu ou, inversement, on oxyde le produit obtenu en son S-oxyde, on élimine éventuellement les groupements protecteurs, sépare éventuellement les isomères du produit obtenu et transforme éventuellement ce produit en un sel, puis
B) pour la préparation dans la formule duquel $R_2$, $R_3$ et n étant définis comme précédemment, $R_1$ représente un atome d'hydrogène, on élimine le radical $R_1$ ou éventuellement on élimine simultanément les radicaux $R_1$ et $R_2$ d'un produit préparé selon A et dans la formule duquel $R_1$ et $R_2$ sont définis comme en 1°)a, à l'exception pour $R_1$ de représenter soit l'hydrogène soit un radical de formule générale:

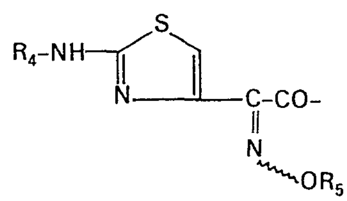

ou bien $R_1$ représente un radical amino-5 adipoyle dont les fonctions amine et acide sont protégées, ou un radical

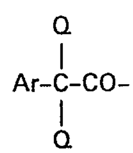

ou $Ar-X-CH_2CO-$

tel que défini précédemment et $R_2$ est défini comme en 1°)b, puis on sépare éventuellement le produit obtenu en ses isomères et le transforme éventuellement en un sel, puis
C) pour la préparation d'un produit dans la formule duquel $R_1$ représente un radical de formule générale:

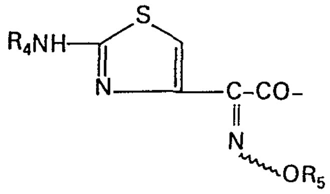

tel que défini respectivement en 2°) ou en 1°)a ou $R_1$ est défini comme en 1°)b à l'exception de représenter l'atome d'hydrogène, on acyle au moyen d'un acide représenté par la formule générale:

$R_1OH$

(dans laquelle $R_1$ est défini comme ci-dessus étant entendu que les cas échéant les radicaux $R_4$ et/ou $R^c_5$ contenus dans $R_1$ sont autres que des atomes d'hydrogène), ou d'un dérivé réactif de cet acide, un produit préparé selon B pour lequel $R_1$ représente l'hydrogène, ou éventuellement un mélange des isomères de ce produit, puis on réduit éventuellement l'oxyde obtenu ou, inversement, on oxyde le produit obtenu en son S-oxyde, on élimine éventuellement les radicaux protecteurs, on sépare éventuellement les isomères du produit obtenu et on le transforme éventuellement en un sel, puis

D) pour la préparation d'un produit dans la formule duquel $R_1$ est défini comme en 2°) ou en 1°)a, $R_2$ représente un radical de formule générale:

$$-\underset{\substack{|\\ R_9}}{CH}-OCO\ R_8$$

tel que défini précédemment et $R_3$ est défini comme précédemment, on estérifie le produit obtenu [dans la formule duquel $R_1$, $R_3$ et n étant définis comme ci-dessus (étant entendu que lorsque $R_1$ contient un radical $R^c_5$ celui-ci est autre qu'un atome d'hydrogène), $R_2$ est un atome d'hydrogène], puis on réduit éventuellement l'oxyde obtenu ou, inversement, on oxyde le produit obtenu en son S-oxyde, on élimine éventuellement les radicaux protecteurs et on sépare éventuellement le produit obtenu en ses isomères, ou bien,

E) pour la préparation d'un produit dans la formule duquel $R_1$ représente un radical de formule générale:

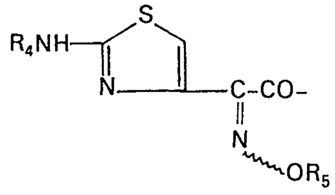

tel que défini en 2°) ou en 1°)a (à l'exception pour $R_4$ de représenter un radical chloracétyle ou trichloracétyle et/ou pour $R_5$ de représenter vinyle), et n, $R_2$ et $R_3$ ont une définition correspondante, on fait agir une thiourée de formule générale:

$R_4NH-CS-NH_2$

dans laquelle $R_4$ est défini comme ci-dessus, sur un dérivé de céphalosporine de formule générale:

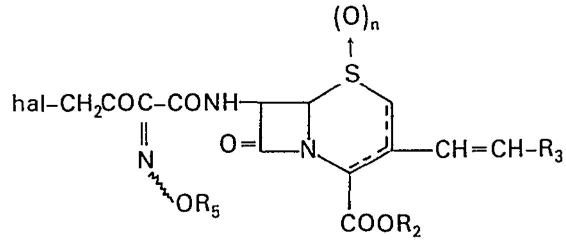

dans laquelle $R_2$, $R_3$, $R_5$ et n sont définis comme ci-dessus et hal représente un atome de chlore ou de brome, ou éventuellement sur un mélange des isomères de ce produit, puis réduit éventuellement l'oxyde obtenu, élimine éventuellement les radicaux protecteurs, sépare éventuellement les isomères du produit obtenu et le transforme éventuellement en sel.

2. Procédé selon la revendication 1 pour la préparation d'un produit dans la formule duquel $R_3$ est un atome de chlore ou de brome, caractérisé en ce que dans l'étape A on isole l'intermédiaire dihalogéné de formule générale:

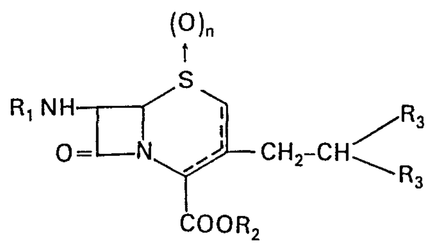

(ou un mélange de ses isomères)
qui se présente sous forme bicyclooctène-2 ou -3 lorsque n = 0 et sous forme bicyclooctène-2 lorsque n = 1, et dans laquelle $R_1$ et $R_2$ sont définis comme dans la revendication 1 pour l'aldéhyde de départ et $R_3$ est défini comme ci-dessus, puis on effectue la déhydrohalogénation de ce dérivé.

**Patentansprüche für die Vertragsstaaten**
**BE CH DE FR GB IT LI LU NL SE**

1. Ein 3-Vinylcephalosporin, dadurch gekennzeichnet, dass es der allgemeinen Formel I

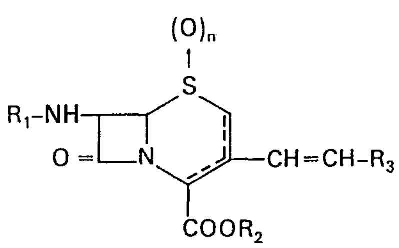

(I)

entspricht, worin n gleich 0 oder 1 ist,

1a) das Symbol $R_1$ ein Wasserstoffatom, einen Rest der allgemeinen Formel II

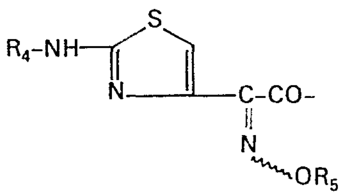

(II)

in der syn- oder anti-Form darstellt, (worin $R_4$ ein Wasserstoffatom oder ein Aminschutzrest ist und $R_5$ ein Wasserstoffatom, ein Alkyl-, Vinyl- oder Cyanomethylrest oder eine Oximschutzgruppe ist), einen Benzhydryl-, Tritylrest, einen Acylrest der allgemeinen Formel $R_6CO-$, worin $R_6$ ein Wasserstoffatom oder ein Alkylrest (gegebenenfalls substituiert durch ein oder mehrere Halogenatome oder durch einen Phenyl- oder Phenoxyrest) oder Phenylrest ist, oder aber einen Rest der allgemeinen Formel $R_7O$ CO–, worin $R_7$ ein nicht substituierter verzweigter Alkylrest oder ein gerader oder verzweigter Alkylrest mit einem oder mehreren Substituenten ist [ausgewählt unter den Halogen atomen und den Resten Cyano, Trialkylsilyl, Phenyl, substituiertes Phenyl (durch ein oder mehrere Alkyloxy-, Nitro- oder Phenylreste)], Vinyl, Allyl oder Chinolyl, oder auch einen Nitrophenylthiorest bedeutet, oder auch $R_1NH-$ ist ersetzt durch einen Methylenaminorest, worin der Methylenrest durch eine Dialkylamino- oder Arylgruppe substituiert ist (die ihrerseits gegebenenfalls durch einen oder mehrere Methoxy- oder Nitroreste substituiert ist) und das Symbol $R_2$ ein Wasserstoffatom, einen auf enzymatischem Wege leicht entfernbaren Rest der allgemeinen Formel

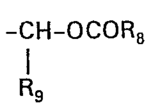

(worin $R_8$ eine Alkylrest oder den Cyclohexylrest bedeutet und $R_9$ ein Wasserstoffatom oder einen Alkylrest darstellt) oder einen Methoxymethyl-, tert.-Butyl-, Benzhydryl-, Nitrobenzyl- oder p-Methoxybenzylrest bedeutet, oder

1b) das Symbol $R_1$ bedeutet ein Wasserstoffatom,
einen Alkanoylrest mit 1 bis 8 Kohlenstoffatomen, einen substituierten Alkanoylrest mit 2 bis 8 Kohlenstoffatomen (durch Chlor- oder Bromatome substituiert),
Azidoacetyl, Cyanoacetyl,
einen Acylrest oder allgemeinen Formel

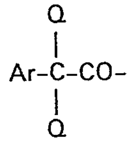

{worin Q Wasserstoff oder Methyl ist und Ar eine Thienyl-2, Thienyl-3-, Furyl-2-, Furyl-3-, Pyrrolyl-2-, Pyrrolyl-3- oder Phenylrest [gegebenenfalls substituiert durch Halogenatome oder Trifluormethylreste, Hydroxy, Alkyl (mit 1 bis 3 Kohlenstoffatome), Cyano oder Nitro substituiert ist, wobei wenigstens eines in meta- oder para-Stellung des Phenyls ist] bedeutet},
einen Acylrest der allgemeinen Formel:

$Ar-X-CH_2-CO-$

worin X Sauerstoff oder Schwefel ist und Ar wie vorstehend definiert ist, oder Ar–X– bedeutet Pyridyl-4-thio, einen Acylrest der allgemeinen Formel

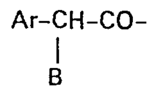

worin Ar wie vorstehend definiert ist und B einen Aminorest, geschützten Aminorest (durch eine Gruppe Benzyloxycarbonyl, Alkyloxycarbonyl, Cyclopentyloxycarbonyl, Cyclohexyloxycarbonyl, Benzhydryloxycarbonyl, Trityl oder 2,2,2-Trichlor ethoxycarbonyl), einen Sulforest, einen Hydroxy- oder Carboxyrest [gegebenenfalls geschützt durch Veresterung (mit einer Alkansäure oder einem Alkohol mit 1 bis 6 Kohlenstoffatomen)], Azido-, Cyano-, oder Carbamoylrest bedeutet, oder einen 2-(Sydnon-3)-alkanoylrest (dessen Alkanoylteil 1 bis 3 Kohlenstoffatomen enthält), oder einen Acylrest der allgemeinen Formel

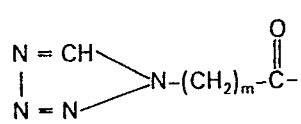

bedeutet, worin m 0 bis 2 ist,
oder einen 5-Aminoadipoylrest [worin die Aminogruppe gegebenenfalls geschützt ist durch einen Alkanoylrest (enthaltend 1 bis 3 Kohlenstoffatome und gegebenenfalls substituiert durch ein Chloratom) und worin die Carboxygruppe durch einen Benzhydryl-, 2,2,2-Trichlorethyl-, tert.-Alkyl- (enthaltend 4 bis 6 Kohlenstoffatome) oder Nitrobenzylrest geschützt ist] oder aber $R_1NH$ ist durch eine cyclische Imidgruppe einer Dicarbonsäure ersetzt,
das Symbol $R_2$ bedeutet einen tert.-Alkylrest mit 4 bis 6 Kohlenstoffatomen, tert.-Alkenylrest mit 6 oder 7 Kohlenstoffatomen, tert.-Alkinylrest mit 6 bis 7 Kohlenstoffatomen, Benzyl, Methoxybenzyl, Nitrobenzyl, 2,2,2-Trichlorethyl, Benzhydryl, Succinimidomethyl oder Phthalimidomethyl oder ein Wasserstoffatom und
das Symbol $R_3$ bedeutet ein Halogenatom oder

2) das Symbol $R_1$ bedeutet einen Rest der allgemeinen Formel II worin $R_4$ wie unter 1a) definiert ist und $R_5$ einen Rest der allgemeinen Formel IIa

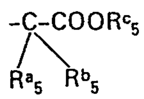
(IIa)

bedeutet (worin
$R^a_5$ und $R^b_5$, welche identisch oder verschieden sind, Wasserstoffatome oder Alkylreste bedeuten oder zusammen einen Alkylenrest mit 2 oder 3 Kohlenstoffatomen bilden und $R^c_5$ ein Wasserstoffatom oder einen Säureschutzrest bedeutet), das Symbol $R_2$ wie vorstehend unter 1a) definiert ist und das Symbol $R_3$ einen Rest der allgemeinen Formel

$R'_3-SO_2O-$ oder $R''_3-COO-$

bedeutet, worin $R'_3$ einen Alkyl-, Trifluormethyl-, Trichlormethyl- oder Phenylrest, nicht substituiert oder substituiert (durch ein Halogenatom oder einen Alkyl- oder Nitrorest) bedeutet und $R''_3$ wie $R'_3$ definiert ist oder einen Acylmethyl-, 2-Acylethyl-, 2-Acylpropyl-, Alkyloxycarbonylmethyl-, 2-Alkyloxycarbonylethyl- oder 2-Alkyloxycarbonylpropylrest bedeutet, oder $R_3$ ein Halogenatom bedeutet,
wobei die oben erwähnten Alkyl- oder Acylteile oder -reste (wenn nichts anderes angegeben ist) gerade oder verzweigt sind und 1 bis 4 Kohlenstoffatome enthalten, wobei das Produkt in der Bicycloocten-2- oder -3-Form vorliegt, wenn n = 0 und in der Bicycloocten-2-Form, wenn n = 1 ist, und der Substituent in 3-Stellung ist in der E- oder Z-Form, sowie die Gemische seiner Isomeren und seine Salze.

2. Ein Produkt gemäss Anspruch 1, dadurch gekennzeichnet, dass n, $R_2$ und $R_3$ wie in Anspruch 1 unter 2) definiert sind, $R_1$ einen Rest der allgemeinen Formel

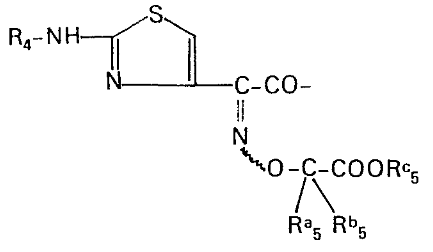

in der syn- oder anti-Form bedeutet, worin $R^a_5$ und $R^b_5$ und $R^c_5$ wie in Anspruch 1 definiert sind und $R_4$ ein Wasserstoffatom oder ein tert.-Butoxycarbonyl-, 2,2,2-Trichlorethoxycarbonyl-, Chloracetyl-, Trichloracetyl-, Trityl-, Benzyl-, Dibenzyl-, Benzyloxycarbonyl-, p-Nitrobenzyloxycarbonyl-, p-Methoxybenzyloxycarbonyl-, Formyl- oder Trifluoracetylrest ist.

3. Ein Produkt gemäss Anspruch 1, dadurch gekennzeichnet, das n, $R_2$ und $R_3$ wie in Anspruch 1 unter 1a) definiert sind, $R_1$ einen Rest der allgemeinen Formel

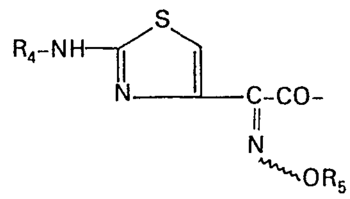

in der syn- oder anti-Form bedeutet, worin $R_4$ ein Wasserstoffatom oder ein Rest tert.-Butoxycarbonyl, 2,2,2-Trichlorethoxycarbonyl, Chloracetyl, Trichloracetyl, Trityl, Benzyl, Dibenzyl, Benzyloxycarbonyl, p-Nitrobenzyloxycarbonyl, p-Methoxybenzyloxycarbonyl ist und $R_5$ ein Wasserstoffatom oder ein Alkyl-, Vinyl-, Cyanomethyl-, Trityl- oder Tetrahydropyrannylrest oder 2-Methoxypropyl-2-Rest ist.

4. Ein Produkt gemäss Anspruch 1, dadurch gekennzeichnet, dass der Schutzrest $R^c_5$ ausgewählt ist unter Methoxymethyl, tert.-Butyl, Benzhydryl, Nitrobenzyl, Benzyl und p-Methoxybenzyl.

5. Ein Produkt gemäss Anspruch 1, dadurch gekennzeichnet, dass der Rest $R_3$ ein Halogenatom ausgewählt unter Chlor, Brom und Jod ist.

6. Verfahren zur Herstellung eines Produktes gemäss einem der Ansprüche 1, 2, 3, 4 oder 5, wobei n wie in Anspruch 1 definiert ist, entweder $R_1$, $R_2$ und $R_3$ wie in Anspruch 1 unter 2) oder in Anspruch 2 definiert sind, oder $R_1$ und $R_2$ sind wie in Anspruch 1 unter 1a) oder in Anspruch 3 definiert, mit der Ausnahme der Bedeutung für $R_1$ eines Wasserstoffatoms, oder aber $R_1$ bedeutet einen Alkanoylrest mit 1 bis 8 Kohlenstoffatomen, substituierten Alkanoylrest mit 2 bis 8 Kohlenstoffatomen (durch Chlor- oder Bromatome), einen Acylrest der allgemeinen Formel

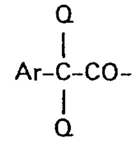

{worin Q Wasserstoff oder Methyl ist und Ar einen Thienyl-2-, Thienyl-3-, Furyl-2-, Furyl-3-, Pyrrolyl-2-, Pyrrolyl-3- oder Phenylrest bedeutet [gegebenenfalls substituiert durch Halogenatome oder Hydroxyreste, Alkyl (enthaltend 1 bis 3 Kohlenstoffatome) oder Alkyloxy (enthaltend 1 bis 3 Kohlenstoffatome), wovon wenigstens einer in meta- oder in para-Stellung des Phenylrests steht]}, einen Acylrest der allgemeinen Formel

$Ar-X-CH_2-CO-$

wie in Anspruch 1 definiert,
einen Acylrest der allgemeinen Formel

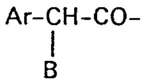

worin Ar wie in Anspruch 1 definiert ist und B einen geschützten Aminorest (durch eine Gruppe Benzyloxycarbonyl, Alkyloxycarbonyl, Cyclopentyloxycarbonyl,
Cyclohexyloxycarbonyl, Benzhydryloxycarbonyl, Trityl oder 2,2,2-Trichlorethoxycarbonyl), einen Sulforest, einen Hydroxy- oder Carboxyrest (gegebenenfalls geschützt durch Veresterung mit einer Alkansäure oder einem Alkohol mit 1 bis 6 Kohlenstoffen) bedeutet oder
einen 5-Aminoadipoylrest bedeutet [worin die Aminogruppe gegebenenfalls geschützt ist durch einen Alkanoylrest (enthaltend 1 bis 3 Kohlenstoffatome und gegebenenfalls substituiert durch ein Chloratom) und worin die Carboxygruppe durch eine Benzydryl-, 2,2,2-Trichlorethyl-, tert.-Alkyl- (enthaltend 4 bis 6 Kohlenstoffatome) oder Nitrobenzylgruppe geschützt ist] und
$R_2$ ist wie vorstehend in Anspruch 1 unter 1b) definiert und $R_3$ ist wie in Anspruch 1 unter 1) definiert, dadurch gekennzeichnet, dass man ein Halogenierungsmittel oder ein Produkt der allgemeinen Formel

$(R'_3SO_2)_2O$

$R'_3SO_2Hal$

$(R''_3CO)_2O$

oder $R''_3COHal$

worin $R'_3$ und $R''_3$ wie in Anspruch 1 definiert sind und Hal ein Halogenatom bedeutet, auf ein Produkt der allgemeinen Formel

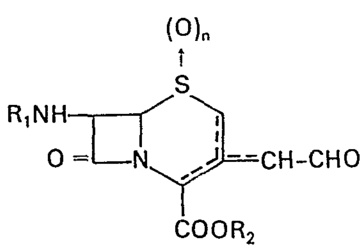

{worin n wie in Anspruch 1 definiert ist, das Produkt in 3-Oxoethylbicycloocten-2- oder -3-Form oder 3-Oxoethylidenbicyclooctanform vorliegt, wenn n = 0 und in 3-Oxoethylbicycloocten-2- oder 3-Oxoethylidenbicyclooctanform vorliegt, wenn n = 1 und $R_1$ ist wie vorstehend definiert [mit Ausnahme der Bedeutung eines Restes der allgemeinen Formel

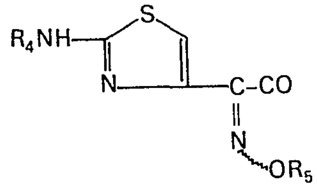

worin $R_4$ und/oder $R_5$ Wasserstoff sind (und/oder $R^c_3$ in $R_5$ ist Wasserstoff, wenn man Halogenierungsmittel einwirken lässt)] und $R_2$ wie vorstehend definiert ist, mit Ausnahme der Bedeutung Wasserstoff} oder auf Gemisch seiner Isomeren einwirken lässt, dass man dann gegebenenfalls das erhaltene Sulfoxid reduziert, gegebenenfalls die Schutzgruppen entfernt, gegebenenfalls die Isomeren des erhaltenen Produkts auftrennt und dieses Produkt gegebenenfalls in ein Salz überführt.

7. Verfahren gemäss Anspruch 6 zur Herstellung eines Produkts gemäss einem der Ansprüche 1, 2, 3, 4 oder 5, worin $R_3$ ein Halogenatom, ausgewählt unter Chlor, Brom und Jod, ist, dadurch gekennzeichnet, dass man ein Halogenierungsmittel, ausgewählt unter den Additionsverbindungen von Chlor, Brom oder Jod und Triarylphosphit, Phosphortrichlorid oder -tribromid, Phosphoroxychlorid oder -oxybromid, Phosphorpentachlorid oder -pentabromid, Dichlortriphenylphosphoran und Catechyltrichlor- (oder -tribrom)-phosphoran.

8. Verfahren gemäss Anspruch 6 zur Herstellung eines Produkts gemäss einem der Ansprüche 1, 2, 3, 4 oder 5, wobei $R_3$ ein Chlor- oder Bromatom ist, dadurch gekennzeichnet, dass man das Dihalogenzwischenprodukt der allgemeinen Formel

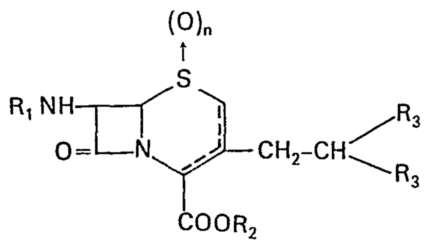

(oder ein Gemisch seiner Isomeren)
das in Bicycloocten-2- oder -3-Form vorliegt, wenn n = 0 und in Bicycloocten-2-Form, wenn n = 1, und worin $R_1$ und $R_2$ wie in Anspruch 6 für den Ausgangsaldehyd definiert ist, und $R_3$ wie vorstehend definiert ist, dass man dann die Dehydrohalogenierung dieses Derivats vornimmt, gegebenenfalls das erhaltene Sulfoxid reduziert, gegebenenfalls die Schutzgruppen entfernt, gegebenenfalls die Isomeren des erhaltenen Produkts auftrennt und gegebenenfalls dieses Produkt in ein Salz überführt.

9. Ein Dihalogenderivat der allgemeinen Formel

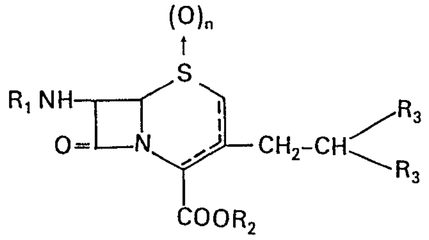

das in Bicyclooncten-2- oder -3-Form vorliegt, wenn n = 0 und in Bicycloocten-2-Form, wenn n = 1, und worin $R_1$ wie in Anspruch 1 definiert ist, $R_2$ wie in Anspruch 4 definiert ist und $R_3$ ein Chlor- oder Bromatom bedeutet sowie die Gemische seiner Isomeren.

10. Verfahren zur Herstellung eines Produktes gemäss Anspruch 1, worin $R_2$, $R_3$ und n wie vorstehend definiert sind, $R_1$ ein Wasserstoffatom bedeutet, dadurch gekennzeichnet, dass man den Rest $R_1$ entfernt oder gegebenenfalls gleichzeitig die Reste $R_1$ und $R_2$ aus einem Produkt gemäss Anspruch 1 entfernt, wobei $R_3$ und n wie in Anspruch 1 definiert sind, $R_1$ und $R_2$ wie in Anspruch 1 unter 1a) definiert sind, mit der Ausnahme der Bedeutung von $R_1$ entweder Wasserstoff oder ein Rest der allgemeinen Formel

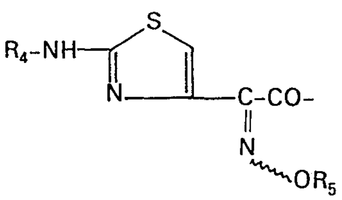

oder aber $R_1$ bedeutet einen 5-Aminoadipoylrest, dessen Amin- und Säurefunktionen geschützt sind, oder einen Rest

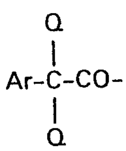

oder Ar-X-$CH_2$CO-

wie in Anspruch 1 definiert und $R_2$ ist wie in Anspruch 1 unter 1b) definiert, dass man dann gegebenenfalls das erhaltene Produkt in seine Isomeren auftrennt und es gegebenenfalls in ein Salz überführt.

11. Verfahren zur Herstellung eines Produkts gemäss einem der Ansprüche 1, 2, 3, 4 oder 5, wobei n, $R_2$ und $R_3$ wie gemäss Anspruch 1 definiert sind, $R_1$ einen Rest der allgemeinen Formel

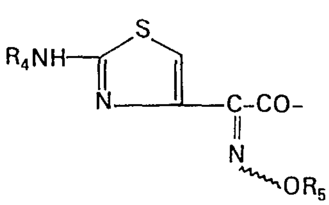

wie er unter 2 oder unter 1a) in Anspruch 1 definiert ist, oder in Anspruch 2 oder 3 definiert ist, oder $R_1$ ist definiert wie unter 1b) in Anspruch 1, mit Ausnahme der Bedeutung Wasserstoff, dadurch gekennzeichnet, dass man mittels einer Säure, dargestellt durch die allgemeine Formel

$R_1OH$

(worin $R_1$ wie vorstehend definiert ist, wobei gegebenenfalls die Reste $R_4$ und/oder $R^c_5$, die in $R_1$ enthalten sind, anders als Wasserstoffatome sind), oder eines reaktiven Derivats dieser Säure, ein Produkt gemäss Anspruch 1, für das $R_1$ Wasserstoff bedeutet oder gegebenenfalls ein Gemisch der Isomeren dieses Produkts acyliert, dass man dann gegebenenfalls das erhaltene Oxid reduziert, gegebenenfalls die Schutzgruppen entfernt, gegebenenfalls die Isomeren des erhaltenen Produkts auftrennt und es gegebenenfalls in ein Salz überführt.

12. Verfahren zur Herstellung eines Produkts gemäss einem der Ansprüche 1, 2, 3, 4 oder 5, worin n = 1, dadurch gekennzeichnet, dass man ein Produkt gemäss Anspruch 1, 2 oder 3, worin n = 0, oxidiert nach jeder Methode, die zur Herstellung eines Sulfoxids aus einem Sulfid bekannt ist, ohne dass der Rest des Moleküls beeinträchtigt wird, dass man dann gegebenenfalls die Isomeren des erhaltenen Produkts auftrennt und es gegebenenfalls in ein Salz überführt.

13. Verfahren zur Herstellung eines Produkts gemäss einem der Ansprüche 1, 2, 3, 4 oder 5, wobei n wie in Anspruch 1 definiert ist und $R_1$ wie in Anspruch 1 under 2) oder unter 1a), oder wie in Anspruch 2, 3 oder 4 definiert ist, $R_2$ einen Rest der allgemeinen Formel

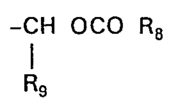

wie in Anspruch 1 definiert ist und $R_3$ wie in Anspruch 1 oder 5 definiert ist, dadurch gekennzeichnet, dass man ein Produkt gemäss einem der Ansprüche 1, 2 oder 3 [wofür $R_1$, $R_3$ und n wie vorstehend definiert sind (wobei, falls $R_1$ einen Rest $R^c_5$ enthält, dieser anders als ein Wasserstoffatom ist), $R_2$ ein Wasserstoffatom ist] verestert, dass man dann das erhaltene Oxid gegebenenfalls reduziert, gegebenenfalls die Schutzgruppen entfernt und gegebenenfalls das erhaltene Produkt in seine Isomeren auftrennt.

14. Verfahren zur Herstellung eines Produkts gemäss einem der Ansprüche 1, 2, 3, 4 oder 5, wobei n wie in Anspruch 1 definiert ist, $R_1$ einen Rest der allgemeinen Formel

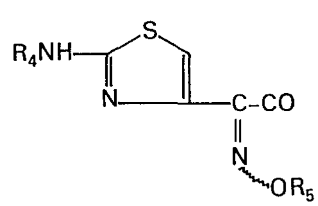

wie in Anspruch 1 unter 2) oder unter 1a) oder in den Ansprüchen 2, 3 oder 4 definiert (mit der Ausnahme, dass $R_4$ einen Chloracetyl- oder Trichloracetylrest bedeutet und/oder $R_5$ Vinyl bedeutet), $R_2$ die entsprechende, in Anspruch 1 angegebene Definition hat und $R_3$ wie in Anspruch 1 oder 5 definiert ist, dadurch gekennzeichnet, dass man einen Thioharnstoff der allgemeinen Formel

$R_4NH-CS-NH_2$

worin $R_4$ wie vorstehend definiert ist, auf ein Cephalosporinderivat der allgemeinen Formel

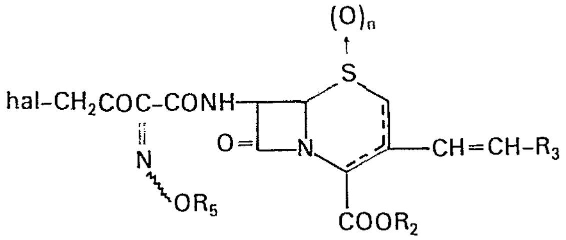

worin $R_3$ und n wie in Anspruch 1 definiert sind, $R_2$ und $R_5$ wie vorstehend definiert sind und hal ein Chlor- oder Bromatom bedeutet, oder gegebenenfalls auf ein Gemisch der Isomeren dieses Produkts einwirken lässt, dass man dann das erhaltene Oxid gegebenenfalls reduziert, gegebenenfalls die Schutzgruppen entfernt, die Isomeren des enthaltenen Produkts gegebenenfalls auftrennt und es gegebenenfalls in ein Salz überführt.

**Patentansprüche für den Vertragsstaat AT**

1. Verfahren zur Herstellung eines 3-Vinyl-cephalosporins der allgemeinen Formel:

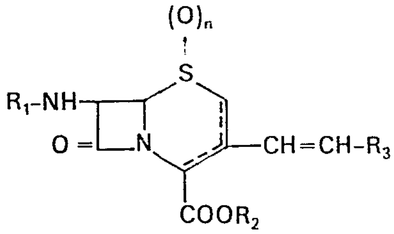

(I)

in welcher n gleich 0 oder 1 ist,
1a) das Symbol $R_1$ darstellt
ein Wasserstoffatom,
einen Rest der allgemeinen Formel:

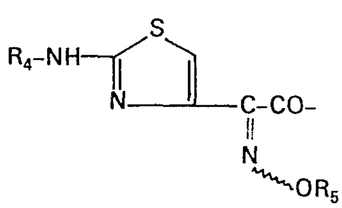

(II)

in syn- oder anti-Form
(in welcher $R_4$ ein Wasserstoffatom oder eine Aminschutzgruppe ist und $R_5$ ein Wasserstoffatom, ein Alkyl-, Vinyl- oder Cyanomethylrest oder eine Oximschutzgruppe ist),
einen Benzydryl-, Tritylrest,
einen Acylrest der allgemeinen Formel:

$R_6CO-$,

in welcher $R_6$ ein Wasserstoffatom, ein Alkylrest (gegebenenfalls substituiert durch ein oder mehrere Halogenatome oder durch einen Phenyl- oder Phenoxyrest) oder ein Phenylrest ist, oder einen Rest der allgemeinen Formel:

$R_7$ O CO-,

in welcher $R_7$ ein verzweigter, nicht substituierter Alkylrest oder ein geradkettiger oder verzweigter Alkylrest mit einem oder mehreren Substituenten [ausgewählt aus den Halogenatomen und den Cyano-, Trialkylsilyl-, Phenyl- und (durch ein oder mehrere Alkyloxy-, Nitro- oder Phenylgruppen substituierten) Phenylresten], ein Vinyl-, Allyl- oder Chinolylrest ist, oder
einen Nitrophenylthiorest, oder
$R_1NH-$ ist ersetzt durch einen Methylenaminorest, worin der Methylenrest substituiert ist durch eine Dialkylamino- oder Arylgruppe (ihrerseits gegebenenfalls substituiert durch einen oder mehrere Methoxy- oder Nitroreste), und
das Symbol $R_2$ darstellt
ein Wasserstoffatom, einen leicht auf enzymatischem Wege entfernbaren Rest der allgemeinen Formel:

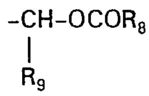

(in welcher $R_8$ einen Alkylrest oder den Cyclohexylrest darstellt und $R_9$ ein Wasserstoffatom oder einen Alkylrest darstellt) oder einen Methoxymethyl-, tert.Butyl-, Benzhydryl-, Nitrobenzyl- oder p-Methoxybenzylrest, oder
1b) das Symbol $R_1$ darstellt
- ein Wasserstoffatom,
- einen Alkanoylrest mit 1 bis 8 Kohlenstoffatomen, einen (durch Chlor- oder Bromatome substituierten) Alkanoylrest mit 2 bis 8 Kohlenstoffatomen,
- Azidoacetyl, Cyanoacetyl
- einen Acylrest der allgemeinen Formel:

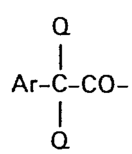

{in welcher Q für H oder Methyl steht und Ar einen 2-Thienyl-, 3-Thienyl-, 2-Furyl-, 3-Furyl-, 2-Pyrrolyl-1, 3-Pyrrolyl- oder [gegebenenfalls durch Halogenatome oder Trifluormethyl-, Hydroxy-, Alkyl- (mit 1 bis 3 Kohlenstoffatomen), Alkyloxy- (mit 1 bis 3 Kohlenstoffatomen), Cyano- oder Nitroreste, von denen sich zumindest einer in meta- oder para-Stellung des Phenyls befindet, substituierten] Phenylrest darstellt},
- einen Acylrest der allgemeinen Formel:

$Ar-X-CH_2-CO-$,

worin X Sauerstoff oder Schwefel ist und Ar die obige Bedeutung hat oder Ar-X- für 4-Pyridylthio steht,
- einen Acylrest der allgemeinen Formel:

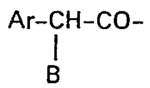

in welcher Ar die obige Bedeutung hat und B einen Aminorest, einen (durch eine Benzyloxycarbonyl-, Alkyloxycarbonyl-, Cyclopentyloxycarbonyl-, Cyclohexyloxycarbonyl-, Benzhydryloxycarbonyl-, Trityl- oder 2,2,2-Trichlor-äthoxycarbonylgruppe) geschützten Aminorest, einen Sulforest, einen Hydroxy- oder Carboxyrest [gegebenenfalls geschützt durch Veresterung (mit einer Alkansäure bzw. einem Alkohol mit 1 bis 6 Kohlenstoffatomen)], Azido, Cyano oder Carbamoyl darstellt,
- oder einen 2-(3-Sydnon)-alkanoylrest (dessen Alkanoylteil 1 bis 3 Kohlenstoffatome enthält),
- oder einen Acylrest der allgemeinen Formel:

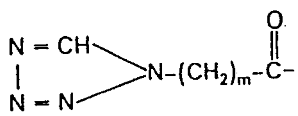

in welcher m gleich 0 bis 2 ist,
- oder einen 5-Aminoadipoylrest [in welchem die Aminogruppe gegebenenfalls geschützt ist durch einen Alkanoylrest (mit 1 bis 3 Kohlenstoffatomen und gegebenenfalls substituiert durch ein Chloratom) und in welchem die Carboxygruppe durch einen Benzhydryl-, 2,2,2-Trichloräthyl-, tert.-Alkyl- (mit 4 bis 6 Kohlenstoffatomen) oder Nitrobenzylrest geschützt ist],
oder $R_1NH-$ ist ersetzt durch eine cyclische Imidgruppe einer Dicarbonsäure,
das Symbol $R_2$ darstellt einen tert.-Alkylrest mit 4 bis 6 Kohlenstoffatomen, tert.-Alkenyl mit 6 oder 7 Kohlenstoffatomen, tert.-Alkynyl mit 6 oder 7 Kohlenstoffatomen, Benzyl, Methoxybenzyl, Nitrobenzyl, 2,2,2-Trichloräthyl, Benzhydryl, Succinimidomethyl oder Phthalimidomethyl oder ein Wasserstoffatom und
das Symbol $R_3$ darstellt ein Halogenatom oder
2) das Symbol $R_1$ darstellt einen Rest der allgemeinen Formel (II), worin $R_4$ die unter 1a) angegebene Bedeutung hat und $R_5$ einen Rest der allgemeinen Formel:

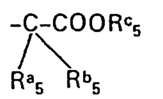 (IIa)

(in welcher $R^a_5$ und $R^b_5$, die gleich oder voneinander verschieden sind, Wasserstoffatome oder Alkylreste darstellen oder zusammen einen Alkylenrest mit 2 oder 3 Kohlenstoffatomen bilden und $R^c_5$ ein Wasserstoffatom oder eine Säureschutzgruppe darstellen), bedeutet,
das Symbol $R_2$ die zuvor unter 1a) angegebene Bedeutung hat und das Symbol $R_3$ ein Halogenatom oder einen Rest der allgemeinen Formel:

$R'_3-SO_2O-$ oder $R''_3-COO-$

darstellt, in welchen $R'_3$ einen Alkyl-, Trifluormethyl-, Trichlormethyl oder gegebenenfalls (durch ein Halogenatom oder einen Alkyl- oder Nitrorest) substituierten Phenylrest darstellt und $R''_3$ die für $R'_3$ angegebene Bedeutung hat oder einen Acylmethyl-, 2-Acyläthyl-, 2-Acylpropyl-, Alkyloxycarbonylmethyl-, 2-Alkyloxycarbonyläthyl- oder 2-Alkyloxycarbonylpropylrest darstellt,
wobei die oben erwähnten Alkyl- oder Acylteile oder -reste (ohne besonderen Hinweis) geradkettig oder verzweigt sind und 1 bis 4 Kohlenstoffatome enthalten, die Verbindung, wenn n = 0, in 2- oder 3-Bicycloocten-Form und, wenn n = 1, in 2-Bicycloocten-Form vorliegt und der Substituent in 3-Stelung E- oder Z-Form hat, sowie der Mischungen seiner Isomeren und Salze, dadurch gekennzeichnet, dass man
A) zur Herstellung einer Verbindung, in deren Formel $R_1$, $R_2$ und $R_3$ die unter 2) angegebene Bedeutung haben oder $R_1$ und $R_2$ die unter 1a) angegebene Bedeutung, mit Ausnahme von Wasserstoff für $R_1$, haben oder $R_1$ einen Alkanoylrest mit 1 bis 8 Kohlenstoffatomen, einen (durch Chlor- oder Bromatome substituierten) Alkanoylrest der allgemeinen Formel:

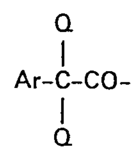

{in welcher Q für H oder Methyl steht und Ar einen 2-Thienyl-, 3-Thienyl-, 2-Furyl-, 3-Furyl-, 2-Pyrrolyl-, 3-Pyrrolyl- oder [gegebenenfalls durch Halogenatome oder Hydroxy-, Alkyl- (mit 1 bis 3 Kohlenstoffatomen) oder Alkyloxyreste (mit 1 bis 3 Kohlenstoffatomen), von denen sich zumindest einer in meta- oder para-Stellung des Phenyls befindet, substituierten] Phenylrest darstellt},
einen Acylrest der allgemeinen Formel:

$Ar-X-CH_2-CO-$

wie oben definiert,
einen Alcylrest entsprechend der allgemeinen Formel:

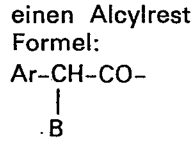

worin Ar die obige Bedeutung hat und B einen (durch eine Benzyl-oxycarbonyl-, Alkyloxycarbonyl-, Cyclopentyloxycarbonyl-, Cyclohexyloxycarbonyl-, Benzhydryloxycarbonyl-, Trityl- oder 2,2,2-Trichchloräthoxycarbonylgruppe) geschützten Aminorest, einen Sulforest, einen (gegebenenfalls durch Veresterung mit einer Alkansäure bzw. einem Alkohol mit 1 bis 6 Kohlenstoffatomen geschützten) Hydroxy- oder Carboxyrest, oder einen 5-Amino-adipoylrest [worin die Aminogruppe gegebenenfalls durch einen Alkanoylrest (mit 1 bis 3 Kohlenstoffatomen und gegebenenfalls substituiert durch ein Chloratom) geschützt ist und worin die Carboxygruppe durch eine Benzhydryl-, 2,2,2-Trichloräthyl-, tert.-Alkyl- (mit 4 bis 6 Kohlenstoffatomen) oder Nitrobenzylgruppe geschützt ist] und

$R_2$ die zuvor under 1b) angegebene Bedeutung hat und

$R_3$ die unter 1) angegebene Bedeutung hat, ein Halogenierungsmittel oder eine Verbindung der allgemeinen Formel:

$(R'_3SO_2)_2O$

$R'_3SO_2Hal$

$(R''_3CO)_2O$

oder $R''_3COHal$

in welchen $R'_3$ und $R''_3$ die oben angegebene Bedeutung haben und Hal ein Halogenatom darstellt, auf eine Verbindung der allgemeinen Formel:

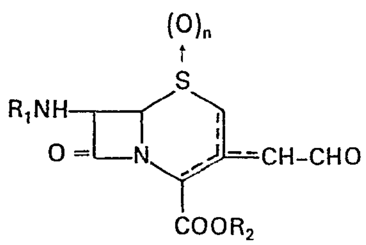

{in welcher die Verbindung, wenn n = 0, in 3-Oxoäthyl-2- oder -3-bicycloocten-Form oder 3-Oxoäthyliden-bicyclooctan-Form und, wenn n = 1, in 3-Oxoäthyl-2-bicycloocten-Form oder 3-Oxoäthyliden-bicyclooctan-Form vorliegt und $R_1$ die obige Bedeutung hat [mit Ausnahme eines Restes der allgemeinen Formel:

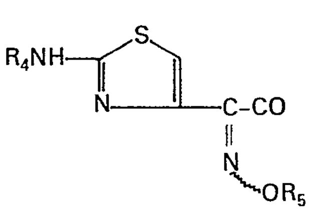

in welcher $R_4$ und/oder $R_5$ Wasserstoff sind (und/oder $R_{c5}$ in $R_5$ Wasserstoff ist, wenn man ein Halogenierungsmittel einsetzt)] und $R_2$ die obige Bedeutung mit Ausnahme von Wasserstoff hat} oder auf eine Mischung ihrer Isomeren einwirken lässt, dann gegebenenfalls das erhaltene Sulfoxid reduziert oder, umgekehrt, die erhaltene Verbindung zu ihrem S-Oxid oxidiert, gegebenenfalls die Schutzgruppen entfernt, gegebenenfalls die Isomeren der erhaltenen Verbindung trennt und gegebenenfalls diese Verbindung in ein Salz überführt, dann

B) zur Herstellung einer Verbindung, in deren Formel $R_2$, $R_3$ und n die obige Bedeutung haben, und $R_1$ ein Wasserstoffatom darstellt, den Rest $R_1$ entfernt oder gegebenenfalls gleichzeitig die Reste $R_1$ und $R_2$ einer gemäss A hergestellten Verbindung, in deren Formel $R_1$ und $R_2$ die unter 1a) angegebene Bedeutung, mit Ausnahme von Wasserstoff oder einem Rest der allgemeinen Formel:

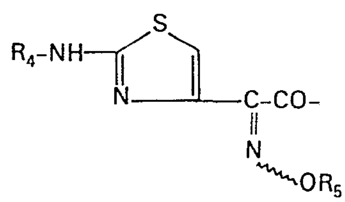

für $R_1$, haben oder $R_1$ einen 5-Amino-adipoylrest, dessen Amin- und Säurefunktionen geschützt sind, oder einen Rest

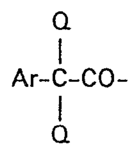

oder $Ar-X-CH_2CO-$

wie zuvor definiert, darstellt und $R_2$ die unter 1b) angegebene Bedeutung hat, entfernt, dann gegebenenfalls die erhaltene Verbindung in ihre Isomeren auftrennt und sie gegebenenfalls in ein Salz überführt und dann

C) zur Herstellung einer Verbindung, in deren Formel $R_1$ einen Rest der allgemeinen Formel:

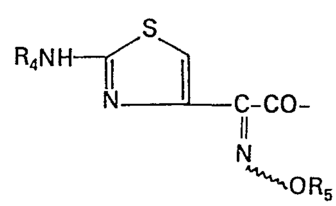

wie unter 2) oder unter 1a) angegeben darstellt oder $R_1$ die unter 1b) angegebene Bedeutung, ausgenommen ein Wasserstoffatom, hat, mittels einer Säure der allgemeinen Formel:

$R_1OH$

[worin $R_1$ die obige Bedeutung hat (selbstverständlich sind zutreffendenfalls die in $R_1$ enthaltenen Reste $R_4$ und/oder $R^c_5$ von Wasserstoffatomen verschieden)] oder eines reaktionsfähigen Derivats dieser Säure eine gemäss B hergestellte Verbindung, worin $R_1$ Wasserstoff darstellt, oder

gegebenenfalls eine Mischung der Isomeren dieser Verbindung acyliert, dann gegebenenfalls das erhaltene Oxid reduziert oder, umgekehrt, die erhaltene Verbindung zu ihrem S-Oxid oxidiert, gegebenenfalls die Schutzgruppen entfernt, gegebenenfalls die Isomeren der erhaltenen Verbindung trennt und sie gegebenenfalls in ein Salz überführt und dann

D) zur Herstellung einer Verbindung, in deren Formel $R_1$ die unter 2) oder unter 1) angegebene Bedeutung hat, $R_2$ einen Rest der allgemeinen Formel:

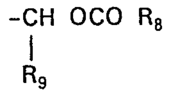

wie oben angegeben, darstellt und $R_3$ die obige Bedeutung hat, die erhaltene Verbindung [in deren Formel $R_1$, $R_3$ und n die zuvor angegebene Bedeutung haben (selbstverständlich ist ein in $R_1$ enthaltener Rest $R^c_5$ von einem Wasserstoffatom verschieden) und $R_2$ ein Wasserstoffatom ist] verestert, dann gegebenenfalls das erhaltene Oxid reduziert oder, umgekehrt, die erhaltene Verbindung zu ihrem S-Oxid oxidiert, gegebenenfalls die Schutzgruppen entfernt und gegebenenfalls die erhaltene Verbindung in ihre Isomeren spaltet, oder

E) zur Herstellung einer Verbindung, in deren Formel $R_1$ einen Rest der allgemeinen Formel:

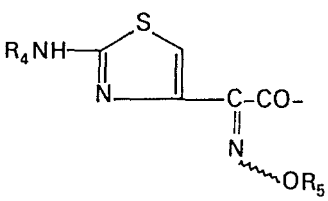

wie unter 2) oder unter 1a) (mit Ausnahme eines Chloracetyl- oder Trichloracetylrests für $R_4$ und/oder von Vinyl für $R_5$) angegeben, darstellt und n, $R_2$ und $R_3$ eine entsprechende Bedeutung haben, einen Thioharnstoff der allgemeinen Formel:

$R_4NH-CS-NH_2$

in welcher $R_4$ die obige Bedeutung hat, auf ein Cephalosporinderivat der allgemeinen Formel:

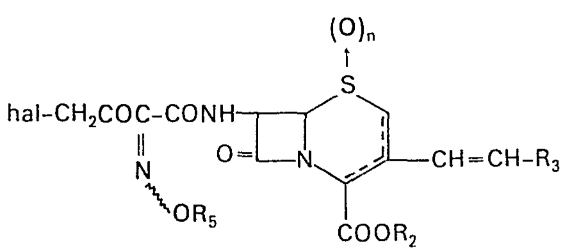

in welcher $R_2$, $R_3$, $R_5$ und n die obige Bedeutung haben und hal ein Chlor- oder Bromatom darstellt, oder gegebenenfalls auf eine Mischung der Isomeren dieser Verbindung einwirken lässt, dann gegebenenfalls das erhaltene Oxid reduziert, gegebenenfalls die Schutzgruppen entfernt, gegebenenfalls die Isomeren der erhaltenen Verbindung trennt und sie gegebenenfalls in ein Salz umwandelt.

2. Verfahren nach Anspruch 1 zur Herstellung einer Verbindung, in deren Formel $R_3$ ein Chlor- oder Bromatom ist, dadurch gekennzeichnet, dass man in der Stufe A das dihalogenierte Zwischenprodukt der allgemeinen Formel:

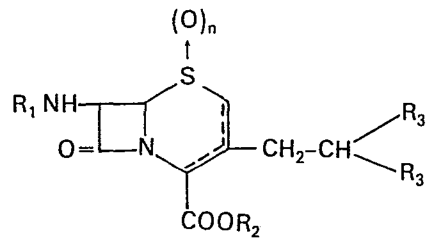

(oder eine Mischung seiner Isomeren),
die in 2- oder 3-Bicycloocten-Form vorliegt, wenn n = 0, und in 2-Bicycloocten-Form, wenn n = 1, und worin $R_1$ und $R_2$ die in Anspruch 1 für den Ausgangsaldehyd angegebene Bedeutung haben, und $R_3$ die obige Bedeutung hat, isoliert und dann die Dehydrohalogenierung dieses Derivats durchführt.

**Claims for the contracting States: BE CH DE FR GB IT LI LU NL SE**

1. A 3-vinylcephalosporin characterised in that it corresponds to the general formula:

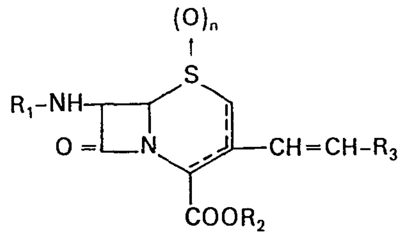

(I)

in which n is equal to 0 or 1,
1a) - the symbol $R_1$ denotes
a hydrogen atom,
a radical of general formula

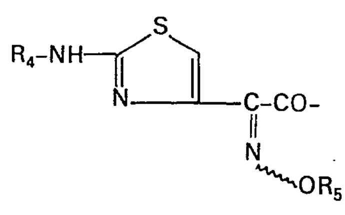

(II)

in syn or anti form,
(in which $R_4$ is a hydrogen atom or an amine-protecting radical and $R_5$ is a hydrogen atom, an alkyl, vinyl or cyanomethyl radical or an oxime-protecting group),
a benzhydryl or trityl radical,
an acyl radical of general formula:

$R_6CO-$

in which $R_6$ is a hydrogen atom or an alkyl (optionally substituted by one or more halogen atoms or by a phenyl or phenoxy radical) or phenyl radical, or
a radical of general formula:

$R_7OCO-$

in which $R_7$ is a branched unsubstituted alkyl radical or a straight-chain or branched alkyl radical bearing one or more substituents [chosen from halogen atoms and cyano, trialkylsilyl, phenyl, substituted phenyl (by one or more alkoxy, nitro or phenyl radicals)], vinyl, allyl or quinolyl radicals, or
a nitrophenylthio radical, or
$R_1NH-$ is replaced by a methyleneamino radical in which the methylene radical is substituted by a dialkylamino group or aryl group (itself optionally substituted by one or more methoxy or nitro radicals) and
the symbol $R_2$ denotes a hydrogen atom, a radical which is readily capable of elimination by an enzymatic route, of general formula:

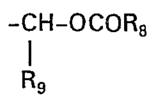

(in wich $R_8$ denotes an alkyl radical or the cyclohexyl radical, and $R_9$ denotes a hydrogen atom or an alkyl radical) or a methoxymethyl, t-butyl, benzhydryl, nitrobenzyl or p-methoxybenzyl radical, or
1b) - the symbol $R_1$ denotes
- a hydrogen atom,
- an alkanoyl radical containing 1 to 8 carbon atoms, alkanoyl containing 2 to 8 carbon atoms which is substituted (by chlorine or bromine atoms),
- azidoacetyl, cyanoacetyl
- an acyl radical of general formula:

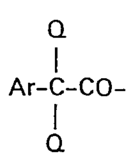

{in which Q is H or methyl and Ar denotes a 2-thienyl, 3-thienyl, 2-furyl, 3-furyl, 2-pyrrolyl, 3-pyrrolyl or phenyl [optionally substituted by halogen atoms or trifluoromethyl, hydroxy, alkyl (containing 1 to 3 carbon atoms), alkoxy (containing 1 to 3 carbon atoms), cyano or nitro radicals, at least one of which is situated in the meta or para position of the phenyl] radical},
an acyl radical of general formula:

$Ar-X-CH_2-CO-$

in which X is oxygen or sulphur and Ar is defined as above, or Ar-X- denotes 4-pyridylthio,
- an acyl radical of general formula:

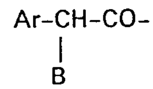

in which Ar is defined as previously and B denotes an amino radical, an amino radical which is protected (by a benzyloxycarbonyl, alkoxycarbonyl, cyclopentyloxycarbonyl, cyclohexyloxycarbonyl, benzhydroloxycarbonyl, trityl or 2,2,2-trichloroethoxycarbonyl group), a sulpho radical, a hydroxy or carboxy radical [which radicals are optionally protected by esterification (with an alkanoic acid or an alcohol containing 1 to 6 carbon atoms respectively)], an azido, cyano or carbamoyl radical,
or a 2-(3-sydnone)alkanoyl radical (the alkanoyl part of which contains 1 to 3 carbon atoms)
or an acyl radical of general formula:

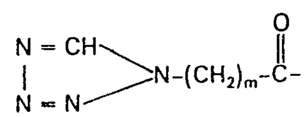

in which m is 0 to 2
or a 5-aminoadipoyl radical [in which the amino group is optionally protected by an alkanoyl radical (containing 1 to 3 carbon atoms and optionally substituted by a chlorine atom) and in which the carboxy group is protected by a benzhydryl, 2,2,2-trichloroethyl, t-alkyl (containing 4 to 6 carbon atoms) or nitrobenzyl radical], or $R_1NH$ is replaced by a dicarboxylic acid cyclic imide group,
the symbole $R_2$ denotes a t-alkyl radical containing 4 to 6 carbon atoms, t-alkenyl radical containing 6 or 7 carbon atoms, t-alkynyl radical containing 6 or 7 carbon atoms, a benzyl, methoxybenzyl, nitrobenzyl, 2,2,2-trichloroethyl, benzhydryl, succinimidomethyl or phthalimidomethyl radical or a hydrogen atom and
the symbol $R_3$ denotes a halogen atom or
2) the symbol $R_1$ denotes a radical of general formula (II) in which $R_4$ is defined as in 1a) - and $R_5$ denotes a radical of general formula

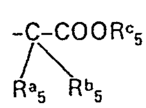 (IIa)

(in wich
$R^a_5$ and $R^b_5$ which are identical or different, denote hydrogen atoms or alkyl radicals or form together an alkylene radical containing 2 or 3 carbon atoms and $R^c_5$ denotes a hydrogen atom or an acid-protecting radical),
the symbol $R_2$ is defined as previously at 1a) – and the symbol $R_3$ denotes a radical of general formula:

$R'_3$-$SO_2O$-

or $R''_3$-COO-

in which formulae $R'_3$ denotes an alkyl, trifluoromethyl, or trichloromethyl radical or a phenyl radical which is unsubstituted or substituted (by a halogen atom or an alkyl or nitro radical) and $R''_3$ is defined as $R'_3$ or denotes an acylmethyl, 2-acylethyl, 2-acylpropyl, alkoxycarbonylmethyl, 2-alkoxycarbonylethyl or 2-alkoxycarbonylpropyl radical, or $R_3$ denotes a halogen atom,
the alkyl or acyl portions or radicals mentioned above being (unless specially mentioned) straight-chain or branched and containing 1 to 4 carbon atoms, the product being in a 2- or 3-bicyclooctene form when n = 0 and in a 2-bicyclooctene form when n = 1 and the substituent in the 3-position being in an E or Z form, as well as the mixtures of its isomers and its salts.

2. A product according to Claim 1, characterised in that, n, $R_2$ and $R_{.3}$ being defined as in Claim 1 at 2),
$R_1$ denotes a radical of general formula:

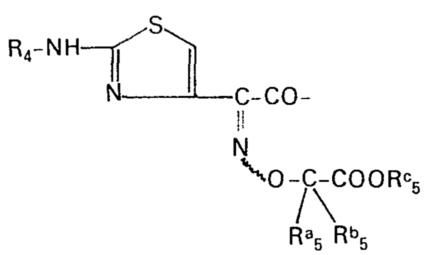

in a syn or anti form, in which $R^a_5$, $R^b_5$ and $R^c_5$ are defined as in Claim 1 and $R_4$ is a hydrogen atom or a t-butoxycarbonyl, 2,2,2-trichloroethoxycarbonyl, chloroacetyl, trichloroacetyl, trityl, benzyl, dibenzyl, benzyloxycarbonyl, p-nitrobenzyloxycarbonyl, p-methoxybenzyloxycarbonyl, formyl or trifluoroacetyl radical.

3. A product according to Claim 1, characterised in that, n, $R_2$ and $R_3$ being defined as in Claim 1 at 1a),
$R_1$ denotes a radical or general formula:

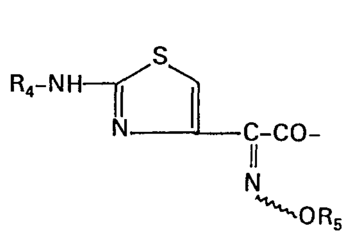

in a syn or anti form, in which $R_4$ is a hydrogen atom or a t-butoxycarbonyl, 2,2,2-trichloroethoxycarbonyl, chloroacetyl, trichloroacetyl, trityl, benzyl, dibenzyl, benzyloxycarbonyl, p-nitrobenzyloxycarbonyl, or p-methoxybenzyloxycarbonyl radical, and $R_5$ is a hydrogen atom or an alkyl, vinyl, cyanomethyl, trityl or tetrahydropyranyl or 2-methoxy-2-propyl radical.

4. A product according to Claim 1, characterised in that the protecting radical $R^c_5$ is chosen from methoxymethyl, t-butyl, benzhydryl, nitrobenzyl, benzyl and p-methoxybenzyl.

5. A product according to Claim 1, characterised in that the radical $R_3$ denotes a halogen atom chosen from chlorine, bromine and iodine.

6. A process for the preparation of a product according to one of Claims 1, 2, 3, 4 or 5 for which, n being defined according to Claim 1, either $R_1$, $R_2$ and $R_3$ are defined as in Claim 1 at 2) or in Claim 2, or $R_1$ and $R_2$ are defined as in Claim 1 at 1a) or in Claim 3, except for $R_1$ denoting the hydrogen atom, or $R_1$ denotes an alkanoyl radical containing 1 to 8 carbon atoms, or an alkanoyl radical containing 2 to 8 carbon atoms which is substituted (by chlorine or bromine atoms),
an acyl radical of general formula:

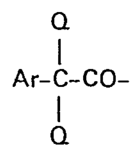

{in which Q is H or methyl and Ar denotes a 2-thienyl, 3-thienyl, 2-furyl, 3-furyl, 2-pyrrolyl, 3-pyrrolyl or phenyl [optionally substituted by halogen atoms or hydroxy, alkyl (containing 1 to 3 carbon atoms) or alkoxy (containing 1 to 3 carbon atoms) radicals, at least one of which is situated in the meta or para position of the phenyl] radical}, an acyl radical of general formula:

Ar-X-$CH_2$-CO-

such as defined in Claim 1,
an acyl radical corresponding to the general formula:

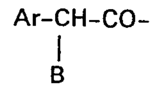

in which Ar is defined as in Claim 1, and B denotes an amino radical which is protected (by a benzyloxycarbonyl, alkoxycarbonyl, cyclopentyloxycarbonyl, cyclohexyloxycarbonyl, benzhydryloxycarbonyl, trityl or 2,2,2-trichloroethoxycarbonyl group) a sulpho radical, a hydroxy or carboxy radical (optionally protected by esterification, with an alkanoic acid or an alcohol containing 1 to 6 carbon atoms respectively), or
a 5-aminoadipoyl radical [in which the amino group is optionally protected by an alkanoyl radical (containing to 3 carbon atoms and optionally

substituted by a chlorine atom) and in which the carboxy group is protected by a benzhydryl, 2,2,2-trichloroethyl, t-alkyl (containing 4 to 6 carbon atoms) or nitrobenzyl group] and $R_2$ is defined as previously in Claim 1 at 1b) and $R_3$ is defined as in Claim 1 at 1),
characterised in that a halogenating agent or a product of general formula:

$(R'_3SO_2)_2O$

$R'_3SO_2Hal$

$(R''_3CO)_2O$

or $R''_3COHal$

in which $R'_3$ and $R''_3$ are defined as in Claim 1 and Hal denotes a halogen atom is made to react with a product of general formula:

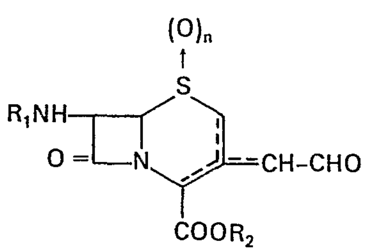

{in which, n being defined as in Claim 1, the product is in a 3-oxoethyl-2-bicyclooctene or -3-bicyclooctene or 3-oxoethylidenebicyclooctane form when n = 0 and in a 3-oxoethyl-2-bicyclooctene or 3-oxoethylidenebicyclooctane form when n = 1, and $R_1$ is defined as above [except for denoting a radical of general formula:

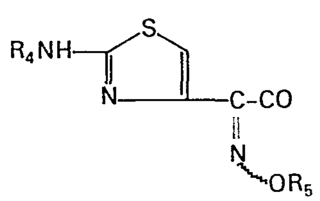

in which $R_4$ and/or $R_5$ are hydrogen, (and/or $R^c_5$ in $R_5$ is hydrogen when a halogenating agent is made to react)] and $R_2$ is defined as above except for denoting hydrogen}, or with a mixture of its isomers, and then the sulphoxide obtained is optionally reduced, the protecting groups are optionally removed, the isomers of the product obtained are optionally separated and this product is optionally converted into a salt.

7. A process according to Claim 6, for the preparation of a product according to one of Claims 1, 2, 3, 4 or 5, for which $R_3$ is a halogen atom chosen from chlorine, bromine and iodine, characterised in that a halogenating agent chosen from the addition compounds of chlorine, bromine or iodine with triaryl phosphite, phosphorus trichloride or tribromide, phosphorus oxychloride or oxybromide, phosphorus pentachloride or pentabromide, dichlorotriphenylphosphorane and catechyltrichloro (or tribromo) phosphorane, is made to react.

8. A process according to Claim 6 for the preparation of a product according to one of Claims 1, 2, 3, 4 or 5, for which $R_3$ is a chlorine or bromine atom, characterised in that the dihalogenated intermediate of general formula:

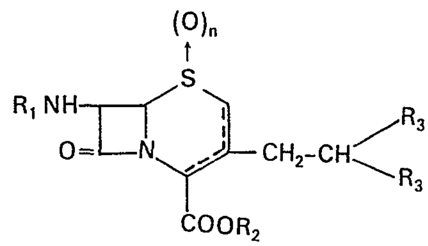

(or a mixture of its isomers)
which is in a 2- or 3-bicyclooctene form when n = 0 and in a 2-bicyclooctene form when n = 1, and in which $R_1$ and $R_2$ are defined as in Claim 6 for the starting aldehyde and $R_3$ is defined as above, is isolated, and then the dehydrohalogenation of this derivative is carried out, the sulphoxide obtained is optionally reduced, the protecting radicals are optionally removed, the isomers of the product obtained are optionally separated and this product is optionally converted into a salt.

9. A dihalogenated derivative, of general formula:

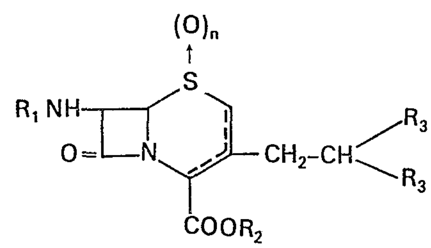

which is in a 2- or 3-bicyclooctene form when n = 0 and in a 2-bicyclooctene form when n = 1 and in which, $R_1$ being defined as in Claim 1, $R_2$ is defined as in Claim 4 and $R_3$ denotes a chlorine or bromine atom, as well as mixtures of its isomers.

10. A process for the preparation of a product according to Claim 1, for which, $R_2$, $R_3$ and n being defined as previously, $R_1$ denotes a hydrogen atom,
characterised in that the radical $R_1$ is removed or optionally in that the radicals $R_1$ and $R_2$ are simultaneously removed from a product according to Claim 1 for which, $R_3$ and n being defined as in Claim 1,
$R_1$ and $R_2$ are defined as in Claim 1 at 1a), except for
$R_1$ denoting either hydrogen or a radical of general formula:

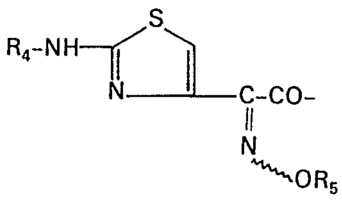

or $R_1$ denotes a 5-aminoadipoyl radical the amine and acid groups of which are protected, or a radical

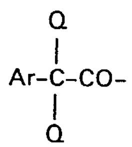

or

$Ar-X-CH_2CO-$

such as defined in Claim 1 and $R_2$ is defined as in Claim 1 at 1b), and then the product obtained is optionally separated into its isomers and is optionally converted into a salt.

11. A process for the preparation of a product according to one of Claims 1, 2, 3, 4 or 5 for which, n, $R_2$ and $R_3$ being defined according to Claim 1, $R_1$ denotes a radical of general formula:

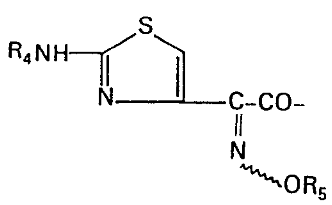

such as defined respectively at 2) or at 1a) in Claim 1 or in Claim 2, or 3, or $R_1$ is defined as at 1b) in Claim 1 except for denoting the hydrogen atom, characterised in that a product according to Claim 1 for which $R_1$ denotes hydrogen, or optionally a mixture of the isomers of this product is acylated by means of an acid denoted by the general formula:

$R_1OH$

(in which $R_1$ is defined as above, it being understood that, where appropriate, the radicals $R_4$ and/or $R^c_5$ which are present in $R_1$ are other than hydrogen atoms), or of a reactive derivative of this acid, and then the oxide obtained is optionally reduced, the protecting radicals are optionally removed, the isomers of the product obtained are optionally separated and it is optionally converted into a salt.

12. Process for the preparation of a product according to one of Claims 1, 2, 3, 4 or 5, for which n is equal to 1, characterised in that a product according to Claim 1, 2 or 3 for which n = 0 is oxidised by any known method for preparing a sulphoxide from a sulphide without affecting the remainder of the molecule, and then the isomers of the product obtained are optionally separated and it is optionally converted into a salt.

13. Process for the preparation of a product according to one of Claims 1, 2, 3, 4 or 5, for which, n being defined as in Claim 1, $R_1$ is defined as in Claim 1 at 2) or at 1a) or in Claim 2, 3 or 4, $R_2$ denotes a radical of general formula:

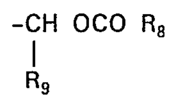

such as defined in Claim 1, and $R_3$ is defined as in Claim 1 or 5, characterised in that a product according to one of Claims 1, 2 or 3 (for which, $R_1$, $R_3$ and n being defined as above [it being understood that when $R_1$ contains a radical $R^c_5$, the latter is other than a hydrogen atom], $R_2$ is a hydrogen atom) is esterified, and then the oxide obtained is optionally reduced, the protecting radicals are optionally removed and the product obtained is optionally separated into its isomers.

14. Process for the preparation of a product according to one of Claims 1, 2, 3, 4 or 5 for which, n being defined as in Claim 1, $R_1$ denotes a radical of general formula:

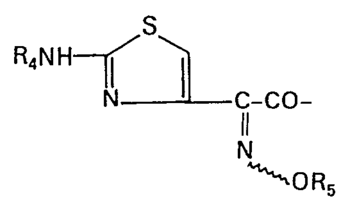

such as defined respectively in Claim 1 at 2) or at 1a) or in Claims 2, 3 or 4 (except for $R_4$ denoting a chloroacetyl or trichloroacetyl radical and/or for $R_5$ denoting vinyl), $R_2$ has the corresponding definition given in Claim 1, and $R_3$ is defined as in Claim 1 or 5, characterised in that a thiourea of general formula:

$R_4NH-CS-NH_2$

in which $R_4$ is defined as above, is made to react with a cephalosporin derivative of general formula

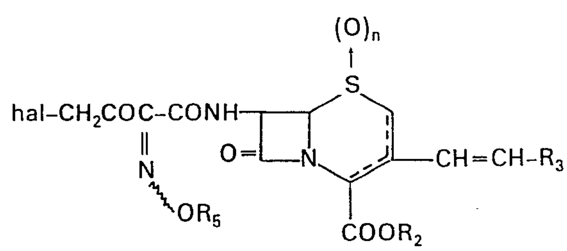

in which $R_3$ and n are defined as in Claim 1, $R_2$ and $R_5$ are defined as above and hal denotes a chlorine or bromine atom, or optionally with a mixture of the isomers of this product, and then the oxide obtained is optionally reduced, the protecting radicals are optionally removed, the isomers of the product obtained are optionally separated and it is optionally converted into a salt.

**Claims for the contracting State: AT**

1. Process for the preparation of a 3-vinylcephalosporin of general formula:

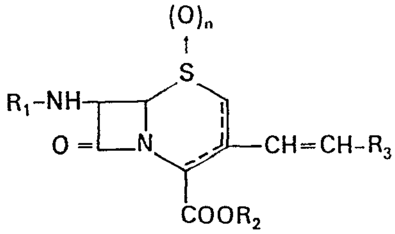

(I)

in which n is equal to 0 or 1,
1a) the symbol $R_1$ denotes
a hydrogen atom,
a radical of general formula

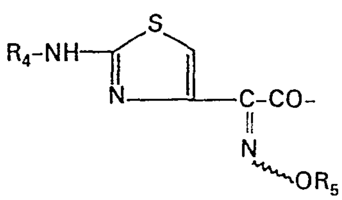

(II)

in syn or anti form,
(in which $R_4$ is a hydrogen atom or an amine-protecting radical and $R_5$ is a hydrogen atom, an alkyl, vinyl or cyanomethyl radical or an oxime-protecting group),
a benzhydryl or trityl radical,
an acyl radical of general formula:

$R_6CO-$

in which $R_6$ is a hydrogen atom or an alkyl (optionally substituted by one or more halogen atoms or by a phenyl or phenoxy radical) or phenyl radical, or
a radical of general formula:

$R_7OCO-$

in which $R_7$ is a branched unsubstituted alkyl radical or a straight-chain or branched alkyl radical bearing one or more substituents [chosen from halogen atoms and cyano, trialkylsilyl, phenyl, substituted phenyl (by one or more alkoxy, nitro or phenyl radicals)], vinyl, allyl or quinolyl radicals, or
a nitrophenylthio radical, or
$R_1NH-$ is replaced by a methyleneamino radical in which the methylene radical is substituted by a dialkylamino group or aryl group (itself optionally substituted by one or more methoxy or nitro radicals) and
the symbol $R_2$ denotes a hydrogen atom, a radical which is readily capable of elimination by an enzymatic route, of general formula:

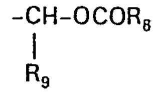

(in which $R_8$ denotes an alkyl radical or the cyclohexyl radical, and $R_9$ denotes a hydrogen atom or an alkyl radical) or a methoxymethyl, t-butyl, benzhydryl, nitrobenzyl or
p-methoxybenzyl radical, or
1b) the symbol $R_1$ denotes
- a hydrogen atom,
- an alkanoyl radical containing 1 to 8 carbon atoms, alkanoyl containing 2 to 8 carbon atoms which is substituted (by chlorine or bromine atoms),
- azidoacetyl, cyanoacetyl
- an acyl radical of general formula:

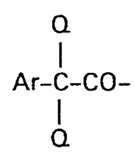

{in which Q is H or methyl and Ar denotes a 2-thienyl, 3-thienyl, 2-furyl, 3-furyl, 2-pyrrolyl, 3-pyrrolyl or phenyl [optionally substituted by halogen atoms or trifluoromethyl, hydroxy, alkyl (containing 1 to 3 carbon atoms), alkoxy (containing 1 to 3 carbon atoms), cyano or nitro radicals, at least one of which is situated in the meta or para position of the phenyl] radical},
an acyl radical or general formula:

$Ar-X-CH_2-CO-$

in which X is oxygen or sulphur and Ar is defined as above, or Ar-X- denotes 4-pyridylthio,
- an acyl radical of general formula:

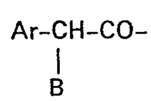

in which Ar is defined as previously and B denotes an amino radical, an amino radical which is protected (by a benzyloxycarbonyl, alkoxycarbonyl, cyclopentyloxycarbonyl, cyclohexyloxycarbonyl, benzhydryloxycarbonyl, trityl or 2,2,2-trichloroethoxycarbonyl group), a sulpho radical, a hydroxy or carboxy radical [which radicals are

optionally protected by esterification (with an alkanoic acid or an alcohol containing 1 to 6 carbon atoms respectively)], an azido, cyano or carbamoyl radical,
or a 2-(3-sydnone) alkanoyl radical (the alkanoyl part of which contains 1 to 3 carbon atoms)
or an acyl radical of general formula:

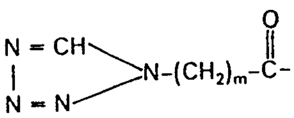

in which m is 0 to 2
or a 5-aminoadipoyl radical [in which the amino group is optionally protected by an alkanoyl radical (containing 1 to 3 carbon atoms and optionally substituted by a chlorine atom) and in which the carboxy group is protected by a benzhydryl, 2,2,2-trichloroethyl, t-alkyl (containing 4 to 6 carbon atoms) or nitrobenzyl radical], or $R_1NH$ is replaced by a dicarboxylic acid cyclic imide group, the symbol $R_2$ denotes a t-alkyl radical containing 4 to 6 carbon atoms, t-alkenyl radical containing 6 or 7 carbon atoms, t-alkynyl radical containing 6 or 7 carbon atoms, a benzyl, methoxybenzyl, nitrobenzyl, 2,2,2-trichloroethyl, benzhydryl, succinimidomethyl or phthalimidomethyl radical or a hydrogen atom and
the symbol $R_3$ denotes a halogen atom or
2) the symbol $R_1$ denotes a radical of general formula (II) in which $R_4$ is defined as in 1a) – and $R_5$ denotes a radical of general formula

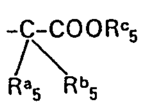 (IIa)

(in which
$R^a_5$ and $R^b_5$ which are identical or different, denote hydrogen atoms or alkyl radicals or form together an alkylene radical containing 2 or 3 carbon atoms and $R^c_5$ denotes a hydrogen atom or an acid-protecting radical), the symbol $R_2$ is defined as previously at 1a) – and the symbol $R_3$ denotes a halogen atom or a radical of general formula:

$R'_3-SO_2O-$

or $R''_3-COO-$

in which formulae $R'_3$ denotes an alkyl, trifluoromethyl, or trichloromethyl radical or a phenyl radical which is unsubstituted or substituted (by a halogen atom or an alkyl or nitro radical) and $R''_3$ is defined as $R'_3$ or denotes an acylmethyl, 2-acylethyl, 2-acylpropyl, alkoxycarbonylmethyl, 2-alkoxycarbonylethyl or 2-alkoxycarbonylpropyl radical,
the alkyl or acyl portions or radical mentioned above being (unless specially mentioned) straight-chain or branched and containing 1 to 4 carbon atoms, the product being in a 2- or 3-bycyclooctene form when n = 0 and in a 2-bicyclooctene form when n = 1 and the substituent in the 3-position being in an E or Z form, as well as the mixtures of its isomers and its salts, characterised in that

A) for the preparation of a product in the formula of which either, $R_1$, $R_2$ and $R_3$ are defined as at 2), or $R_1$ and $R_2$ are defined as at 1a), except for $R_1$ denoting the hydrogen atom, or $R_1$ denotes an alkanoyl radical containing 1 to 8 carbon atoms, or an alkanoyl radical containing 2 to 8 carbon atoms which is substituted (by chlorine or bromine atoms),
an acyl radical of general formula:

Q<br>|<br>Ar–C–CO–<br>|<br>Q

{in which Q is H or methyl and Ar denotes a 2-thienyl, 3-thienyl, 2-furyl, 3-furyl, 2-pyrrolyl, 3-pyrrolyl or phenyl [optionally substituted by halogen atoms or hydroxy, alkyl (containing 1 to 3 carbon atoms) or alkoxy (containing 1 to 3 carbon atoms) radicals, at least one of which is situated in the meta or para position of the phenyl] radical}, an acyl radical of general formula:

$Ar-X-CH_2-CO-$

such as defined above,
an acyl radical corresponding to the general formula:

Ar–CH–CO–<br>|<br>B

in which Ar is defined as above, and B denotes an amino radical which is protected (by a benzyloxycarbonyl, alkoxycarbonyl, cyclopentyloxycarbonyl, cyclohexyloxycarbonyl, benzhydryloxycarbonyl, trityl or 2,2,2-trichloroethoxycarbonyl group), a sulpho radical, a hydroxy or carboxy radical (optionally protected by esterification, with an alkanoic acid or an alcohol containing 1 to 6 carbon atoms respectively), or
a 5-aminoadipoyl radical [in which the amino group is optionally protected by an alkanoyl radical (containing 1 to 3 carbon atoms and optionally substituted by a chlorine atom) and in which the carboxy group is protected by a benzhydryl, 2,2,2-trichloroethyl, t-alkyl (containing 4 to 6 carbon atoms) or nitrobenzyl group] and
$R_2$ is defined as previously at 1b) and
$R_3$ is defined as at 1),
a halogenating agent or a product of general formula:

$(R'_3SO_2)_2O$

$R'_3SO_2Hal$

$(R''_3CO)_2O$

or $R''_3COHal$

in which $R'_3$ and $R''_3$ are defined as in Claim 1 and Hal denotes a halogen atom is made to react with a product of general formula:

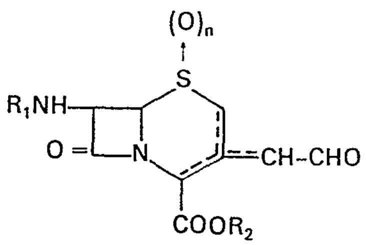

{in which the product is in a 3-oxoethyl-2-bicyclooctene or -3-bicyclooctene or 3-oxoethylidenebicyclooctane form when n = 0 and in a 3-oxoethyl-2-bicyclooctene or 3-oxoethylidenebicyclooctane form when n = 1, and $R_1$ is defined as above [except for denoting a radical of general formula:

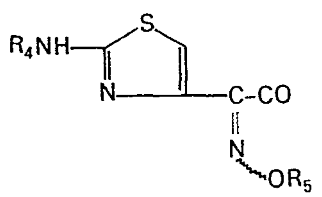

in which $R_4$ and/or $R_5$ are hydrogen, (and/or $R^c_5$ in $R_5$ is hydrogen when a halogenating agent is made to react)] and $R_{.2}$ is defined as above except for denoting hydrogen}, or with a mixture of its isomers, and then the sulphoxide obtained is optionally reduced or, conversely, the product obtained is oxidised to its S-oxide, the protecting groups are optionally removed, the isomers of the product obtained are optionally separated and this product is optionally converted into a salt, then

B) for the preparation of a product in the formula of which $R_2$, $R_3$ and n being defined as previously, $R_1$ denotes a hydrogen atom,

the radical $R_1$ is removed or optionally the radicals $R_1$ and $R_{.2}$ are simultaneously removed from a product prepared according to A) and in the formula of which $R_1$ and $R_2$ are defined as at 1a), except for $R_1$ denoting either hydrogen or a radical of general formula:

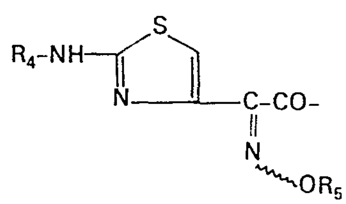

or $R_1$ denotes a 5-aminoadipoyl radical the amine and acid groups of which are protected, or a radical

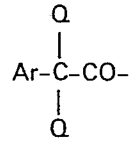

or $Ar-X-CH_2CO-$

such as defined previously and $R_2$ is defined as at 1b), and then the product obtained is optionally separated into its isomers and is optionally converted into a salt, then

C) for the preparation of a product in the formula of which $R_1$ denotes a radical of general formula:

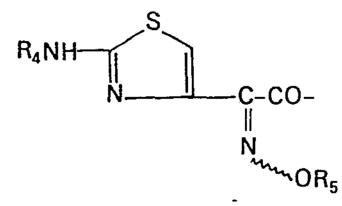

such as defined respectively at 2) or at 1a) or $R_1$ is defined as at 1b) except for denoting the hydrogen atom, a product prepared according to B) for which $R_1$ is defined as at 1b) except for denoting the hydrogen atom, a product prepared according to B) for which $R_1$ denotes hydrogen, or optionally a mixture of the isomers of this product is acylated by means of an acid denoted by the general formula:

$R_1OH$

(in which $R_1$ is defined as above, it being understood that, where appropriate, the radicals $R_4$ and/or $R^c_5$ which are present in $R_1$ are other than hydrogen atoms), or of a reactive derivative of this acid, and then the oxide obtained is optionally reduced or, conversely, the product obtained is oxidised to its S-oxide, the protecting radicals are optionally removed, the isomers of the product obtained are optionally separated and it is optionally converted into a salt, then

D) for the preparation of a product in the formula of which $R_1$ is defined as at 2) or at 1a), $R_2$ denotes a radical of general formula:

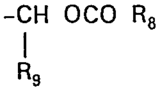

such as defined previously, and $R_3$ is defined as previously, the product obtained [in the formula of which, $R_1$, $R_3$ and n being defined as above (it being understood that when $R_1$ contains a radical $R^c_5$, the latter is other than a hydrogen atom), $R_2$ is a hydrogen atom] is esterified, and then the oxide obtained is optionally reduced or, conversely, the product obtained is oxidised to its S-oxide, the protecting radicals are optionally removed and the product obtained is optionally separated into its isomers, or

E) for the preparation of a product in the formula of which $R_1$ denotes a radical of general formula:

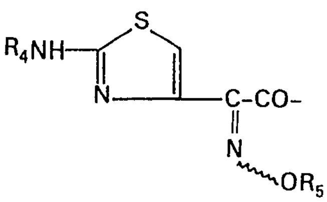

such as defined at 2) or at 1a) (except for $R_4$ denoting a chloroacetyl or trichloroacetyl radical and/or for $R_5$ denoting vinyl), and n, $R_2$ and $R_3$ have a corresponding definition, a thiourea of general formula:

$R_4NH\text{-}CS\text{-}NH_2$

in which $R_4$ is defined as above, is made to react with a cephalosporin derivative of general formula

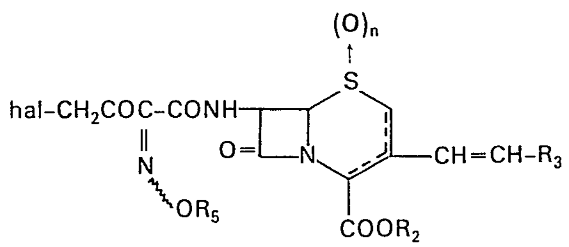

in which $R_2$, $R_3$, $R_5$ and n are defined as above and hal denotes a chlorine or bromine atom, or optionally with a mixture of the isomers of this product, and then the oxide obtained is optionally reduced, the protecting radicals are optionally removed, the isomers of the product obtained are optionally separated and it is optionally converted into a salt.

2. Process according to Claim 1 for the preparation of a product in the formula of which $R_3$ is a chlorine or bromine atom, characterised in that in step A the dihalogenated intermediate of general formula:

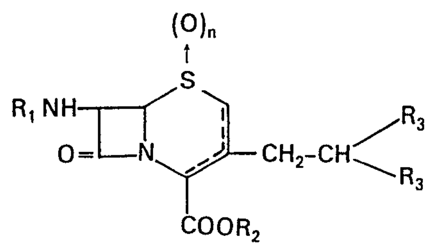

(or a mixture of its isomers)
which is in a 2- or 3-bicyclooctene form when n = 0 and in a 2-bicyclooctene form when n = 1, and in which $R_1$ and $R_2$ are defined as in Claim 1 for the starting aldehyde and $R_3$ is defined as above, is isolated, and then dehydrohalogenation of this derivative is carried out.